# EUROPEAN PATENT SPECIFICATION

(11) **EP 3 317 271 B1**
(45) Date of publication and mention of the grant of the patent: **13.05.2020**
(21) Application number: 16735714.4
(22) Date of filing: 30.06.2016
(51) Int. Cl.: C07D 409/14, C07D 495/04, A61K 31/55, A61K 31/4545, A61P 35/00

(54) **INHIBITORS OF EZH2 (ENHANCER OF ZESTE HOMOLOG 2)**
INHIBITOREN VON EZH2 (ENHANCER OF ZESTE HOMOLOG 2)
INHIBITEURS DE EZH2 (ENHANCER OF ZESTE HOMOLOG 2)

(30) Priority: 30.06.2015 US 201562186550 P; 14.10.2015 US 201562241254 P; 25.03.2016 US 201662313207 P; 05.05.2016 US 201662332099 P
(43) Date of publication of application: 09.05.2018
(73) Proprietor: GlaxoSmithKline Intellectual Property (No. 2) Limited, Brentford, Middlesex TW8 9GS (GB)
(72) Inventor: BRACKLEY, III, James A., Collegeville, Pennsylvania 19426 (US); GRAVES, III, Alan Peterson, King of Prussia Pennsylvania 19406 (US); KNIGHT, Steven David, Collegeville, Pennsylvania 19426 (US); MCNULTY, Kenneth C., Mississauga, Ontario L5H 1S9 (CA); NEWLANDER, Kenneth Allen, Collegeville, Pennsylvania 19426 (US); TIAN, Xinrong, Collegeville, Pennsylvania 19426 (US)
(74) Representative: Shore, Andrew David
(86) International application number: PCT/IB2016/053929
(87) International publication number: WO 2017/002064

(56) References cited:
- WO-A1-2014/177982
- WO-A1-2014/195919
- WO-A1-2015/004618
- WO-A1-2016/066697
- WO-A2-2012/118812

## Description

### FIELD OF THE INVENTION

This invention relates to compounds which inhibit Enhancer of Zeste Homolog 2 (EZH2) and thus are useful for inhibiting the proliferation of and/or inducing apoptosis in cancer cells.

### BACKGROUND OF THE INVENTION

Epigenetic modifications play an important role in the regulation of many cellular processes including cell proliferation, differentiation, and cell survival. Global epigenetic modifications are common in cancer, and include global changes in DNA and/or histone methylation, dysregulation of non-coding RNAs and nucleosome remodeling leading to aberrant activation or inactivation of oncogenes, tumor suppressors and signaling pathways. However, unlike genetic mutations which arise in cancer, these epigenetic changes can be reversed through selective inhibition of the enzymes involved. Several methylases involved in histone or DNA methylation are known to be dysregulated in cancer. Thus, selective inhibitors of particular methylases will be useful in the treatment of proliferative diseases such as cancer.

EZH2 (human EZH2 gene: Cardoso, C, et al; European J of Human Genetics, Vol. 8, No. 3 Pages 174-180, 2000) is the catalytic subunit of the Polycomb Repressor Complex 2 (PRC2) which functions to silence target genes by tri-methylating lysine 27 of histone H3 (H3K27me3). Histone H3 is one of the five main histone proteins involved in the structure of chromatin in eukaryotic cells. Featuring a main globular domain and a long N-terminal tail, Histones are involved with the structure of the nucleosomes, a 'beads on a string' structure. Histone proteins are highly post-translationally modified however Histone H3 is the most extensively modified of the five histones. The term "Histone H3" alone is purposely ambiguous in that it does not distinguish between sequence variants or modification state. Histone H3 is an important protein in the emerging field of epigenetics, where its sequence variants and variable modification states are thought to play a role in the dynamic and long term regulation of genes.

Increased EZH2 expression has been observed in numerous solid tumors including those of the prostate, breast, skin, bladder, liver, pancreas, head and neck and correlates with cancer aggressiveness, metastasis and poor outcome (Varambally et al. Nature 419:624-629, 2002; Kleer et al. Proc Natl Acad Sci USA 100:11606-11611, 2003; Breuer et al. Neoplasia 6:736-743, 2004; Bachmann et al. Prostate 65:252-259, 2005; Weikert et al. Int. J. Mol. Med. 16:349-353, 2005; Sudo et al. British Journal of Cancer 92:1754-1758, 2005; Bachmann et al. Journal of Clinical Oncology 24:268-273, 2006). For instance, there is a greater risk of recurrence after prostatectomy in tumors expressing high levels of EZH2, increased metastasis, shorter disease-free survival and increased death in breast cancer patients with high EZH2 levels (Varambally et al. Nature 419:624-629, 2002; Kleer et al. Proc Natl Acad Sci USA 100:11606-11611, 2003). More recently, inactivating mutations in UTX (ubiquitously transcribed tetratricopeptide repeats X), a H3K27 demethylase which functions in opposition to EZH2, have been identified in multiple solid and hematological tumor types (including renal, glioblastoma, esophageal, breast, colon, non-small cell lung, small cell lung, bladder, multiple myeloma, and chronic myeloid leukemia tumors), and low UTX levels correlate with poor survival in breast cancer suggesting that loss of UTX function leads to increased H3K27me3 and repression of target genes (Wang et al. Genes & Development 24:327-332, 2010). Together, these data suggest that increased H3K27me3 levels contribute to cancer aggressiveness in many tumor types and that inhibition of EZH2 activity may provide therapeutic benefit.

Numerous studies have reported that direct knockdown of EZH2 via siRNA or shRNA or indirect loss of EZH2 via treatment with the SAH hydrolase inhibitor 3-deazaneplanocin A (DZNep) decreases cancer cell line proliferation and invasion *in vitro* and tumor growth *in vivo* (Gonzalez et al., 2008, GBM 2009). While the precise mechanism by which aberrant EZH2 activity leads to cancer progression is not known, many EZH2 target genes are tumor suppressors suggesting that loss of tumor suppressor function is a key mechanism. In addition, EZH2 overexpression in immortalized or primary epithelial cells promotes anchorage independent growth and invasion and requires EZH2 catalytic activity (Kleer et al. Proc Natl Acad Sci USA 100:11606-11611, 2003; Cao et al. Oncogene 27:7274-7284, 2008).

Thus, there is strong evidence to suggest that inhibition of EZH2 activity decreases cellular proliferation and invasion. Accordingly, compounds that inhibit EZH2 activity would be useful for the treatment of cancer.

Latent human immunodeficiency virus (HIV) proviruses are silenced as the result of deacetylation and methylation of histones located at the viral long terminal repeat (LTR). Chromatin immunoprecipitation experiments using latently infected Jurkat T-cell lines demonstrated that EZH2 was present at high levels at the LTR of silenced HIV proviruses and was rapidly displaced following proviral reactivation. Knockdown of EZH2 induced up to 40% of the latent HIV proviruses. Knockdown of EZH2 also sensitized latent proviruses to external stimuli, such as T-cell receptor stimulation, and slowed the reversion of reactivated proviruses to latency. Similarly, cell populations that responded poorly to external stimuli carried HIV proviruses that were enriched in H3K27me3 and relatively depleted in H3K9me3. These findings suggest that PRC2-mediated silencing is an important feature of HIV latency and that inhibitors of histone methylation may play a useful role in induction strategies designed to eradicate latent HIV pools (Friedman et al. J. Virol. 85: 9078-9089, 2011). Additional studies have shown that H3K27 demethylation at the proviral promoter sensitizes latent HIV to the effects of vorinostat in *ex vivo* cultures of resting CD4⁺ T cells (Tripathy et al. J. Virol. 89: 8392-8405, 2015).

WO2014/177982, WO2015/004618 and WO2014/195919 disclose compounds which are EZH2 inhibitors and their use in therapy and the treatment of cancers.

WO2012/118812 discloses 6, 5-fused bicylic heteroaryl compounds and their use in the treatment of cancer.

### SUMMARY OF THE INVENTION

The present invention relates to compounds according to Formula (I) or pharmaceutically acceptable salts thereof: 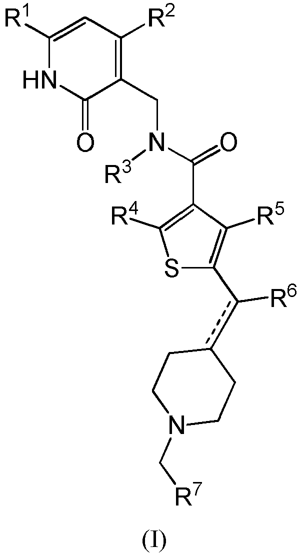 wherein:
----- represents a single or double bond;
R¹ is -NH₂, (C₁-C₄)alkyl, or hydroxy(C₁-C₄)alkyl;
R² is (C₁-C₄)alkyl;
R³ and R⁴ are each hydrogen;
or R³ and R⁴ taken together represent -CH₂CH₂- or -CH₂CH₂CH₂-;
R⁵ is hydrogen, halogen, or (C₁-C₃)alkyl;
R⁶ is hydrogen or (C₁-C₃)alkyl; and
R⁷ is a 6-membered saturated or unsaturated ring optionally containing one, two, or three heteroatoms independently selected from oxygen, nitrogen, and sulfur, wherein said ring is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-.

Another aspect of the invention relates to pharmaceutical compositions comprising compounds of Formula (I) and pharmaceutically acceptable excipients.

In another aspect, the invention provides a compound of Formula (I) or a pharmaceutically acceptable salt thereof for use in therapy.

In another aspect, there is provided a compound of Formula (I) or a pharmaceutically acceptable salt thereof for use in the treatment of cancer, including the treatment of solid tumors, for example brain (gliomas), glioblastomas, leukemias, lymphomas, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, gastric, bladder, head and neck, kidney, lung, liver, melanoma, renal, ovarian, pancreatic, prostate, sarcoma, osteosarcoma, giant cell tumor of bone, and thyroid.

### DETAILED DESCRIPTION OF THE INVENTION

This invention relates to compounds of the Formula (I) as defined above or pharmaceutically acceptable salts thereof.

In another embodiment, this invention also relates to compounds of Formula (II) or pharmaceutically acceptable salts thereof: 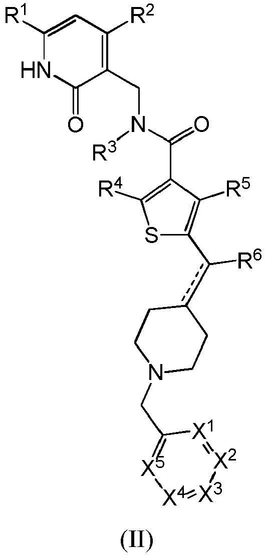 wherein:
----- represents a single or double bond;
X¹, X², X³, X⁴, and X⁵ are each independently N, CH, or CR⁸, provided that at least two of X¹, X², X³, X⁴, and X⁵ are CH;
R¹ is -NH₂, (C₁-C₄)alkyl, or hydroxy(C₁-C₄)alkyl;
R² is (C₁-C₄)alkyl;
R³ and R⁴ are each hydrogen;
or R³ and R⁴ taken together represent -CH₂CH₂- or -CH₂CH₂CH₂-;
R⁵ is hydrogen, halogen, or (C₁-C₃)alkyl;
R⁶ is hydrogen or (C₁-C₃)alkyl; and
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, hydroxyl, (C₁-C₄)alkoxy, hydroxy(C₂-C₄)alkoxy-, and (C₁-C₄)alkoxy(C₂-C₄)alkoxy-.

In another embodiment, this invention also relates to compounds of Formula (III) or pharmaceutically acceptable salts thereof: 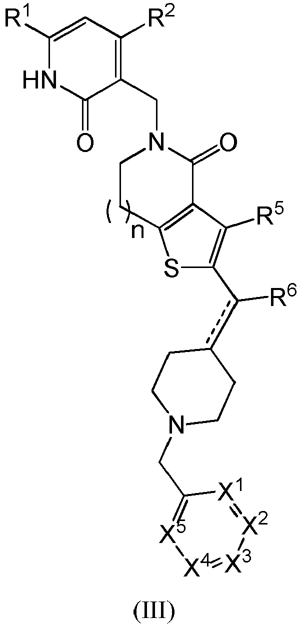 wherein:
----- represents a single or double bond;
X¹, X², X³, X⁴, and X⁵ are each independently N, CH, or CR⁸, provided that at least two of X¹, X², X³, X⁴, and X⁵ are CH;
n is 1 or 2;
R¹ is -NH₂, (C₁-C₄)alkyl, or hydroxy(C₁-C₄)alkyl;
R² is (C₁-C₄)alkyl;
R⁵ is hydrogen, halogen, or (C₁-C₃)alkyl;
R⁶ is hydrogen or (C₁-C₃)alkyl; and
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, hydroxyl, (C₁-C₄)alkoxy, hydroxy(C₂-C₄)alkoxy-, and (C₁-C₄)alkoxy(C₂-C₄)alkoxy-.

In another embodiment, this invention also relates to compounds of Formula (IV) or pharmaceutically acceptable salts thereof: 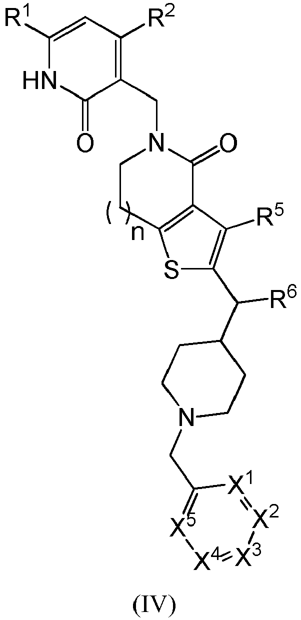 wherein:
X¹, X², X³, X⁴, and X⁵ are each independently N, CH, or CR⁸, provided that at least two of X¹, X², X³, X⁴, and X⁵ are CH;
n is 1 or 2;
R¹ is -NH₂, (C₁-C₄)alkyl, or hydroxy(C₁-C₄)alkyl;
R² is (C₁-C₄)alkyl;
R⁵ is hydrogen, halogen, or (C₁-C₃)alkyl;
R⁶ is hydrogen or (C₁-C₃)alkyl; and
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, hydroxyl, (C₁-C₄)alkoxy, hydroxy(C₂-C₄)alkoxy-, and (C₁-C₄)alkoxy(C₂-C₄)alkoxy-.

In another embodiment, this invention also relates to compounds of Formula (IV)(a) or pharmaceutically acceptable salts thereof: 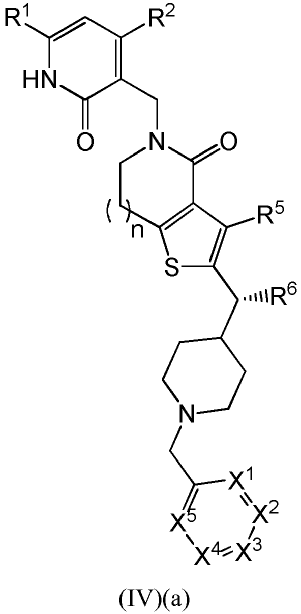 wherein:
X¹, X², X³, X⁴, and X⁵ are each independently N, CH, or CR⁸, provided that at least two of X¹, X², X³, X⁴, and X⁵ are CH;
n is 1 or 2;
R¹ is -NH₂, (C₁-C₄)alkyl, or hydroxy(C₁-C₄)alkyl;
R² is (C₁-C₄)alkyl;
R⁵ is hydrogen, halogen, or (C₁-C₃)alkyl;
R⁶ is hydrogen or (C₁-C₃)alkyl; and
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, hydroxyl, (C₁-C₄)alkoxy, hydroxy(C₂-C₄)alkoxy-, and (C₁-C₄)alkoxy(C₂-C₄)alkoxy-.

In another embodiment, this invention also relates to compounds of Formula (V) or pharmaceutically acceptable salts thereof: 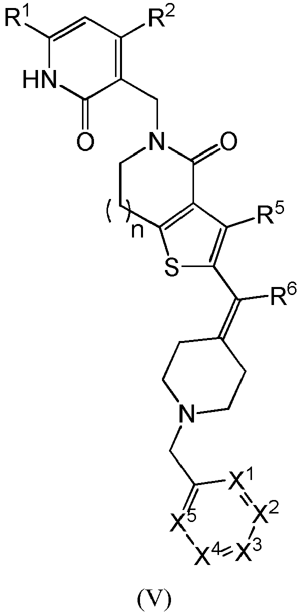 wherein:
X¹, X², X³, X⁴, and X⁵ are each independently N, CH, or CR⁸, provided that at least two of X¹, X², X³, X⁴, and X⁵ are CH;
n is 1 or 2;
R¹ is -NH₂, (C₁-C₄)alkyl, or hydroxy(C₁-C₄)alkyl;
R² is (C₁-C₄)alkyl;
R⁵ is hydrogen, halogen, or (C₁-C₃)alkyl;
R⁶ is hydrogen or (C₁-C₃)alkyl; and
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, hydroxyl, (C₁-C₄)alkoxy, hydroxy(C₂-C₄)alkoxy-, and (C₁-C₄)alkoxy(C₂-C₄)alkoxy-.

In one embodiment, this invention relates to compounds of Formula (I), (II), or (III) wherein ----- represents a single bond. In another embodiment, this invention relates to compounds of Formula (I), (II), or (III) wherein ----- represents a double bond.

In another embodiment, this invention relates to compounds of Formula (I), (II), (III), (IV), (IV)(a), or (V) wherein R¹ is (C₁-C₄)alkyl. In a specific embodiment, this invention relates to compounds of Formula (I), (II), (III), (IV), (IV)(a), or (V) wherein R¹ is -NH₂. In another embodiment, this invention relates to compounds of Formula (I), (II), (III), (IV), (IV)(a), or (V) wherein R¹ and R² are each independently methyl, ethyl, n-propyl, or n-butyl. In a specific embodiment, this invention relates to compounds of Formula (I), wherein R¹ and R² are each methyl.

In another specific embodiment, this invention relates to compounds of Formula (I) or (II) wherein R³ and R⁴ are each hydrogen. In another embodiment, this invention relates to compounds of Formula (I) or (II) wherein R³ and R⁴ taken together represent -CH₂CH₂- or -CH₂CH₂C_{H}2-. In a specific embodiment, this invention relates to compounds of Formula (I) or (II) wherein R³ and R⁴ taken together represent -CH₂CH₂-. In another specific embodiment, this invention relates to compounds of Formula (I) or (II) wherein R³ and R⁴ taken together represent -CH₂CH₂CH₂-.

In another embodiment, this invention relates to compounds of Formula (I), (II), (III), (IV), (IV)(a), or (V) wherein R⁵ is hydrogen, fluoro, chloro, methyl, ethyl, n-propyl, or isopropyl. In another embodiment, this invention relates to compounds of Formula (I), (II), (III), (IV), (IV)(a), or (V) wherein R⁵ is methyl, ethyl, n-propyl, or isopropyl. In another embodiment, this invention relates to compounds of Formula (I), (II), (III), (IV), (IV)(a), or (V) wherein R⁵ is methyl or chloro. In a specific embodiment, this invention relates to compounds of Formula (I), (II), (III), (IV), (IV)(a), or (V) wherein R⁵ is methyl. In another specific embodiment, this invention relates to compounds of Formula (I), (II), (III), (IV), (IV)(a), or (V) wherein R⁵ is chloro.

In another embodiment, this invention relates to compounds of Formula (I), (II), (III), (IV), (IV)(a), or (V) wherein R⁶ is hydrogen, methyl, ethyl, *n*-propyl, or isopropyl. In another embodiment, this invention relates to compounds of Formula (I), (II), (III), (IV), (IV)(a), or (V) wherein R⁶ is hydrogen, methyl, or ethyl. In a specific embodiment, this invention relates to compounds of Formula (I), (II), (III), (IV), (IV)(a), or (V) wherein R⁶ is ethyl.

In another embodiment, this invention relates to compounds of Formula (I) wherein R⁷ is selected from the group consisting of cyclohexyl, piperidinyl, tetrahydropyranyl, tetrahydrothiopyranyl, piperazinyl, morpholinyl, thiomorpholinyl, 1,4-dioxanyl, 1,4-dithianyl, phenyl, pyridinyl, pyridazinyl, pyrimidinyl, pyrazinyl, and triazinyl, each of which is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-. In another embodiment, this invention relates to compounds of Formula (I) wherein R⁷ is selected from the group consisting of phenyl, pyridinyl, pyridazinyl, pyrimidinyl, pyrazinyl, and triazinyl, each of which is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-.

In another embodiment, this invention relates to compounds of Formula (I) wherein R⁷ is phenyl optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-. In another embodiment, this invention relates to compounds of Formula (I) wherein R⁷ is phenyl optionally substituted by one or two groups independently selected from halogen, (C₁-C₄)alkyl, halo(C₁-C₄)alkyl, hydroxyl, and (C₁-C₄)alkoxy.

In another embodiment, this invention relates to compounds of Formula (I) wherein R⁷ is pyridinyl optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-. In another embodiment, this invention relates to compounds of Formula (I) wherein R⁷ is pyridinyl optionally substituted by one or two groups independently selected from halogen, (C₁-C₄)alkyl, halo(C₁-C₄)alkyl, hydroxyl, and (C₁-C₄)alkoxy.

In another embodiment, this invention relates to compounds of Formula (I) wherein R⁷ is pyridazinyl, pyrimidinyl, or pyrazinyl, each optionally substituted by one or two groups independently selected from halogen, (C₁-C₄)alkyl, halo(C₁-C₄)alkyl, hydroxyl, and (C₁-C₄)alkoxy.

In another embodiment, this invention relates to compounds of Formula (I) wherein R⁷ is selected from the group consisting of cyclohexyl, piperidinyl, tetrahydropyranyl, tetrahydrothiopyranyl, piperazinyl, morpholinyl, thiomorpholinyl, 1,4-dioxanyl, and 1,4-dithianyl, each of which is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl, -CONH₂, -CONH(C₁-C₄)alkyl, -CON(C₁-C₄)alkyl(C₁-C₄)alkyl, -NH₂, -NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-. In another embodiment, this invention relates to compounds of Formula (I) wherein R⁷ is selected from the group consisting of cyclohexyl, piperidinyl, tetrahydropyranyl, tetrahydrothiopyranyl, piperazinyl, morpholinyl, and thiomorpholinyl, each optionally substituted by one or two groups independently selected from halogen, (C₁-C₄)alkyl, halo(C₁-C₄)alkyl, hydroxyl, and (C₁-C₄)alkoxy. In another embodiment, this invention relates to compounds of Formula (I) wherein R⁷ is cyclohexyl which is optionally substituted by one or two groups independently selected from halogen, (C₁-C₄)alkyl, halo(C₁-C₄)alkyl, hydroxyl, and (C₁-C₄)alkoxy. In another embodiment, this invention relates to compounds of Formula (I) wherein R⁷ is selected from the group consisting of piperidinyl, tetrahydropyranyl, piperazinyl, and morpholinyl, each optionally substituted by one or two groups independently selected from halogen, (C₁-C₄)alkyl, halo(C₁-C₄)alkyl, hydroxyl, and (C₁-C₄)alkoxy.

In another embodiment, this invention relates to compounds of Formula (II), (III), (IV), (IV)(a), or (V) wherein X¹, X², X³, X⁴, and X⁵ are each independently N, CH, or CR⁸, provided that at least three of X¹, X², X³, X⁴, and X⁵ are CH. In another embodiment, this invention relates to compounds of Formula (II), (III), (IV), (IV)(a), or (V) wherein X¹, X², X³, X⁴, and X⁵ are each independently N, CH, or CR⁸, provided that not more than one of X¹, X², X³, X⁴, and X⁵ is N and at least three of X¹, X², X³, X⁴, and X⁵ are CH. In another embodiment, this invention relates to compounds of Formula (II), (III), (IV), (IV)(a), or (V) wherein X¹, X², X³, X⁴, and X⁵ are each independently N or CH, provided that at least three of X¹, X², X³, X⁴, and X⁵ are CH. In another embodiment, this invention relates to compounds of Formula (II), (III), (IV), (IV)(a), or (V) wherein X¹, X², X³, X⁴, and X⁵ are each independently CH or CR⁸, provided that at least three of X¹, X², X³, X⁴, and X⁵ are CH.

In another embodiment, this invention relates to compounds of Formula (II), (III), (IV), (IV)(a), or (V) wherein each R⁸ is independently selected from halogen, (C₁-C₄)alkyl, halo(C₁-C₄)alkyl, hydroxyl, and (C₁-C₄)alkoxy. In another embodiment, this invention relates to compounds of Formula (II), (III), (IV), (IV)(a), or (V) wherein each R⁸ is independently selected from fluoro, chloro, methyl, trifluoromethyl, and methoxy.

In a specific embodiment, this invention relates to compounds of Formula (III), (IV), (IV)(a), or (V) wherein n is 1. In another specific embodiment, this invention relates to compounds of Formula (III), (IV), (IV)(a), or (V) wherein n is 2.

Specific compounds of this invention include:
(*R*)-5-((4,6-dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-*c*]pyridin-4(5*H*)-one;
(*R*)-2-(1-(1-benzylpiperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)-one;
(*R*)-5-((4,6-dimethyl-2-oxo-1,2-dihydropyridin-3-yl)methyl)-3-methyl-2-(1-(1-(pyridin-4-ylmethyl)piperidin-4-yl)propyl)-6,7-dihydrothieno[3,2-*c*]pyridin-4(5*H*)-one;
(*R*)-2-(1-(1-((5-chloropyridin-2-yl)methyl)piperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)-one;
*N*-((4,6-dimethyl-2-oxo-1,2-dihydropyridin-3-yl)methyl)-5-(1-(1-((2-methoxypyridin-4-yl)methyl)piperidin-4-ylidene)propyl)-4-methylthiophene-3-carboxamide;
(*R*)-5-((4,6-dimethyl-2-oxo-1,2-dihydropyridin-3-yl)methyl)-3-methyl-2-(1-(1-(pyridin-2-ylmethyl)piperidin-4-yl)ethyl)-5,6,7,8-tetrahydro-4*H*-thieno[3,2-*c*]azepin-4-one;
(*R*)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4*H-*thieno[3,2-*c*]azepin-4-one;
(*R*)-2-(1-(1-((5-chloropyridin-2-yl)methyl)piperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-one;
(*R*)-5-((4,6-dimethyl-2-oxo-1,2-dihydropyridin-3-yl)methyl)-3-methyl-2-(1-(1-((6-methylpyridin-2-yl)methyl)piperidin-4-yl)ethyl)-5,6,7,8-tetrahydro-4*H*-thieno[3,2-*c*]azepin-4-one;
(*R*)-2-(1-(1-benzylpiperidin-4-yl)ethyl)-5-((4,6-dimethyl-2-oxo-1,2-dihydropyridin-3-yl)methyl)-3-methyl-5,6,7,8-tetrahydro-4*H-*[3,2-*c*]azepin-4-one;
(*R*)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-6,7-dihydrothieno[3,2-*c*]pyridin-4(5*H*)-one;
(*R*)-5-((4,6-dimethyl-2-oxo-1,2-dihydropyridin-3-yl)methyl)-2-(1-(1-((6-methoxypyridin-2-yl)methyl)piperidin-4-yl)ethyl)-3-methyl-5,6,7,8-tetrahydro-4*H-*thieno[3,2-*c*]azepin-4-one;
(*R*)-2-(1-(1-(cyclohexylmethyl)piperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-one; and
(*R*)-5-((4,6-dimethyl-2-oxo-1,2-dihydropyridin-3-yl)methyl)-3-methyl-2-(1-(1-((1-methylcyclohexyl)methyl)piperidin-4-yl)ethyl)-5,6,7,8-tetrahydro-4*H*-thieno[3,2-*c*]azepin-4-one;
or pharmaceutically acceptable salts thereof.

Typically, but not absolutely, the salts of the present invention are pharmaceutically acceptable salts. Salts of the disclosed compounds containing a basic amine or other basic functional group may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, pyranosidyl acid, such as glucuronic acid or galacturonic acid, alpha-hydroxy acid, such as citric acid or tartaric acid, amino acid, such as aspartic acid or glutamic acid, aromatic acid, such as benzoic acid or cinnamic acid, sulfonic acid, such as *p*-toluenesulfonic acid, methanesulfonic acid, ethanesulfonic acid or the like. Examples of pharmaceutically acceptable salts include sulfates, pyrosulfates, bisulfates, sulfites, bisulfites, phosphates, chlorides, bromides, iodides, acetates, propionates, decanoates, caprylates, acrylates, formates, isobutyrates, caproates, heptanoates, propiolates, oxalates, malonates succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, phenylacetates, phenylpropionates, phenylbutrates, citrates, lactates, γ-hydroxybutyrates, glycolates, tartrates mandelates, and sulfonates, such as xylenesulfonates, methanesulfonates, propanesulfonates, naphthalene-1-sulfonates and naphthalene-2-sulfonates.

Salts of the disclosed compounds containing a carboxylic acid or other acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, *N*,*N*'-dibenzylethylenediamine, 2-hydroxyethylamine, *bis*-(2-hydroxyethyl)amine, tri-(2-hydroxyethyl)amine, procaine, dibenzylpiperidine, dehydroabietylamine, *N*,*N*'-*bis*dehydroabietylamine, glucamine, *N*-methylglucamine, collidine, quinine, quinoline, and basic amino acid such as lysine and arginine.

Other salts, which are not pharmaceutically acceptable, may be useful in the preparation of compounds of this invention and these should be considered to form a further aspect of the invention. These salts, such as oxalic or trifluoroacetate, while not in themselves pharmaceutically acceptable, may be useful in the preparation of salts useful as intermediates in obtaining the compounds of the invention and their pharmaceutically acceptable salts.

The compound of Formula (I) or a salt thereof may exist in stereoisomeric forms (e.g., it contains one or more asymmetric carbon atoms). The individual stereoisomers (enantiomers and diastereomers) and mixtures of these are included within the scope of the present invention. Likewise, it is understood that a compound or salt of Formula (I) may exist in tautomeric forms other than that shown in the formula and these are also included within the scope of the present invention. It is to be understood that the present invention includes all combinations and subsets of the particular groups defined hereinabove. The scope of the present invention includes mixtures of stereoisomers as well as purified enantiomers or enantiomerically/diastereomerically enriched mixtures.

The subject invention also includes isotopically-labeled compounds, which are identical to those recited in Formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulfur, fluorine, chlorine, and iodine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I, and ¹²⁵I.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds of Formula (I) and following of this invention can generally be prepared by carrying out the procedures disclosed in the Schemes and/or in the Examples below, by substituting a readily available isotopically labeled reagent for a non-isotopically labeled reagent.

The invention further provides a pharmaceutical composition (also referred to as pharmaceutical formulation) comprising a compound of Formula (I) or pharmaceutically acceptable salt thereof and one or more excipients (also referred to as carriers and/or diluents in the pharmaceutical arts). The excipients are acceptable in the sense of being compatible with the other ingredients of the formulation and not deleterious to the recipient thereof (i.e., the patient).

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting of the compound or compounds of the invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, hemectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

Pharmaceutical compositions may be in unit dose form containing a predetermined amount of active ingredient per unit dose. Such a unit may contain a therapeutically effective dose of the compound of Formula (I) or salt thereof or a fraction of a therapeutically effective dose such that multiple unit dosage forms might be administered at a given time to achieve the desired therapeutically effective dose. Preferred unit dosage formulations are those containing a daily dose or sub-dose, as herein above recited, or an appropriate fraction thereof, of an active ingredient. Furthermore, such pharmaceutical compositions may be prepared by any of the methods well-known in the pharmacy art.

Pharmaceutical compositions may be adapted for administration by any appropriate route, for example, by oral (including buccal or sublingual), rectal, nasal, topical (including buccal, sublingual, or transdermal), vaginal, or parenteral (including subcutaneous, intramuscular, intravenous, or intradermal) routes. Such compositions may be prepared by any method known in the art of pharmacy, for example, by bringing into association the active ingredient with the excipient(s).

When adapted for oral administration, pharmaceutical compositions may be in discrete units such as tablets or capsules; powders or granules; solutions or suspensions in aqueous or non-aqueous liquids; edible foams or whips; oil-in-water liquid emulsions or water-in-oil liquid emulsions. The compound or salt thereof of the invention or the pharmaceutical composition of the invention may also be incorporated into a candy, a wafer, and/or tongue tape formulation for administration as a "quick-dissolve" medicine.

For instance, for oral administration in the form of a tablet or capsule, the active drug component can be combined with an oral, non-toxic pharmaceutically acceptable inert carrier such as ethanol, glycerol, water, and the like. Powders or granules are prepared by comminuting the compound to a suitable fine size and mixing with a similarly comminuted pharmaceutical carrier such as an edible carbohydrate, as, for example, starch or mannitol. Flavoring, preservative, dispersing, and coloring agents can also be present.

Capsules are made by preparing a powder mixture, as described above, and filling formed gelatin or non-gelatinous sheaths. Glidants and lubricants such as colloidal silica, talc, magnesium stearate, calcium stearate, solid polyethylene glycol can be added to the powder mixture before the filling operation. A disintegrating or solubilizing agent such as agar-agar, calcium carbonate, or sodium carbonate can also be added to improve the availability of the medicine when the capsule is ingested.

Moreover, when desired or necessary, suitable binders, lubricants, disintegrating agents, and coloring agents can also be incorporated into the mixture. Suitable binders include starch, gelatin, natural sugars, such as glucose or beta-lactose, corn sweeteners, natural and synthetic gums such as acacia, tragacanth, sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methylcellulose, agar, bentonite, xanthan gum, and the like.

Tablets are formulated, for example, by preparing a powder mixture, granulating or slugging, adding a lubricant and disintegrant, and pressing into tablets. A powder mixture is prepared by mixing the compound, suitably comminuted, with a diluent or base as described above, and optionally, with a binder such as carboxymethylcellulose, and aliginate, gelatin, or polyvinyl pyrrolidone, a solution retardant such as paraffin, a resorption accelerator such as a quaternary salt, and/or an absorption agent such as bentonite, kaolin, or dicalcium phosphate. The powder mixture can be granulated by wetting a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compound or salt of the present invention can also be combined with a free-flowing inert carrier and compressed into tablets directly without going through the granulating or slugging steps. A clear opaque protective coating consisting of a sealing coat of shellac, a coating of sugar, or polymeric material, and a polish coating of wax can be provided. Dyestuffs can be added to these coatings to distinguish different dosages.

Oral fluids such as solutions, syrups, and elixirs can be prepared in dosage unit form so that a given quantity contains a predetermined amount of active ingredient. Syrups can be prepared by dissolving the compound or salt thereof of the invention in a suitably flavoured aqueous solution, while elixirs are prepared through the use of a non-toxic alcoholic vehicle. Suspensions can be formulated by dispersing the compound or salt of the invention in a non-toxic vehicle. Solubilizers and emulsifiers, such as ethoxylated isostearyl alcohols and polyoxyethylene sorbitol ethers, preservatives, flavor additives such as peppermint oil, natural sweeteners, saccharin, or other artificial sweeteners, and the like, can also be added.

Where appropriate, dosage unit formulations for oral administration can be microencapsulated. The formulation can also be prepared to prolong or sustain the release as, for example, by coating or embedding particulate material in polymers, wax, or the like.

In the present invention, tablets and capsules are preferred for delivery of the pharmaceutical composition.

In accordance with another aspect of the invention there is provided a process for the preparation of a pharmaceutical composition comprising mixing (or admixing) a compound of Formula (I) or salt thereof with at least one excipient.

The present invention also provides a compound of the invention for use in therapy. The compounds and compositions of the invention are used to treat cellular proliferation diseases. Disease states which can be treated by the compositions provided herein include, but are not limited to, cancer (further discussed below), autoimmune disease, fungal disorders, arthritis, graft rejection, inflammatory bowel disease, proliferation induced after medical procedures, including, but not limited to, surgery, angioplasty, and the like. It is appreciated that in some cases the cells may not be in a hyper or hypo proliferation state (abnormal state) and still requires treatment. For example, during wound healing, the cells may be proliferating "normally", but proliferation enhancement may be desired.

The compositions provided herein are particularly deemed useful for the treatment of cancer including tumors such as prostate, breast, brain, skin, cervical carcinomas, testicular carcinomas, etc. They are particularly useful in treating metastatic or malignant tumors. More particularly, cancers that may be treated by the compositions of the invention include, but are not limited to tumor types such as astrocytic, breast, cervical, colorectal, endometrial, esophageal, gastric, head and neck, hepatocellular, laryngeal, lung, oral, ovarian, prostate and thyroid carcinomas and sarcomas. More specifically, these compounds can be used to treat: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Karposi's sarcoma, moles dysplastic nevi, lipoma, angioma, dermatofibroma, keloids, psoriasis; and Adrenal glands: neuroblastoma. Thus, the term "cancerous cell" as provided herein, includes a cell afflicted by any one or related of the above identified conditions.

The compounds and compositions of the invention may also be used to treat or cure human immunodeficiency virus (HIV) infection. In one embodiment, there is provided the use of a compound of Formula (I) or a pharmaceutically acceptable salt or solvate thereof, in the manufacture of a medicament for use in the treatment of HIV infection. In another embodiment, there is provided the use of a compound of Formula (I) or a pharmaceutically acceptable salt or solvate thereof, in the manufacture of a medicament for use in the cure of HIV infection. In another embodiment, there is provided a compound of Formula (I) or a pharmaceutically acceptable salt thereof for use in the treatment of HIV infection. In another embodiment, there is provided a compound of Formula (I) or a pharmaceutically acceptable salt thereof for use in the cure of HIV infection.

The instant compounds can be combined with or co-administered with other therapeutic agents, particularly agents that may enhance the activity or time of disposition of the compounds. Combination therapies according to the invention comprise the administration of at least one compound of the invention and the use of at least one other treatment method. In one embodiment, combination therapies according to the invention comprise the administration of at least one compound of the invention and surgical therapy. In one embodiment, combination therapies according to the invention comprise the administration of at least one compound of the invention and radiotherapy. In one embodiment, combination therapies according to the invention comprise the administration of at least one compound of the invention and at least one supportive care agent (e.g., at least one anti-emetic agent). In one embodiment, combination therapies according to the present invention comprise the administration of at least one compound of the invention and at least one other chemotherapeutic agent. In one particular embodiment, the invention comprises the administration of at least one compound of the invention and at least one anti-neoplastic agent. In yet another embodiment, the invention comprises a therapeutic regimen where the EZH2 inhibitors of this disclosure are not in and of themselves active or significantly active, but when combined with another therapy, which may or may not be active as a standalone therapy, the combination provides a useful therapeutic outcome.

By the term "co-administering" and derivatives thereof as used herein refers to either simultaneous administration or any manner of separate sequential administration of an EZH2 inhibiting compound, as described herein, and a further active ingredient or ingredients, known to be useful in the treatment of cancer, including chemotherapy and radiation treatment. The term further active ingredient or ingredients, as used herein, includes any compound or therapeutic agent known to or that demonstrates advantageous properties when administered to a patient in need of treatment for cancer. Preferably, if the administration is not simultaneous, the compounds are administered in a close time proximity to each other. Furthermore, it does not matter if the compounds are administered in the same dosage form, e.g. one compound may be administered topically and another compound may be administered orally.

Typically, any anti-neoplastic agent that has activity versus a susceptible tumor being treated may be co-administered in the treatment of specified cancers in the present invention. Examples of such agents can be found in Cancer Principles and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; DNA methyltransferase inhibitors such as azacitidine and decitabine; signal transduction pathway inhibitors; non-receptor tyrosine kinase angiogenesis inhibitors; immunotherapeutic agents; proapoptotic agents; and cell cycle signaling inhibitors.

Typically, any chemotherapeutic agent that has activity against a susceptible neoplasm being treated may be utilized in combination with the compounds the invention, provided that the particular agent is clinically compatible with therapy employing a compound of the invention. Typical anti-neoplastic agents useful in the present invention include, but are not limited to: alkylating agents, anti-metabolites, antitumor antibiotics, antimitotic agents, nucleoside analogues, topoisomerase I and II inhibitors, hormones and hormonal analogues; retinoids, histone deacetylase inhibitors; signal transduction pathway inhibitors including inhibitors of cell growth or growth factor function, angiogenesis inhibitors, and serine/threonine or other kinase inhibitors; cyclin dependent kinase inhibitors; antisense therapies and immunotherapeutic agents, including monoclonals, vaccines or other biological agents.

Select oncology agents that may be used in combination with a compound of Formula (I) or a pharmaceutically acceptable salt thereof, include but are not limited to: 5-fluorouracil, abarelix, abiraterone, ado-trastuzumab emtansine, afatinib, aflibercept, alectinib, anastrozole, atezolizumab, axitinib, belinostat, bendamustine, bevacizumab, blinatumomab, bortezomib, bosutinib, brentuximab vedotin, cabazitaxel, cabozantinib, carfilzomib, ceritinib, cetuximab, clofarabine, cobimetinib, crizotinib, dabrafenib, daratumumab, dasatinib, degarelix, denosumab, dinutuximab, docetaxel, doxorubicin, elotuzumab, enzalutamide, epirubicin, eribulin, erlotinib, everolimus, filgrastim, flutamide, fulvestrant, gefitinib, gemcitabine, gemtuzumab ozogamicin, goserelin, ibritumomab, ibrutinib, idelalisib, imatinib, ipilimumab, irinotecan, ixabepilone, ixazomib, lapatinib, lenalidomide, lenvatinib, letrozole, leucovorin, leuprolide, mechlorethamine, necitumumab, nelarabine, netupitant, nilotinib, nivolumab, obinutuzumab, ofatumumab, olaparib, omacetaxine, osimertinib, oxaliplatin, paclitaxel, palbociclib, palonosetron, panitumumab, panobinostat, pazopanib, pembrolizumab, pemetrexed, pertuzumab, plerixafor, pomalidomide, ponatinib, pralatrexate, ramucirumab, regorafenib, rituximab, rolapitant, romidepsin, sipuleucel-T, sonidegib, sorafenib, sunitinib, temsirolimus, tipiracil, topotecan, trabectedin, trametinib, trastuzumab, trifluridine, triptorelin, uridine, vandetanib, vemurafenib, venetoclax, vincristine, vismodegib, and vorinostat.

Nucleoside analogues are those compounds which are converted to deoxynucleotide triphosphates and incorporated into replicating DNA in place of cytosine. DNA methyltransferases become covalently bound to the modified bases resulting in an inactive enzyme and reduced DNA methylation. Examples of nucleoside analogues include azacitidine and decitabine which are used for the treatment of myelodysplastic disorder. Histone deacetylase (HDAC) inhibitors include vorinostat, for the treatment of cutaneous T-cell lymphoma. HDACs modify chromatin through the deacetylation of histones. In addition, they have a variety of substrates including numerous transcription factors and signaling molecules. Other HDAC inhibitors are in development.

Signal transduction pathway inhibitors are those inhibitors which block or inhibit a chemical process which evokes an intracellular change. As used herein this change is cell proliferation or differentiation or survival. Signal transduction pathway inhibitors useful in the present invention include, but are not limited to, inhibitors of receptor tyrosine kinases, non-receptor tyrosine kinases, SH2/SH3 domain blockers, serine/threonine kinases, phosphatidyl inositol-3-OH kinases, myoinositol signaling, and Ras oncogenes. Signal transduction pathway inhibitors may be employed in combination with the compounds of the invention in the compositions and methods described above.

Receptor kinase angiogenesis inhibitors may also find use in the present invention. Inhibitors of angiogenesis related to VEGFR and TIE-2 are discussed above in regard to signal transduction inhibitors (both are receptor tyrosine kinases). Other inhibitors may be used in combination with the compounds of the invention. For example, anti-VEGF antibodies, which do not recognize VEGFR (the receptor tyrosine kinase), but bind to the ligand; small molecule inhibitors of integrin (alphaᵥ beta₃) that inhibit angiogenesis; endostatin and angiostatin (non-RTK) may also prove useful in combination with the compounds of the invention. One example of a VEGFR antibody is bevacizumab (AVASTIN®).

Several inhibitors of growth factor receptors are under development and include ligand antagonists, antibodies, tyrosine kinase inhibitors, anti-sense oligonucleotides and aptamers. Any of these growth factor receptor inhibitors may be employed in combination with the compounds of the invention in any of the compositions and methods/uses described herein. Trastuzumab (Herceptin®) is an example of an anti-erbB2 antibody inhibitor of growth factor function. One example of an anti-erbB1 antibody inhibitor of growth factor function is cetuximab (Erbitux™, C225). Bevacizumab (Avastin®) is an example of a monoclonal antibody directed against VEGFR. Examples of small molecule inhibitors of epidermal growth factor receptors include but are not limited to lapatinib (Tykerb®) and erlotinib (TARCEVA®). Imatinib mesylate (GLEEVEC®) is one example of a PDGFR inhibitor. Examples of VEGFR inhibitors include pazopanib (Votrient®), ZD6474, AZD2171, PTK787, sunitinib and sorafenib.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti - cancer agents that operate at the G₂/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2*R*,3*S*)-*N*-benzoyl-3-phenylisoserine; is a natural diterpene product isolated from the Pacific yew tree *Taxus brevifolia* and is commercially available as an injectable solution TAXOL®. It is a member of the taxane family of terpenes. It was first isolated in 1971 by Wani et al. J. Am. Chem, Soc., 93:2325 (1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77:1561-1565 (1980); Schiff et al., Nature, 277:665-667 (1979); Kumar, J. Biol, Chem, 256: 10435-10441 (1981). For a review of synthesis and anticancer activity of some paclitaxel derivatives see: D. G. I. Kingston et al., Studies in Organic Chemistry vol. 26, entitled "New trends in Natural Products Chemistry 1986", Attaur-Rahman, P.W. Le Quesne, Eds. (Elsevier, Amsterdam, 1986) pp 219-235.

Paclitaxel has been approved for clinical use in the treatment of refractory ovarian cancer in the United States (Markman et al., Yale Journal of Biology and Medicine, 64:583, 1991; McGuire et al., Ann. Int. Med., 111:273,1989) and for the treatment of breast cancer (Holmes et al., J. Nat. Cancer Inst., 83:1797,1991.). It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide 1998) related to the duration of dosing above a threshold concentration (50nM) (Kearns, C.M. et. al., Seminars in Oncology, 3(6) p.16-23, 1995).

Docetaxel, (2*R*,3*S*)-*N*-carboxy-3-phenylisoserine *N-tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel *q.v.*, prepared using a natural precursor, 10-deacetyl-baccatin III, extracted from the needle of the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo aquation and form intra- and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as PLATINOL® as an injectable solution. Cisplatin is primarily indicated in the treatment of metastatic testicular and ovarian cancer and advanced bladder cancer. The primary dose limiting side effects of cisplatin are nephrotoxicity, which may be controlled by hydration and diuresis, and ototoxicity.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced ovarian carcinoma. Bone marrow suppression is the dose limiting toxicity of carboplatin.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias. Alopecia, nausea, vomiting and leukopenia are the most common dose limiting side effects of cyclophosphamide.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease. Bone marrow suppression is the most common dose limiting side effect of chlorambucil.

Busulfan, 1,4-butanediol dimethanesulfonate, is commercially available as MYLERAN® TABLETS. Busulfan is indicated for the palliative treatment of chronic myelogenous leukemia. Bone marrow suppression is the most common dose limiting side effects of busulfan.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease. Nausea, vomiting, and anorexia are the most common dose limiting side effects of dacarbazine.

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also known as Actinomycin D, is commercially available in injectable form as COSMEGEN®. Dactinomycin is indicated for the treatment of Wilm's tumor and rhabdomyosarcoma. Nausea, vomiting, and anorexia are the most common dose limiting side effects of dactinomycin.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma. Myelosuppression is the most common dose limiting side effect of daunorubicin.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas. Myelosuppression is the most common dose limiting side effect of doxorubicin.

Bleomycin, a mixture of cytotoxic glycopeptide antibiotics isolated from a strain of *Streptomyces verticillus,* is commercially available as BLENOXANE®. Bleomycin is indicated as a palliative treatment, as a single agent or in combination with other agents, of squamous cell carcinoma, lymphomas, and testicular carcinomas. Pulmonary and cutaneous toxicities are the most common dose limiting side effects of bleomycin.

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as a single agent or in combination with other chemotherapy agents in the treatment of testicular and non-small cell lung cancers. Myelosuppression is the most common side effect of etoposide. The incidence of leukopenialeukopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON® and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leukopenialeukopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2(1*H*)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leukopenialeukopenia, thrombocytopenia, and mucositis.

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppression and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID®. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leukopenialeukopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Gemcitabine, 2'-deoxy-2', 2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer. Myelosuppression, including leukopenialeukopenia, thrombocytopenia, and anemia, is the most common dose limiting side effect of gemcitabine administration.

Methotrexate, *N*-[4[[(2,4-diamino-6-pteridinyl)methyl]methylamino]benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder. Myelosuppression (leukopenia, thrombocytopenia, and anemia) and mucositis are expected side effect of methotrexate administration.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCl, (4S)-4,1 1-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR®.

Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA: irintecan or SN-38 ternary complex with replication enzymes. Irinotecan is indicated for treatment of metastatic cancer of the colon or rectum. The dose limiting side effects of irinotecan HCl are myelo suppression, including neutropenia, and GI effects, including diarrhea.

Topotecan HCl, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer. The dose limiting side effect of topotecan HCl is myelo suppression, primarily neutropenia.

In addition, the compounds of Formula (I) may be used in combination with one or more other agents that may be useful in the treatment or cure of HIV.

Examples of such agents include, but are not limited to:
Nucleotide reverse transcriptase inhibitors such as zidovudine, didanosine, lamivudine, zalcitabine, abacavir, stavudine, adefovir, adefovir dipivoxil, fozivudine, todoxil, emtricitabine, alovudine, amdoxovir, elvucitabine, and similar agents;
Non-nucleotide reverse transcriptase inhibitors (including an agent having antioxidation activity such as immunocal, oltipraz, etc.) such as nevirapine, delavirdine, efavirenz, loviride, immunocal, oltipraz, capravirine, lersivirine, GSK2248761, TMC-278, TMC-125, etravirine, and similar agents;
Protease inhibitors such as saquinavir, ritonavir, indinavir, nelfinavir, amprenavir, fosamprenavir, brecanavir, darunavir, atazanavir, tipranavir, palinavir, lasinavir, and similar agents;
Entry, attachment and fusion inhibitors such as enfuvirtide (T-20), T-1249, PRO-542, PRO-140, TNX-355, BMS-806, BMS-663068 and BMS-626529, 5-Helix and similar agents;
Integrase inhibitors such as raltegravir, elvitegravir, dolutegravir, cabotegravir and similar agents;
Maturation inhibitors such as PA-344 and PA-457, and similar agents; and
CXCR4 and/or CCR5 inhibitors such as vicriviroc (Sch-C), Sch-D, TAK779, maraviroc (UK 427,857), TAK449, as well as those disclosed in WO 02/74769, WO2004/054974,

WO2004/055012, WO2004/4055010, WO2004/055016, WO2004/055011, andWO2004/054581, and similar agents.

Further examples where_the compounds of the present invention may be used in combination with one or more agents useful in the prevention or treatment of HIV are found in Table 1.

**Table 1:**

| **FDA Approval** | **Brand Name** | **Generic Name** | **Manufacturer** |
|---|---|---|---|
| ***Nucleoside Reverse Transcriptase Inhibitors (NRTIs)*** | | | |
| 1987 | Retrovir | zidovudine, azidothymidine, AZT, ZDV | GlaxoSmithKline |
| 1991 | Videx | didanosine, dideoxyinosine, ddI | Bristol-Myers Squibb |
| 1992 | Hivid | zalcitabine, dideoxycytidine, ddC | Roche Pharmaceuticals |
| 1994 | Zerit | stavudine, d4T | Bristol- Myers Squibb |
| 1995 | Epivir | lamivudine, 3TC | GlaxoSmithKline |
| 1997 | Combivir | lamivudine + zidovudine | GlaxoSmithKline |
| 1998 | Ziagen | abacavir sulfate, ABC | GlaxoSmithKline |
| 2000 | Trizivir | abacavir+ lamivudine+ zidovudine | GlaxoSmithKline |
| 2000 | Videx EC | enteric coated didanosine, ddI EC | Bristol-Myers Squibb |
| 2001 | Viread | tenofovir disoproxil fumarate, TDF | Gilead Sciences |
| 2003 | Emtriva | emtricitabine, FTC | Gilead Sciences |
| 2004 | Epzicom | abacavir+ lamivudine | GlaxoSmithKline |
| 2004 | Truvada | emtricitabine + tenofovir disoproxil fumarate | Gilead Sciences |

| ***Non-Nucleosides Reverse Transcriptase Inhibitors (NNRTIs)*** | | | |
|---|---|---|---|
| 1996 | Viramune | nevirapine, NVP | Boehringer Ingelheim |
| 1997 | Rescriptor | delavirdine, DLV | Pfizer |
| 1998 | Sustiva | efavirenz, EFV | Bristol- Myers Squibb |
| 2008 | Intelence | Etravirine | Tibotec Therapeutics |

| ***Protease Inhibitors (Pis)*** | | | |
|---|---|---|---|
| 1995 | Invirase | saquinavir mesylate, SQV | Roche Pharmaceuticals |
| 1996 | Norvir | ritonavir, RTV | Abbott Laboratories |
| 1996 | Crixivan | indinavir, IDV | Merck |
| 1997 | Viracept | nelfinavir mesylate, NFV | Pfizer |
| 1997 | Fortovase | saquinavir (no longer marketed) | Roche Pharmaceuticals |
| 1999 | Agenerase | amprenavir, APV | GlaxoSmithKline |
| 2000 | Kaletra | lopinavir+ ritonavir, LPV/RTV | Abbott Laboratories |
| 2003 | Reyataz | atazanavir sulfate, ATV | Bristol-Myers Squibb |
| 2003 | Lexiva | fosamprenavir calcium, FOS-APV | GlaxoSmithKline |
| 2005 | Aptivus | tripranavir, TPV | Boehringer Ingelheim |
| 2006 | Prezista | Darunavir | Tibotec Therapeutics |

| ***Fusion Inhibitors*** | | | |
|---|---|---|---|
| 2003 | Fuzeon | Enfuvirtide, T-20 | Roche Pharmaceuticals & Trimeris |

| ***Entry Inhibitors*** | | | |
|---|---|---|---|
| 2007 | Selzentry | Maraviroc | Pfizer |

| ***Integrase Inhibitors*** | | | |
|---|---|---|---|
| 2007 | Isentress | Raltegravir | Merck |
| 2013 | Tivicay | Dolutegravir | ViiV Healthcare |
| --- | --- | Cabotegravir | |

The scope of combinations of compounds of this invention with HIV agents is not limited to those mentioned above, but includes in principle any combination with any pharmaceutical composition useful for the cure or treatment of HIV. As noted, in such combinations the compounds of the present invention and other HIV agents may be administered separately or in conjunction. In addition, one agent may be prior to, concurrent to, or subsequent to the administration of other agent(s).

Compounds of the present invention may be used in combination with one or more agents useful as pharmacological enhancers as well as with or without additional compounds for the prevention or treatment of HIV. Examples of such pharmacological enhancers (or pharmakinetic boosters) include, but are not limited to, ritonavir, GS-9350, and SPI-452. Ritonavir is 10-hydroxy-2-methyl-5-(1-methyethyl)-1-1[2-(1-methylethyl)-4-thiazolyl]-3,6-dioxo-8,11-bis(phenylmethyl)-2,4,7,12-tetraazatridecan-13-oic acid, 5-thiazolylmethyl ester, [5S-(5S*,8R*,10R*,11R*)] and is available from Abbott Laboratories of Abbott park, Illinois, as Norvir. Ritonavir is an HIV protease inhibitor indicated with other antiretroviral agents for the treatment of HIV infection. Ritonavir also inhibits P450 mediated drug metabolism as well as the P-gycoprotein (Pgp) cell transport system, thereby resulting in increased concentrations of active compound within the organism. GS-9350 is a compound being developed by Gilead Sciences of Foster City California as a pharmacological enhancer. SPI-452 is a compound being developed by Sequoia Pharmaceuticals of Gaithersburg, Maryland, as a pharmacological enhancer.

Provided herein are the compounds of the present invention for use in the treatment or prevention of autoimmune and inflammatory conditions and diseases that can be improved by inhibiting EZH1 and / or EZH2 and thereby, e.g., modulate the level of expression of methylation activated and methylation repressed target genes, or modulate the activity of signalling proteins.

Inflammation represents a group of vascular, cellular and neurological responses to trauma. Inflammation can be characterised as the movement of inflammatory cells such as monocytes, neutrophils and granulocytes into the tissues. This is usually associated with reduced endothelial barrier function and oedema into the tissues. Inflammation can be classified as either acute or chronic. Acute inflammation is the initial response of the body to harmful stimuli and is achieved by the increased movement of plasma and leukocytes from the blood into the injured tissues. A cascade of biochemical event propagates and matures the inflammatory response, involving the local vascular system, the immune system, and various cells within the injured tissue. Prolonged inflammation, known as chronic inflammation, leads to a progressive shift in the type of cells which are present at the site of inflammation and is characterised by simultaneous destruction and healing of the tissue from the inflammatory process.

When occurring as part of an immune response to infection or as an acute response to trauma, inflammation can be beneficial and is normally self-limiting. However, inflammation can be detrimental under various conditions. This includes the production of excessive inflammation in response to infectious agents, which can lead to significant organ damage and death (for example, in the setting of sepsis). Moreover, chronic inflammation is generally deleterious and is at the root of numerous chronic diseases, causing severe and irreversible damage to tissues. In such settings, the immune response is often directed against self-tissues (autoimmunity), although chronic responses to foreign entities can also lead to bystander damage to self tissues.

The aim of anti-inflammatory therapy is therefore to reduce this inflammation, to inhibit autoimmunity when present and to allow for the physiological process or healing and tissue repair to progress.

The agents may be used to treat inflammation of any tissue and organs of the body, including musculoskeletal inflammation, vascular inflammation, neural inflammation, digestive system inflammation, ocular inflammation, inflammation of the reproductive system, and other inflammation, as exemplified below.

Musculoskeletal inflammation refers to any inflammatory condition of the musculoskeletal system, particularly those conditions affecting skeletal joints, including joints of the hand, wrist, elbow, shoulder, jaw, spine, neck, hip, knew, ankle, and foot, and conditions affecting tissues connecting muscles to bones such as tendons. Examples of musculoskeletal inflammation which may be treated with compounds of the invention include arthritis (including, for example, osteoarthritis, psoriatic arthritis, ankylosing spondylitis, acute and chronic infectious arthritis, arthritis associated with gout and pseudogout, and juvenile idiopathic arthritis), tendonitis, synovitis, tenosynovitis, bursitis, fibrositis (fibromyalgia), epicondylitis, myositis, and osteitis (including, for example, Paget's disease, osteitis pubis, and osteitis fibrosa cystic).

Ocular inflammation refers to inflammation of any structure of the eye, including the eye lids. Examples of ocular inflammation which may be treated in this invention include blepharitis, blepharochalasis, conjunctivitis, dacryoadenitis, keratitis, keratoconjunctivitis sicca (dry eye), scleritis, trichiasis, and uveitis.

Examples of inflammation of the nervous system which may be treated in this invention include encephalitis, Guillain-Barre syndrome, meningitis, neuromyotonia, narcolepsy, multiple sclerosis, myelitis and schizophrenia.

Examples of inflammation of the vasculature or lymphatic system which may be treated in this invention include arthrosclerosis, arthritis, phlebitis, vasculitis, and lymphangitis.

Examples of inflammatory conditions of the digestive system which may be treated in this invention include cholangitis, cholecystitis, enteritis, enterocolitis, gastritis, gastroenteritis, ileitis, and proctitis.

Examples of inflammatory conditions of the reproductive system which may be treated in this invention include cervicitis, chorioamnionitis, endometritis, epididymitis, omphalitis, oophoritis, orchitis, salpingitis, tubo-ovarian abscess, urethritis, vaginitis, vulvitis, and vulvodynia.

The agents may be used to treat autoimmune conditions having an inflammatory component. Such conditions include acute disseminated alopecia universalise, Behcet's disease, Chagas' disease, chronic fatigue syndrome, dysautonomia, encephalomyelitis, ankylosing spondylitis, aplastic anemia, hidradenitis suppurativa, autoimmune hepatitis, autoimmune oophoritis, celiac disease, Crohn's disease, diabetes mellitus type 1, giant cell arteritis, goodpasture's syndrome, Grave's disease, Guillain-Barre syndrome, Hashimoto's disease, Henoch-Schonlein purpura, Kawasaki's disease, lupus erythematosus, microscopic colitis, microscopic polyarteritis, mixed connective tissue disease, multiple sclerosis, myasthenia gravis, opsocionus myoclonus syndrome, optic neuritis, ord's thyroiditis, pemphigus, polyarteritis nodosa, polymyalgia, Reiter's syndrome, Sjogren's syndrome, temporal arteritis, Wegener's granulomatosis, warm autoimmune haemolytic anemia, interstitial cystitis, lyme disease, morphea, sarcoidosis, scleroderma, ulcerative colitis, and vitiligo.

The agents may be used to treat T-cell mediated hypersensitivity diseases having an inflammatory component. Such conditions include contact hypersensitivity, contact dermatitis (including that due to poison ivy), uticaria, skin allergies, respiratory allergies (hayfever, allergic rhinitis) and gluten-sensitive enteropathy (Celliac disease).

Other inflammatory conditions which may be treated in this invention include, for example, appendicitis, dermatitis, dermatomyositis, endocarditis, fibrositis, gingivitis, glossitis, hepatitis, hidradenitis suppurativa, iritis, laryngitis, mastitis, myocarditis, nephritis, otitis, pancreatitis, parotitis, percarditis, peritonoitis, pharyngitis, pleuritis, pneumonitis, prostatistis, pyelonephritis, and stomatisi, transplant rejection (involving organs such as kidney, liver, heart, lung, pancreas (e.g., islet cells), bone marrow, cornea, small bowel, skin allografts, skin homografts, and heart valve xengrafts, sewrum sickness, and graft vs host disease), acute pancreatitis, chronic pancreatitis, acute respiratory distress syndrome, Sexary's syndrome, congenital adrenal hyperplasis, nonsuppurative thyroiditis, hypercalcemia associated with cancer, pemphigus, bullous dermatitis herpetiformis, severe erythema multiforme, exfoliative dermatitis, seborrheic dermatitis, seasonal or perennial allergic rhinitis, bronchial asthma, contact dermatitis, astopic dermatitis, drug hypersensistivity reactions, allergic conjunctivitis, keratitis, herpes zoster ophthalmicus, iritis and oiridocyclitis, chorioretinitis, optic neuritis, symptomatic sarcoidosis, fulminating or disseminated pulmonary tuberculosis chemotherapy, idiopathic thrombocytopenic purpura in adults, secondary thrombocytopenia in adults, acquired (autoimmune) haemolytic anemia, leukaemia and lymphomas in adults, acute leukaemia of childhood, regional enteritis, autoimmune vasculitis, multiple sclerosis, chronic obstructive pulmonary disease, solid organ transplant rejection, sepsis.

Preferred treatments include any one of treatment of transplant rejection, psoriatic arthritis, multiple sclerosis, Type 1 diabetes, asthma, systemic lupus erythematosis, chronic pulmonary disease, and inflammation accompanying infectious conditions (e.g., sepsis).

Pharmaceutical compositions may be presented in unit dose forms containing a predetermined amount of active ingredient per unit dose. Such a unit may contain, for example, 0.5 mg to 1 g, preferably 1 mg to 700 mg, more preferably 5 mg to 100 mg of a compound of the Formula (I), depending on the condition being treated, the route of administration and the age, weight and condition of the patient, or pharmaceutical compositions may be presented in unit dose forms containing a predetermined amount of active ingredient per unit dose. Preferred unit dosage compositions are those containing a daily dose or sub-dose, as herein above recited, or an appropriate fraction thereof, of an active ingredient. Furthermore, such pharmaceutical compositions may be prepared by any of the methods well known in the pharmacy art.

Pharmaceutical compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by bringing into association a compound of formal (I) with the carrier(s) or excipient(s).

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of a compound of Formula (I). Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit pharmaceutical compositions for oral administration can be microencapsulated. The formulation can also be prepared to prolong or sustain the release as for example by coating or embedding particulate material in polymers, wax or the like.

Pharmaceutical compositions adapted for rectal administration may be presented as suppositories or as enemas.

Pharmaceutical compositions adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the composition isotonic with the blood of the intended recipient; and aqueous and non-aqueous sterile suspensions which may include suspending agents and thickening agents. The pharmaceutical compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the pharmaceutical compositions may include other agents conventional in the art having regard to the type of formulation in question, for example those suitable for oral administration may include flavoring agents.

A therapeutically effective amount of a compound of the present invention will depend upon a number of factors including, for example, the age and weight of the intended recipient, the precise condition requiring treatment and its severity, the nature of the formulation, and the route of administration, and will ultimately be at the discretion of the attendant prescribing the medication. However, an effective amount of a compound of Formula (I) for the treatment of anemia will generally be in the range of 0.001 to 100 mg/kg body weight of recipient per day, suitably in the range of .01 to 10 mg/kg body weight per day. For a 70 kg adult mammal, the actual amount per day would suitably be from 7 to 700 mg and this amount may be given in a single dose per day or in a number (such as two, three, four, five or six) of sub-doses per day such that the total daily dose is the same. An effective amount of a salt or solvate, etc., may be determined as a proportion of the effective amount of the compound of Formula (I) *per se.* It is envisaged that similar dosages would be appropriate for treatment of the other conditions referred to above.

### DEFINITIONS

Terms are used within their accepted meanings. The following definitions are meant to clarify, but not limit, the terms defined.

As used herein, the term "alkyl" represents a saturated, straight or branched hydrocarbon moiety having the specified number of carbon atoms. The term "(C₁-C₄)alkyl" refers to an alkyl moiety containing from 1 to 4 carbon atoms. Exemplary alkyls include methyl, ethyl, *n*-propyl, isopropyl, *n*-butyl, isobutyl, *s*-butyl, and *t*-butyl.

"Alkoxy" refers to a group containing an alkyl radical, defined hereinabove, attached through an oxygen linking atom. The term "(C₁-C₄)alkoxy" refers to a straight- or branched-chain hydrocarbon radical having at least 1 and up to 4 carbon atoms attached through an oxygen linking atom. Exemplary "(C₁-C₄)alkoxy" groups useful in the present invention include methoxy, ethoxy, *n*-propoxy, isopropoxy, *n*-butoxy, *s*-butoxy, isobutoxy, and *t*-butoxy.

When the term "alkyl" is used in combination with other substituent groups, such as "halo(C₁-C₄)alkyl", "hydroxy(C₁-C₄)alkyl", or "(C₁-C₄)alkoxy(C₁-C₄)alkyl", the term "alkyl" is intended to encompass a divalent straight or branched-chain hydrocarbon radical, wherein the point of attachment is through the alkyl moiety. The term "halo(C₁-C₄)alkyl" is intended to mean a radical having one or more halogen atoms, which may be the same or different, at one or more carbon atoms of an alkyl moiety containing from 1 to 4 carbon atoms, which is a straight or branched-chain carbon radical. Examples of "halo(C₁-C₄)alkyl" groups useful in the present invention include, but are not limited to, -CF₃ (trifluoromethyl), -CCl₃ (trichloromethyl), 1,1-difluoroethyl, 2-fluoro-2-methylpropyl, 2,2-difluoropropyl, 2,2,2-trifluoroethyl, and hexafluoroisopropyl. Examples of "hydroxy(C₁-C₄)alkyl" groups useful in the present invention include, but are not limited to, hydroxymethyl, hydroxyethyl, and hydroxyisopropyl. Examples of "(C₁-C₄)alkoxy(C₁-C₄)alkyl" groups useful in the present invention include, but are not limited to, methoxymethyl, methoxyethyl, methoxyisopropyl, ethoxymethyl, ethoxyethyl, ethoxyisopropyl, isopropoxymethyl, isopropoxyethyl, isopropoxyisopropyl, *t-*butoxymethyl, *t*-butoxyethyl, and *t*-butoxyisopropyl.

When the term "alkoxy" is used in combination with other substituent groups, such as "hydroxy(C₂-C₄)alkoxy-" or "(C₁-C₄)alkoxy(C₂-C₄)alkoxy-", the term "alkoxy-" is intended to encompass a divalent straight or branched-chain hydrocarbon radical attached through an oxygen linking atom, wherein the other substituent is attached to the alkyl moiety of the alkoxy group. Examples of "hydroxy(C₂-C₄)alkoxy-" groups useful in the present invention include, but are not limited to, hydroxyethoxy, hydroxypropoxy, and hydroxybutoxy. Examples of "(C₁-C₄)alkoxy(C₂-C₄)alkoxy-" groups useful in the present invention include, but are not limited to, methoxyethoxy, methoxypropoxy, methoxybutoxy, ethoxyethoxy, ethoxypropoxy, ethoxybutoxy, isopropoxyethoxy, isopropoxypropoxy, isopropoxybutoxy, *t*-butoxyethoxy, *t*-butoxypropoxy, and t-butoxybutoxy.

As used herein, the term "cycloalkyl" refers to a non aromatic, saturated, cyclic hydrocarbon ring containing the specified number of carbon atoms. The term "(C₃-C₆)cycloalkyl" refers to a non aromatic cyclic hydrocarbon ring having from three to six ring carbon atoms. Exemplary "(C₃-C₆)cycloalkyl" groups useful in the present invention include cyclopropyl, cyclobutyl, cyclopentyl, and cyclohexyl.

The terms "halogen" and "halo" represent fluoro, chloro, bromo, or iodo substituents. "Hydroxy" or "hydroxyl" is intended to mean the radical -OH. "Cyano" is intended to mean the radical -CN. "Oxo" represents a double-bonded oxygen moiety; for example, if attached directly to a carbon atom forms a carbonyl moiety (C=O).

As used herein, the term "optionally" means that the subsequently described event(s) may or may not occur, and includes both event(s) that occur and event(s) that do not occur.

As used herein, the term "treatment" refers to alleviating the specified condition, eliminating or reducing one or more symptoms of the condition, slowing or eliminating the progression of the condition, and delaying the reoccurrence of the condition in a previously afflicted or diagnosed patient or subject.

"Cure" or "Curing" a disease in a patient is used to denote the eradication, stoppage, halt or end of the human immunodeficiency virus or symptoms, or the progression of the symptoms or virus, for a defined period. As an example, in one embodiment, "cure" or "curing" refers to a therapeutic administration or a combination of administrations that alone or in combination with one or more other compounds induces and maintains sustained viral control (undetectable levels of plasma viremia by, e.g., a polymerase chain reaction (PCR) test, a bDNA (branched chain DNA) test or a NASBA (nucleic acid sequence based amplification) test) of human immunodeficiency virus after a minimum of two years without any other therapeutic intervention. The above PCR, bDNA and NASBA tests are carried out using techniques known and familiar to one skilled in the art. As an example, the eradication, stoppage, halt or end of the human immunodeficiency virus or symptoms, or the progression of the symptoms or virus, may be sustained for a minimum of two years.

As used herein, the term "effective amount" means that amount of a drug or pharmaceutical agent that will elicit the biological or medical response of a tissue, system, animal, or human that is being sought, for instance, by a researcher or clinician.

The term "therapeutically effective amount" means any amount which, as compared to a corresponding subject who has not received such amount, results in improved treatment, healing, or amelioration of a disease, disorder, or side effect, or a decrease in the rate of advancement of a disease or disorder. The term also includes within its scope amounts effective to enhance normal physiological function. For use in therapy, therapeutically effective amounts of a compound of Formula (I), as well as salts thereof, may be administered as the raw chemical. Additionally, the active ingredient may be presented as a pharmaceutical composition.

### Compound Preparation

### Abbreviations

- AcOH: acetic acid
- Ag₂O: silver oxide
- Ar: Ar gas
- BnCl: benzyl chloride
- Boc: *tert*-butyloxycarbonyl
- Boc₂O: di-*tert*-butyl dicarbonate
- Bu₄NCl: tetrabutylammonium chloride
- CHCl₃: chloroform
- CH₃CN: acetonitrile
- CH₃NO₂: nitromethane
- DCE: 1,2-dichloroethane
- DCM: dichloromethane
- DIBAL-H: diisobutylaluminium hydride
- DIPEA: diisopropylethylamine
- DMF: *N*,*N-*dimethylformamide
- DMSO: dimethyl sulfoxide
- EDC: 1-ethyl-3-(3-dimethylaminopropyl)carbodiimide
- ES: electrospray
- Et₃N: triethylamine
- Et₂O: diethyl ether
- EtOAc: ethyl acetate
- EtOH: ethanol
- h: hour(s)
- H₂: hydrogen gas
- HATU: 1-[bis(dimethylamino)methylene]-1*H*-1,2,3-triazolo[4,5-*b*]pyridinium 3-oxid hexafluorophosphate
- HCl: hydrochloric acid
- H₂O: water
- HOAt: 1-hydroxy-7-azabenzotriazole
- H₂SO₄: sulfuric acid
- HPLC: high-performance liquid chromatography
- In(OTf)₃: indium (III) trifluoromethanesulfonate
- *i*-PrOH: isopropanol
- [Ir(OMe)(1,5-cod)]₂: (1,5-cyclooctadiene)(methoxy)iridium(I) dimer
- KF: potassium fluoride
- KOtBu: potassium *tert*-butoxide
- LCMS: liquid chromatography mass spectrometry
- LiAlH₄: lithium aluminum hydride
- LiBH₄: lithium borohydride
- LiClO₄: lithium perchlorate
- MeOH: methanol
- MgSO₄: magnesium sulfate
- min: minute(s)
- M: molar
- MS: mass spectrometry
- N: normal
- N₂: nitrogen gas
- NaBH₄: sodium borohydride
- NaBH₃CN: sodium cyanoborohydride
- NaBH(OAc)₃: sodium triacetoxyborohydride
- Na₂CO₃: sodium carbonate
- NaHCO₃: sodium bicarbonate
- NaHMDS: sodium *bis*(trimethylsilyl)amide
- NaOH: sodium hydroxide
- Na₂SO₄: sodium sulphate
- NBS: *N*-bromosuccinimide
- NH₄Cl: ammonium chloride
- NH₄OAc: ammonium acetate
- NH₄OH: ammonium hydroxide
- NMM: *N*-methylmorpholine
- Pd/C: palladium on carbon
- P₂O₅: phosphorus pentoxide
- Pd(OAc)₂: palladium(II) acetate
- Pd(PPh₃)₄: tetrakis(triphenylphosphine)palladium(0)
- POCl₃: phosphoryl chloride
- (*R*,*R*)-[COD]Ir[cy₂PThrePHOX]: ((4*R*,5*R*)-(+)-*O*-[1-benzyl-1-(5-methyl-2-phenyl-4,5-dihydrooxazol-4-yl)-2-phenylethyl] (dicyclohexylphosphinite)(1,5-cyclooctadiene)iridium(I) tetrakis(3,5-bis(trifluoromethyl)phenylborate
- r.t.: room temperature
- sat.: saturated
- SOCl₂: thionyl chloride
- TBME: *tert*-butyl methyl ether
- TFA: trifluoroacetic acid
- THF: tetrahydrofuran
- TiCl₄: titanium(IV) chloride
- TMSCl: trimethylsilyl chloride

### Generic synthesis schemes

The compounds of this invention may be made by a variety of methods, including well-known standard synthetic methods. Illustrative general synthetic methods are set out below and then specific compounds of the invention are prepared in the working examples. The skilled artisan will appreciate that if a substituent described herein is not compatible with the synthetic methods described herein, the substituent may be protected with a suitable protecting group that is stable to the reaction conditions. The protecting group may be removed at a suitable point in the reaction sequence to provide a desired intermediate or target compound. In all of the schemes described below, protecting groups for sensitive or reactive groups are employed where necessary in accordance with general principles of synthetic chemistry. Protecting groups are manipulated according to standard methods of organic synthesis (T.W. Green and P.G.M. Wuts, (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of the present invention. Starting materials are commercially available or are made from commercially available starting materials using methods known to those skilled in the art.

Certain compounds of Formula (I) can be prepared according to Scheme 1 or analogous methods. Condensation of an appropriately substituted thiophenecarbaldehyde with nitromethane provides the corresponding nitrovinyl thiophene. Reduction of the nitrovinyl, followed by trapping of the resultant amine yields the corresponding urethane. Treatment of the urethane with POCl₃/POCl₅ furnishes the lactam. An iridium-mediated borylation, followed by a Suzuki coupling with an appropriately substituted triflate gives the corresponding coupled olefin. Reduction of the olefin, followed by alkylation of the lactam nitrogen with an appropriately substituted alkylhalide affords the elaborated thiophenelactam. Removal of the benzyl and tert-butylcarbonyl protecting groups provides the pyridone. Reductive amination with an appropriately substituted aldehyde affords compounds of Formula (I).

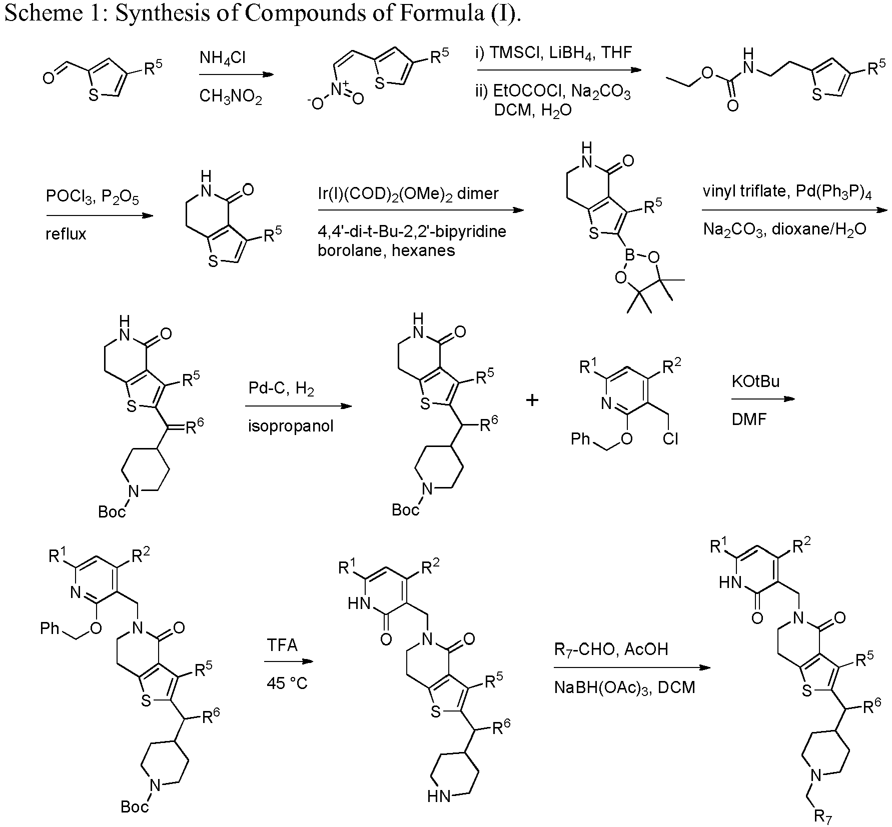

Additional compounds of Formula (I) can be prepared according to Scheme 2 or analogous methods. Esterification of an appropriately substituted thiophene-3-carboxylic acid provides the corresponding ester. An indium-mediated acylation reaction with an appropriately substituted anyhydride (or acylchloride) affords the 5-acylthiophene. A McMurray coupling with an appropriately substituted ketone affords the tetra-substituted olefin. Saponification of the ester, followed by coupling of the resultant carboxylic acid with an appropriately substituted amine affords the corresponding pyridones. Reductive alkylation with appropriately substituted aldehydes furnishes compounds of Formula (I).

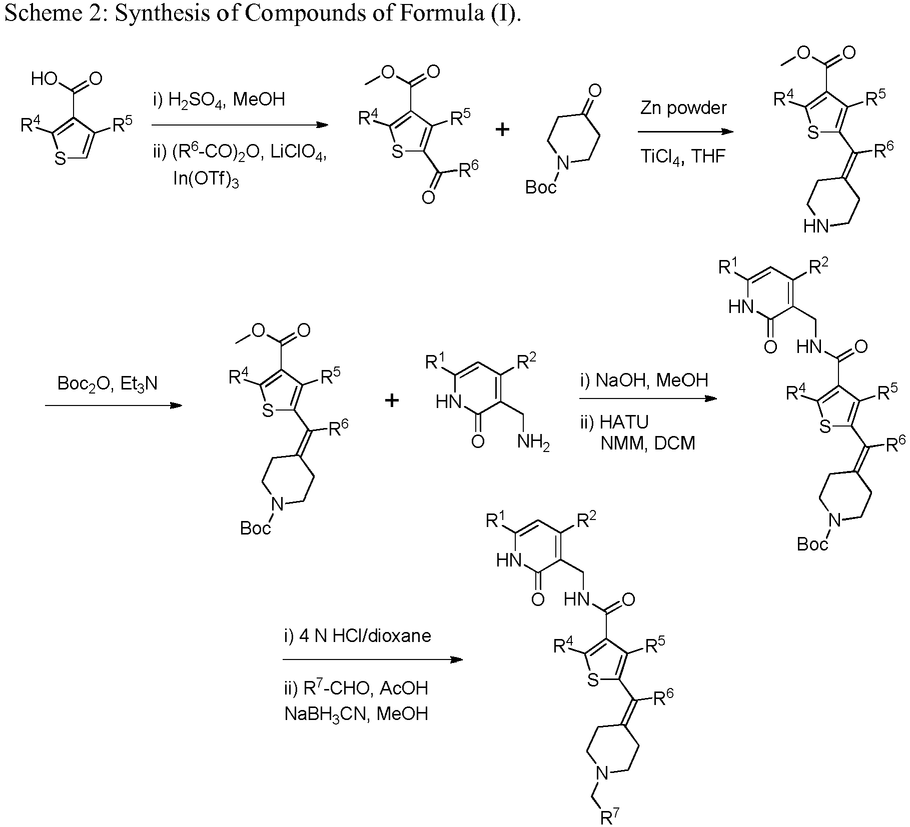

Similarly, compounds of Formula (I) can be prepared according to Scheme 3 or analogous methods. Esterification of an appropriately substituted thiophene-3-carboxylic acid provides the corresponding ester. An indium-mediated acylation reaction with an appropriately substituted anyhydride (or acylchloride) affords the 5-acylthiophene. A McMurray coupling with an appropriately substituted ketone affords the tetra-substituted olefin. Alkylation with appropriately substituted alkyl halides or reductive amination with appropriately substituted aldehydes furnishes the substituted derivatives. Saponification of the ester, followed by coupling of the resultant carboxylic acid with an appropriately substituted amine affords compounds of Formula (I).

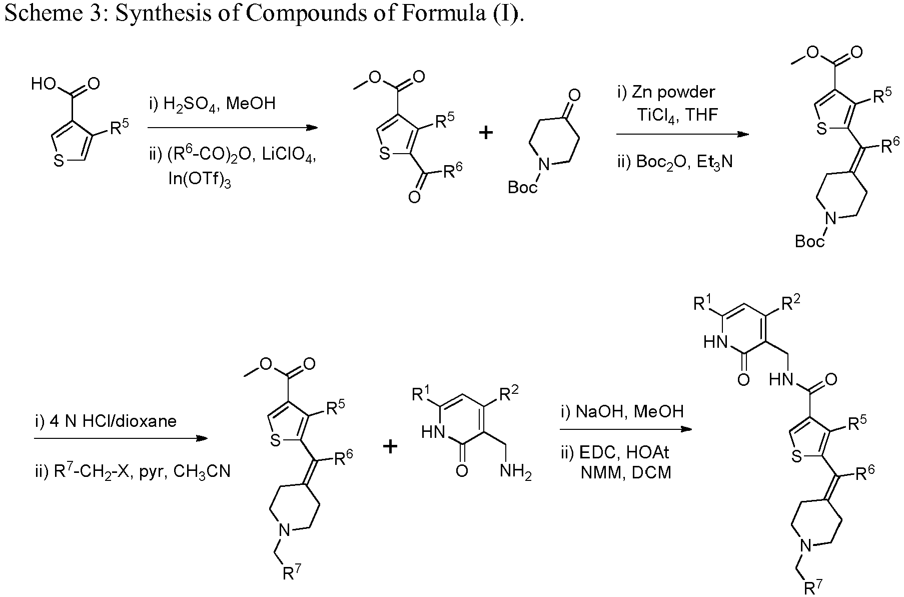

Additional compounds of Formula (I) can be prepared according to Scheme 4 or analogous methods. Formation of an appropriately substituted ketone from its corresponding Weinreb amide is accomplished with an appropriate Grignard (or alkyllithium) reagent. Formation of the corresponding vinyl triflate, followed by palladium-mediated coupling to an appropriately substituted bromothiophene affords the tri-substituted olefin. Reduction of the olefin, followed by alkylation with appropriately substituted alkyl halides or reductive amination with appropriately substituted aldehydes furnishes the substituted derivatives. Saponification of the ester, followed by coupling of the resultant carboxylic acid with an appropriately substituted amine affords compounds of Formula (I).

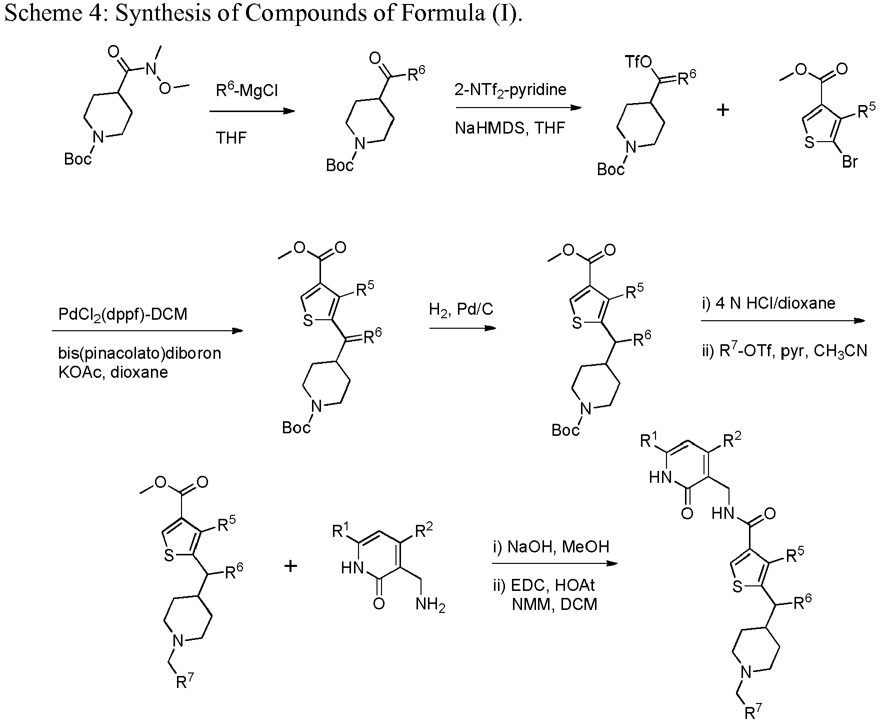

The 6-membered lactam intermediates in Scheme 1 can also be prepared according to Scheme 5 or analogous methods. An iridium-mediated borylation, followed by a Suzuki coupling with an appropriately substituted triflate gives the corresponding coupled olefin. An iridium-mediated asymmetric reduction of the olefin, followed by bromination provides the bromothiophene. A palladium-mediated Suzuki coupling of the bromothiophene with an isoxazole-boronate, followed by ring opening furnishes the nitrile. Reduction of the nitrile and intramolecular amide formation affords the elaborated thiophenelactam intermediate.

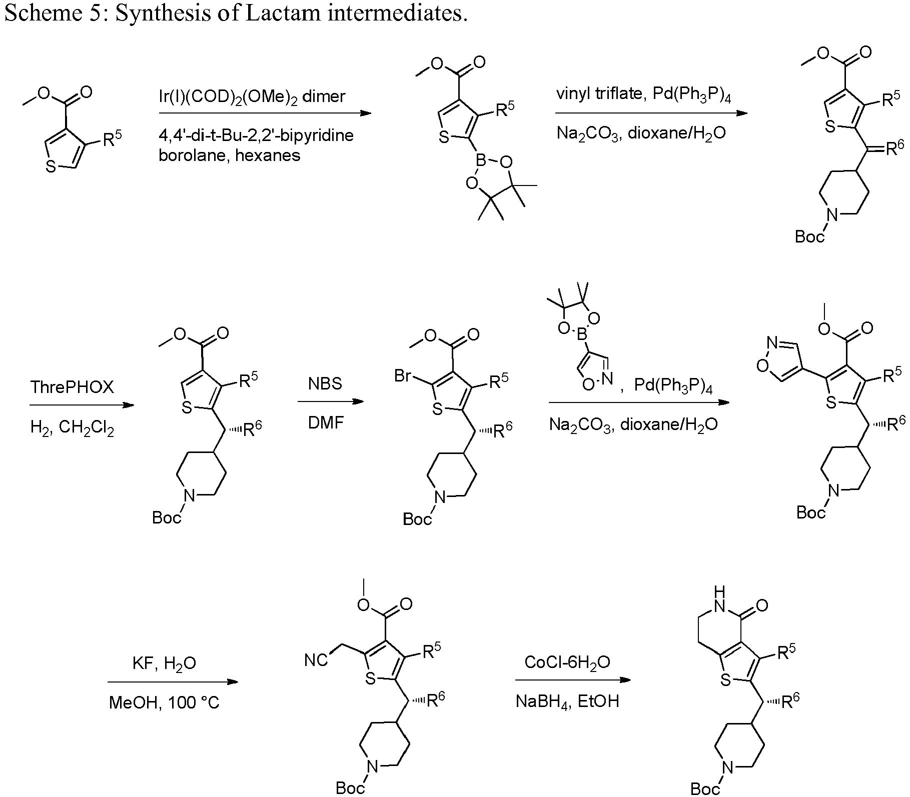

Additional compounds of Formula (I) can be prepared according to Scheme 6 or analogous methods. A palladium-mediated Heck coupling of the bromothiophene, prepared as in Scheme 5 above, with allyl alcohol, followed by a reductive amination of the resultant aldehyde with an appropriately substituted amine furnishes the secondary amine. Saponfication of the ester and intramolecular amide formation affords the elaborated thiophenelactam. Removal of the methyl and tert-butylcarbonyl protecting groups provides the pyridone. Reductive alkylation with an appropriately substituted aldehyde affords compounds of Formula (I).

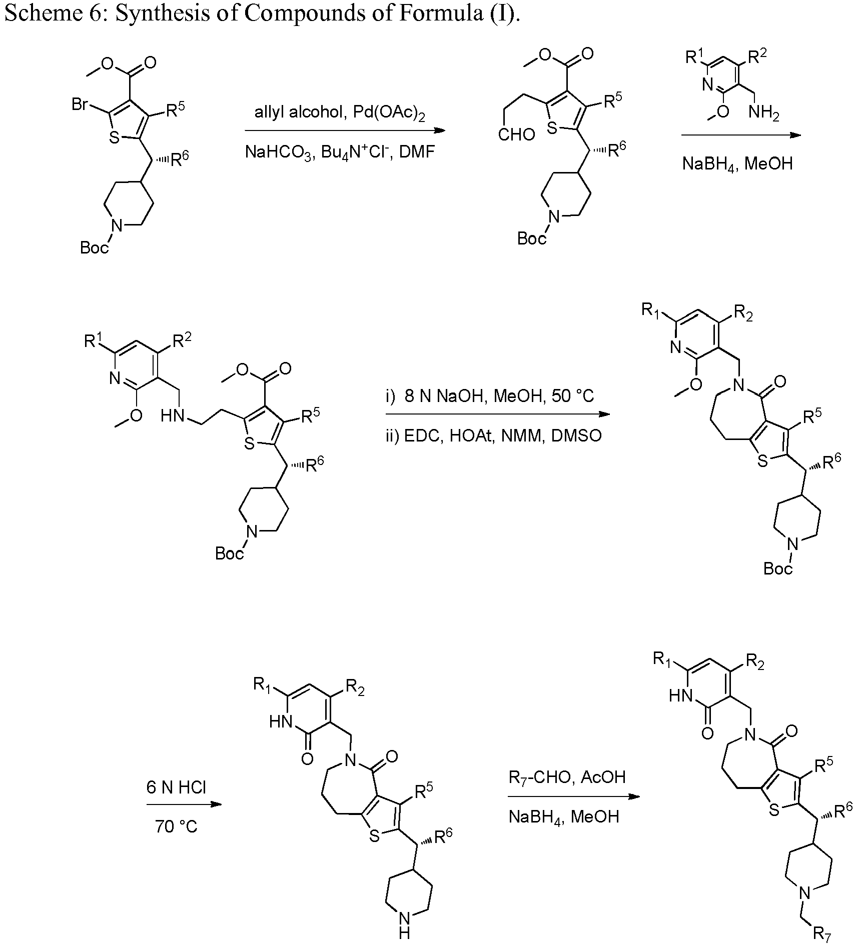

### EXPERIMENTALS

The following guidelines apply to all experimental procedures described herein. All reactions were conducted under a positive pressure of nitrogen using oven-dried glassware, unless otherwise indicated. Temperatures designated are external (i.e. bath temperatures), and are approximate. Air and moisture-sensitive liquids were transferred *via* syringe. Reagents were used as received. Solvents utilized were those listed as "anhydrous" by vendors. Molarities listed for reagents in solutions are approximate, and were used without prior titration against a corresponding standard. All reactions were agitated by stir bar, unless otherwise indicated. Heating was conducted using heating baths containing silicon oil, unless otherwise indicated. Reactions conducted by microwave irradiation (0 - 400 W at 2.45 GHz) were done so using a Biotage® Initiator 2.0 instrument with Biotage® microwave EXP vials (0.2 - 20 mL) and septa and caps. Irradiation levels utilized (i.e. high, normal, low) based on solvent and ionic charge were based on vendor specifications. Cooling to temperatures below -70 °C was conducted using dry ice/acetone or dry ice/2-propanol. Magnesium sulfate and sodium sulfate used as drying agents were of anhydrous grade, and were used interchangeably. Solvents described as being removed "*in vacuo*" or "under reduced pressure" were done so by rotary evaporation.

Preparative normal phase silica gel chromatography was carried out using either a Teledyne ISCO® CombiFlash Companion instrument with RediSep or ISCO® Gold silica gel cartridges (4 g-330 g), or an Analogix® IF280 instrument with SF25 silica gel cartridges (4 g - 3-00g), or a Biotage® SP1 instrument with HP® silica gel cartridges (10g-100 g). Purification by reverse phase HPLC was conducted using a YMC-pack column (ODS-A 75x30mm) as solid phase, unless otherwise noted. A mobile phase of 25mL/min A (CH₃CN-0.1% TFA): B (water-0.1% TFA), 10-80% gradient A (10 min) was utilized with UV detection at 214 nM, unless otherwise noted.

A PE Sciex® API 150 single quadrupole mass spectrometer (PE Sciex, Thornhill, Ontario, Canada) was operated using electrospray ionization in the positive ion detection mode. The nebulizing gas was generated from a zero air generator (Balston Inc., Haverhill, MA, USA) and delivered at 448.2 kPa (65 psi) and the curtain gas was high purity nitrogen delivered from a Dewar liquid nitrogen vessel at 344.7 kPa (50 psi). The voltage applied to the electrospray needle was 4.8 kV. The orifice was set at 25 V and mass spectrometer was scanned at a rate of 0.5 scan/sec using a step mass of 0.2 amu and collecting profile data.

Method A LCMS. Samples were introduced into the mass spectrometer using a CTC® PAL autosampler (LEAP Technologies, Carrboro, NC) equipped with a Hamilton® 10 uL syringe which performed the injection into a Valco 10-port injection valve. The HPLC pump was a Shimadzu® LC-10ADvp (Shimadzu Scientific Instruments, Columbia, MD) operated at 0.3 mL/min and a linear gradient 4.5% A to 90% B in 3.2 min. with a 0.4 min. hold. The mobile phase was composed of 100% (H₂O 0.02% TFA) in vessel A and 100% (CH₃CN 0.018% TFA) in vessel B. The stationary phase is Aquasil® (C18) and the column dimensions were 1 mm x 40 mm. Detection was by UV at 214 nm, evaporative light-scattering (ELSD) and MS.

Method B, LCMS. Alternatively, an Agilent® 1100 analytical HPLC system with an LC/MS was used and operated at 1 mL/min and a linear gradient 5% A to 100% B in 2.2 min with a 0.4 min hold. The mobile phase was composed of 100% (H₂O 0.02% TFA) in vessel A and 100% (CH₃CN 0.018% TFA) in vessel B. The stationary phase was Zobax® (C8) with a 3.5 um partical size and the column dimensions were 2.1 mm x 50 mm. Detection was by UV at 214 nm, evaporative light-scattering (ELSD) and MS.

Method C, LCMS. Alternatively, an MDSSCIEX® API 2000 equipped with a capillary column of (50 × 4.6 mm, 5 *µ*m) was used. HPLC was done on Agilent® 1200 series UPLC system equipped with column Zorbax® SB-C18 (50 × 4.6 mm, 1.8 *µ*m) eluting with CH₃CN: NH₄OAc buffer.

¹H-NMR spectra were recorded at 400 MHz using a Bruker® AVANCE 400 MHz instrument, with ACD Spect manager v. 10 used for reprocessing. Multiplicities indicated are: s=singlet, d=doublet, t=triplet, q=quartet, quint= quintet, sxt= sextet, m=multiplet, dd = doublet of doublets, dt=doublet of triplets etc. and br indicates a broad signal. All NMRs in DMSO-d₆ unless otherwise noted.

Analytical HPLC: Products were analyzed by Agilent® 1100 Analytical Chromatography system, with 4.5 x 75 mm Zorbax® XDB-C18 column (3.5 um) at 2 mL/min with a 4 min gradient from 5% CH₃CN (0.1% formic acid) to 95% CH₃CN (0.1% formic acid) in H₂O (0.1% formic acid) and a 1 min hold.

### Intermediates

### Intermediate 1

### a) 2-(Benzyloxy)-4,6-dimethylnicotinonitrile

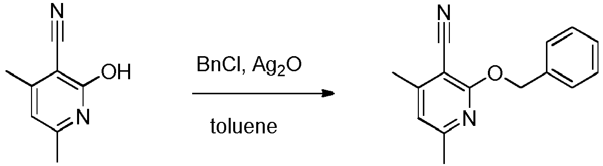

A solution of 2-hydroxy-4,6-dimethylnicotinonitrile (5 g, 33.7 mmol) in toluene (50 mL) was treated with BnCl (4.70 mL, 40.5 mmol) and Ag₂O (8.60 g, 37.1 mmol), then stirred at 110°C overnight. The reaction was filtered through Celite® and the solids were washed with DCM (2 x 100 mL). The combined organic layers were washed with brine (30 mL), filtered through Na₂SO₄ and concentrated *in vacuo* to give a residue. The residue was purified through a plug of silica with vacuum using 20-30% DCM in petroleum ether. The desired fractions were combined and concentrated to furnish 2-(benzyloxy)-4,6-dimethylnicotinonitrile (9 g, 35.9 mmol, >100% yield) as a white solid. ¹H NMR (400 MHz, CDCl₃) δ 7.47-7.56 (m, 2H), 7.31-7.43 (m, 3H), 6.72 (s, 1H), 5.51 (s, 2H), 2.48 (d, *J*=3.03 Hz, 6H). MS(ES) [M+H]⁺ 239.0.

### b) 2-(Benzyloxy)-4,6-dimethylnicotinaldehyde

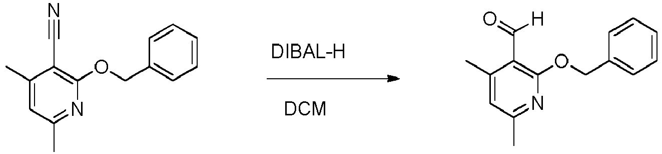

To a cooled (ice bath) solution of 2-(benzyloxy)-4,6-dimethylnicotinonitrile (9 g, 35.9 mmol) in DCM (100 mL) under an inert atmosphere was slowly added a solution of 1 M DIBAL-H in toluene (43.1 mL, 43.1 mmol) via syringe. The reaction was stirred at 0 °C for 20 min, at which time the ice-bath was removed and the reaction stirred at r.t. overnight. LCMS showed ∼14% starting material remained. An additional portion of 1 M DIBAL-H in toluene (10.76 mL, 10.76 mmol) was added and the reaction continued to stir at r.t. LCMS indicated the reaction was complete. The reaction was cooled (ice bath) and quenched with 1N HCl (50 mL). **Caution- exothermic. The reaction was stirred 30 min until the aluminum salts were free flowing. The reaction was neutralized with 2.5 N NaOH (∼15 mL, ∼pH7.5). The biphasic mixture was filtered and the filtrate washed with DCM (100 mL, 2X). The layers were separated and the aqueous was extracted with DCM (100 mL). The combined organic layers were washed with brine (30 mL), filtered through Na₂SO₄ and concentrated *in vacuo.* The residue was purified by flash chromatography (Column: 80 gram silica. Eluent: 0-5% EtOAc in Heptanes. Gradient: 15 min). The desired fractions were combined and concentrated *in vacuo* to give 2-(benzyloxy)-4,6-dimethylnicotinaldehyde (3.5 g, 14.36 mmol, 40.0% yield) as a fluffy white solid. ¹H NMR (400 MHz, CDCl₃) δ 10.58 (s, 1H), 7.49 (d, *J*=7.07 Hz, 2H), 7.31-7.44 (m, 3H), 6.67 (s, 1H), 5.54 (s, 2H), 2.59 (s, 3H), 2.50 (s, 3H). MS(ES) [M+H]⁺ 242.1, [M+Na]⁺ 264.0.

### c) (2-(Benzyloxy)-4,6-dimethylpyridin-3-yl)methanol

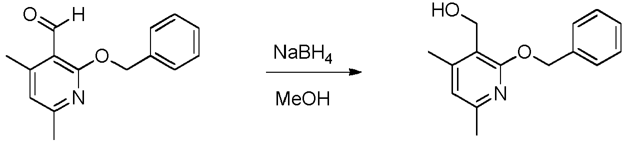

A suspention of 2-(benzyloxy)-4,6-dimethylnicotinaldehyde (3.46 g, 14.34 mmol) in MeOH (100 mL) was kept under inert atmosphere and cooled to 0 °C in an ice bath. To the stirred suspension was added NaBH₄ (0.651 g, 17.21 mmol) in two portions. The suspension went into solution after the first portion of borohydride was added. The reaction was stirred at 0 °C for 10 min, at which time the ice-bath was removed and the reaction stirred at r.t. overnight. The reaction solvent was removed *in vacuo* and the remaining white solid residue was partitioned between saturated NaHCO₃ (60 mL) and EtOAc (125 mL). The aqueous layer was extracted with EtOAc (125 mL). The combined organic layers were washed with brine (20 mL), filtered through Na₂SO₄ and concentrated *in vacuo* to give (2-(benzyloxy)-4,6-dimethylpyridin-3-yl)methanol (3.5 g, 14.39 mmol, 100% yield) as a colorless translucent oil, which was carried on to the next step without further purification. ¹H NMR (400 MHz, CDCl₃) δ 7.44-7.53 (m, 2H), 7.30-7.43 (m, 3H), 6.63 (s, 1H), 5.46 (s, 2H), 4.72 (s, 2H), 2.43 (s, 3H), 2.35 (s, 3H), 2.25 (br. s., 1H). MS(ES) [M+H]⁺ 244.1.

### d) 2-(Benzyloxy)-3-(chloromethyl)-4,6-dimethylpyridine

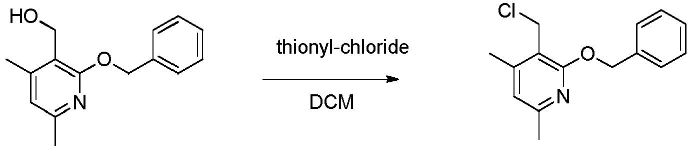

A suspention of (2-(benzyloxy)-4,6-dimethylpyridin-3-yl)methanol (3.5 g, 14.39 mmol) in DCM (70 mL) was kept under inert atmosphere and cooled to -40 °C in dry ice/CH₃CN bath for 30 min. To the chilled solution was added 2 M SOCl₂ in DCM (10.79 mL, 21.58 mmol) in one portion and the reaction continued to stir at -40 °C. After 1 h, LCMS showed 5% starting material remained. Additional 2 M SOCl₂ in DCM (1.439 mL, 2.88 mmol) was added and the reaction continued. After 20 min, the reaction was poured into ice water and the pH was adjusted to 7-8 with saturated NaHCO₃ (30 mL). The aqueous layer was extracted with DCM (125 mL, 2X). The combined organic layers were washed with brine (50 mL), filtered through Na₂SO₄ and concentrated *in vacuo.* The residue was purified by flash chromatography (Column: 80 grams silica. Eluent: 0-10% EtOAc/Heptanes. Gradient: 14 min) to give 2-(benzyloxy)-3-(chloromethyl)-4,6-dimethylpyridine (2.84 g, 10.74 mmol, 74.7% yield) as a colorless oil. ¹H NMR (400 MHz, CDCl₃) δ 7.48-7.57 (m, 2H), 7.30-7.45 (m, 3H), 6.64 (s, 1H), 5.47 (s, 2H), 4.74 (s, 2H), 2.43 (s, 3H), 2.38 (s, 3H). MS(ES) [M+H]⁺ 262.1.

### Intermediate 2

### (Z)-tert-butyl 4-(1-(((trifluoromethyl)sulfonyl)oxy)prop-1-en-1-yl)piperidine-1-carboxylate

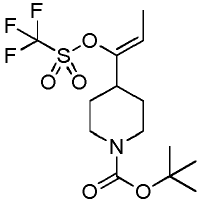

### a) tert-Butyl 4-propionylpiperidme-1-carboxylate

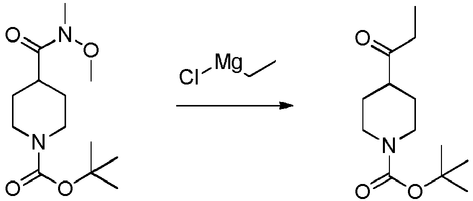

To a stirred solution of tert-butyl 4-(methoxy(methyl)carbamoyl)piperidine-1-carboxylate (10.0 g, 36.7 mmol) in THF (100 mL) at 0 °C (ice bath) under nitrogen was added dropwise 2 N ethylmagnesium chloride in THF (28 mL, 56.0 mmol). The reaction was stirred at 0 °C for 4 h, then quenched with saturated NH₄Cl, extracted with EtOAc, washed with brine, dried (Na₂SO₄), filtered and evaporated to dryness under vacuum. The crude product was purified by silica gel chromatography (Isco® RediSep Rf Gold 220 g, 0 to 40% EtOAc in hexanes). (UV negative, visualized by charring with H₂SO₄ in EtOH.) The pure fractions were combined and evaporated to dryness to give tert-butyl 4-propionylpiperidine-1-carboxylate (8.10 g, 33.6 mmol, 91% yield) as a colorless oil. MS(ES) [M+H]+ -isobutylene - 18 167.9, [M+H]+ -isobutylene 186.0, M+Na+ 264.1.

### b) (Z)-tert-Butyl 4-(1-(((trifluoromethyl)sulfonyl)oxy)prop-1-en-1-yl)piperidine-1-carboxylate

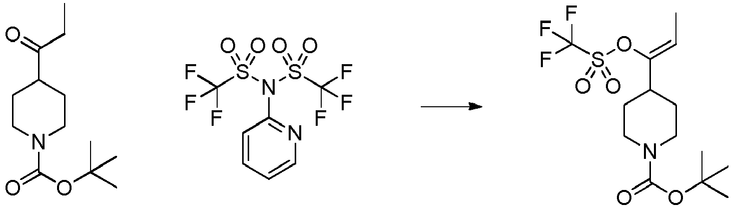

To a stirred solution of tert-butyl 4-propionylpiperidine-1-carboxylate (6.9 g, 28.6 mmol) in THF (80 mL) at -78 °C under nitrogen was added dropwise 1 N NaHMDS in THF (31 mL, 31.0 mmol). The reaction was stirred at -78 °C for 1 h. A solution of 1,1,1-trifluoro-N-(pyridin-2-yl)-N-((trifluoromethyl)sulfonyl)-methanesulfonamide (11.4 g, 31.8 mmol) in THF (50 mL) was next added dropwise over 5 min. The reaction was stirred for 1 h at -78 °C, then at 0 °C for 30 min. The reaction was quenched with water (150 mL), extracted with EtOAc (2 x 150 mL), washed with brine, dried (Na₂SO₄), filtered and concentrated under vacuum. The crude product was purified by silica gel chromatography (Isco® RediSep Rf Gold 220 g, 0 to 20% EtOAc in hexanes). (UV negative, visualized by charring with H₂SO₄ in EtOH.) The pure fractions were combined and evaporated to dryness to give (Z)-tert-butyl 4-(1-(((trifluoromethyl)sulfonyl)oxy)prop-1-en-1-yl)piperidine-1-carboxylate (9.15 g, 24.51 mmol, 86% yield) as a colorless oil. MS(ES) [M+H]⁺ -isobutylene 318.1.

### Intermediate 3

### a) Methyl 4-methylthiophene-3-carboxylate

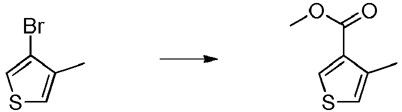

To a stirred solution of 3-bromo-4-methylthiophene (20.0 g, 113 mmol) in THF (100 mL) under nitrogen at RT was added isopropylmagnesium chloride lithium chloride complex 1.3 N in THF (90 mL, 117 mmol) dropwise. The reaction was stirred overnight. The reaction was cooled to -78 °C and treated with methyl chloroformate (12 mL, 155 mmol). The reaction was allowed to warm to RT and stirred for 1 hr. The reaction was diluted with EtOAc, washed with saturated NaHCO₃, stirred for 30 min, (formed a white suspension that stayed in the aqueous phase), washed with brine, dried (Na₂SO₄), filtered and concentrated under vacuum. The product was short path distilled under vacuum (4 to 2 mm Hg) at 44 to 50 °C (oil bath 50 to 75 °C). The main and late fractions were combined to give the product methyl 4-methylthiophene-3-carboxylate (13.2 g, 85 mmol, 74.8% yield) as a clear liquid. MS(ES) [M+H]⁺ 156.8.

### b) Methyl 4-methyl-5-propionylthiophene-3-carboxylate

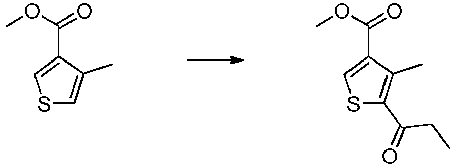

To a stirred solution of methyl 4-methylthiophene-3-carboxylate (5.0 g, 32.0 mmol) in CH₃NO₂ (50 mL) was added LiClO₄ (4.0 g, 37.6 mmol), propionic anhydride (5.87 mL, 38.4 mmol) and In(OTf)₃ (0.9 g, 1.601 mmol). The reaction was stirred at 50 °C for 2 hr. LCMS showed that the reaction was complete. The reaction was diluted with water (100 mL), extracted with DCM (2x 50 mL), dried (Na₂SO₄), filtered and evaporated to dryness under vacuum. The remaining brown solid was purified by silica gel chromatography (Isco® RediSep Rf Gold 120g, 0 to 25% EtOAc in hexanes) (loaded with DCM). The pure fractions were combined and evaporated to dryness. The remaining light yellow solid was triturated with hexanes, filtered and dried under vacuum to give the product methyl 4-methyl-5-propionylthiophene-3-carboxylate (4.60 g, 21.67 mmol, 67.7 % yield) as a white solid. MS(ES) [M+H]⁺ 212.9.

### Intermediate 4

### (2-Methoxy-4,6-dimethylpyridin-3-yl)methanamine

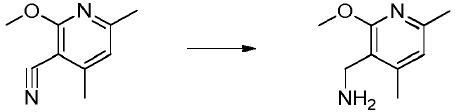

To a cooled (ice water bath) solution of 2-methoxy-4,6-dimethylnicotinonitrile (10 g, 61.7 mmol) in Et₂O (200 mL) was added dropwise 1 M LiAlH₄ in Et₂O (123 mL, 123 mmol). The ice bath was removed and the reaction mixture was stirred at r.t. for 16 h. The reaction mixture was cooled in an ice water bath and quenched with a mininum amount of water (until no more hydrogen was generated). The reaction was filtered and the insoluble material was washed with 10:1 DCM/MeOH. The combined organic filtrates were concentrated. The residue was purified via column chromatography (0 - 30% MeOH/DCM; 100 g-HP- silica gel column) to give (2-methoxy-4,6-dimethylpyridin-3-yl)methanamine (8.9 g) as a yellowish semi-solid.

### Examples

### Example 1

### (R)-5-((4,6-dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-c]pyridin-4(5H)-one

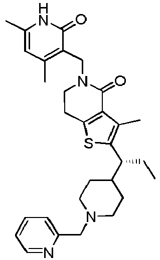

### a) (Z)-4-Methyl-2-(2-nitrovinyl)thiophene

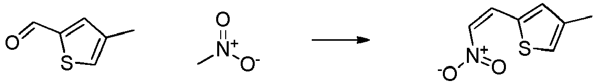

A solution of 4-methylthiophene-2-carbaldehyde (10.0 g, 79.3 mmol), nitromethane (100 mL) and NH₄OAc (1.1 g, 14.27 mmol) was heated at 100 °C for 4 h. The reaction was allowed to cool to r.t. and concentrated under vacuum. The residue was taken up in EtOAc, washed with 1 N HCl, aq. NaHCO₃, brine, dried (MgSO₄), filtered, and evaporated to dryness. The residue was purified by silica gel chromatography (Isco® RediSep Rf Gold 120 g, 0 to 15% EtOAc in hexanes). The pure fractions were combined and evaporated to dryness under vacuum to give (Z)-4-methyl-2-(2-nitrovinyl)thiophene (9.63 g, 56.91 mmol, 71.8% yield) as a yellow oil which solidified under vacuum. ¹H NMR (400 MHz, CDCl₃) δ 8.10 (d, *J*=13.4 Hz, 1H), 7.47 (d, *J*=13.4 Hz, 1H), 7.27 (s, 1H), 7.17 (s, 1H), 2.31 (d, *J*=0.8 Hz, 3H). MS(ES) [M+H]⁺ 170.0.

### b) Ethyl (2-(4-methylthiophen-2-yl)ethyl)carbamate

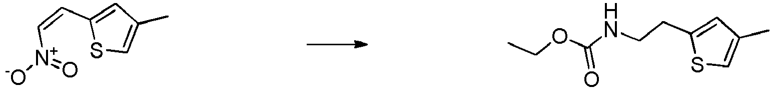

To a solution of 2 N LiBH₄ (120 mL, 240 mmol) under nitrogen was added dropwise TMSCl (60 mL, 473 mmol) over 10 min. The reaction became a white suspension. After stirring for 15 min a solution of (Z)-4-methyl-2-(2-nitrovinyl)thiophene (9.60 g, 56.74 mmol) in THF (50 mL) was added slowly dropwise over about 20 min. Vigorous gas evolution was observed. The reaction got slightly warm to the touch and was cooled in a water bath while periodically adding ice. The reaction was stirred at r.t. for 4 h, then warmed to 50 °C and stirred overnight. The reaction was cooled in an ice bath and carefully quenched with MeOH (200 mL). After stirring for 1 h the reaction was concentrated under vacuum to give crude 2-aminoethyl-4-methyl thiophene. MS(ES) [M+H]⁺ 142.1.

To a cooled (0 °C) solution of the crude 2-aminoethyl-4-methyl thiophene in DCM (200 mL) and water (100 mL) was slowly added Na₂CO₃ (25 g, 235.9 mmol) and ethyl chloroformate (0.710 mL, 7.39 mmol) dropwise. The resulting mixture was allowed to warm to r.t. and stirred for 1 h. The reaction was filtered through a pad of Celite® and the clear filtrate transferred to a separatory funnel. The lower organic phase was removed, dried (MgSO₄), filtered and concentrated under vacuum. The residue was purified by silica gel chromatography (Isco® RediSep Rf Gold 120 g, 10 to 30% EtOAc in hexanes). The pure fractions were combined and evaporated to dryness under vacuum to give ethyl (2-(4-methylthiophen-2-yl)ethyl)carbamate (9.29 g, 43.55 mmol, 76.7% yield) as a colorless oil. ¹H NMR (400 MHz, CDCl₃) δ 6.74 (s, 1H), 6.66 (s, 1H), 4.80 (br. s., 1H), 4.14 (q, *J*=6.9 Hz, 2H), 3.46 (q, *J*=6.3 Hz, 2H), 2.98 (t, *J*=6.6 Hz, 2H), 2.24 (d, *J*=0.8 Hz, 3H), 1.26 (t, *J*=7.1 Hz, 3H). MS(ES) [M+H]⁺ 214.1.

### c) 3-Methyl-6,7-dihydrothieno[3,2-c]pyridin-4(5H)-one

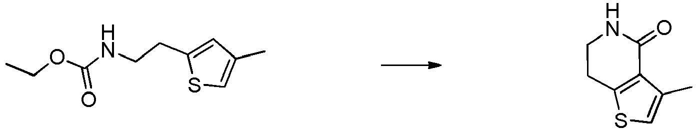

To ethyl (2-(4-methylthiophen-2-yl)ethyl)carbamate (9.20 g, 43.13 mmol) was added POCl₃ (100 mL, 107 mmol) and P₂O₅ (14 g, 98.6 mmol). The mixture was heated at reflux for 3 h (the mixture briefly formed a gummy ppt. which eventually dissolved with heating). The dark reaction mixture was allowed to cool to r.t. and evaporated to dryness under vacuum. The residue was carefully quenched with ice, basified with aq. Na₂CO₃, extracted with DCM, dried (Na₂SO₄), filtered, and concentrated under vacuum. The residue was purified by silica gel chromatography (Isco® RediSep Rf Gold 80 g, 30 to 80% EtOAc in hexanes). The pure fractions were combined and evaporated to dryness under vacuum, triturated with hexanes, filtered, and dried under vacuum to give 3-methyl-6,7-dihydrothieno[3,2-c]pyridin- 4(5H)-one (2.22 g,13.27 mmol, 30.78% yield) as an off-white solid. ¹H NMR (400 MHz, CDCl₃) δ 6.72 (m, 1H), 5.75 (br. s., 1H), 3.62 (t, *J*=6.2 Hz, 2H), 3.05 (t, *J*=6.7 Hz, 2H), 2.50 (d, *J*=1.3 Hz, 3H). MS(ES) [M+H]⁺ 168.0.

### d) 3-Methyl-2-(4,4,5,5-tetramethyl-1,3,2-dioxaborolan-2-yl)-6,7-dihydrothieno[3,2-c]pyridin-4(5H)-one

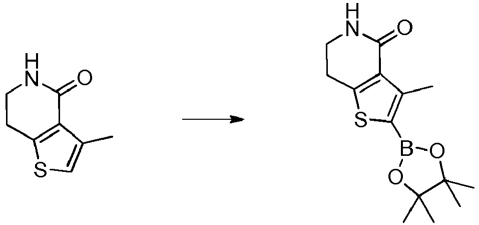

To a 50 mL round bottom flask containing [Ir(OMe)(1,5-cod)]₂ (106.4 mg, 0.161 mmol) under Ar was added 4,4,5,5-tetramethyl-1,3,2-dioxaborolane (2.8 mL, 19.30 mmol), followed by a solution of 4,4'-di-*tert*-butyl-2,2'-bipyridine (85.6 mg, 0.319 mmol) in *n-*hexane (16 mL). The reaction mixture was stirred for 1 min, at which time a solution of 3-methyl-6,7-dihydrothieno[3,2-c]pyridin-4(5H)-one (1.785 g, 10.67 mmol) in THF (8 mL) was added dropwise. The reaction was stirred for 2 h. The reaction was monitored for completion by LCMS (the product shows up as a mixture of boronic acid and pinacol boronate). As the reaction proceeded, it became heterogeneous. An additional equivalent of [Ir(OMe)(1,5-cod)]₂, 4,4'-di-*tert*-butyl-2,2'-bipyridine, and 4,4,5,5-tetramethyl-1,3,2-dioxaborolane were required to drive the reaction to completion. The mixture was evaporated to dryness under vacuum. The residue was triturated with a small volume of cold hexanes, filtered, rinsed with a small amount of hexanes and dried under vacuum to give 3-methyl-2-(4,4,5,5-tetramethyl-1,3,2-dioxaborolan-2-yl)-6,7-dihydrothieno[3,2-c]pyridin-4(5H)-one (2.286 g, 7.80 mmol, 73% yield) as a brown solid. MS(ES) [M+H]⁺ 212 (boronic acid), 294.2 (boronate).

### e) (E)-tert-Butyl 4-(1-(3-methyl-4-oxo-4,5,6,7-tetrahydrothieno[3,2-c]pyridin-2-yl)prop-1-en-1-yl)piperidine-1-carboxylate

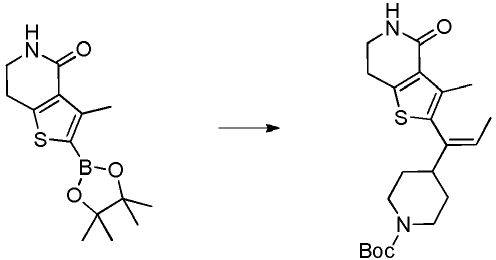

A solution of 3-methyl-2-(4,4,5,5-tetramethyl-1,3,2-dioxaborolan-2-yl)-6,7-dihydrothieno[3,2-c]pyridin-4(5H)-one (1.883 g, 6.42 mmol), (Z)-tert-butyl 4-(1-(((trifluoromethyl)sulfonyl)oxy)prop-1-en-1-yl)piperidine-1-carboxylate (2.524 g, 6.76 mmol), NaHCO₃ (1.802 g, 21.45 mmol) and Pd(PPh₃)₄ (735 mg, 0.636 mmol) in 1,4-dioxane (18 mL) and water (4.50 mL) was flushed with Ar, capped and heated in an oil bath at 70 °C for 2 h. The reaction mixture was partitioned between CHCl₃ and H₂O and the organic layer was dried over Na₂SO₄, filtered, adsorbed onto silica. Purification by column chromatography (Isco® CombiFlash Rf, 30 - 90% 80:20:2 EtOAc/hexane; 40 g column) gave (Z)-tert-butyl 4-(1-(3-methyl-4-oxo-4,5,6,7-tetrahydrothieno[3,2-c]pyridin-2-yl)prop-1-en-1-yl)piperidine-1-carboxylate (2.07 g, 5.30 mmol, 83% yield) as a light yellow solid. MS(ES) [M+H]⁺ 391.

### f) tert-Butyl 4-(1-(3-methyl-4-oxo-4,5,6,7-tetrahydrothieno[3,2-c]pyridin-2-yl)propyl)piperidine-1-carboxylate

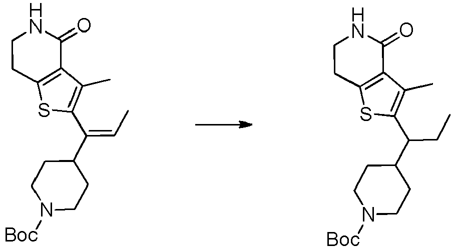

A 200 mL round bottom flask was charged with Pd/C (4.25 g, 39.9 mmol) and purged with Ar. *i*-PrOH (14 mL) was added, followed by a solution of (Z)-tert-butyl 4-(1-(3-methyl-4-oxo-4,5,6,7-tetrahydrothieno[3,2-c]pyridin-2-yl)prop-1-en-1-yl)piperidine-1-carboxylate (2.07 g, 5.30 mmol) in MeOH (70 mL). The mixture was degassed and maintained under a H₂ atmosphere for 24 h. The reaction mixture was filtered through Celite® and then through a 0.2 µM nylon disc. The solution was adsorbed onto silica and purified *via* column chromatography (Isco® CombiFlash Rf; 20 - 75% EtOAc/hexanes; 40 g column) to give tert-butyl 4-(1-(3-methyl-4-oxo-4,5,6,7-tetrahydrothieno[3,2-c]pyridin-2-yl)propyl)piperidine-1-carboxylate (1.40 g, 3.57 mmol, 67.3% yield) as a white solid.

The racemic product was resolved by chiral HPLC (Chiralpak®, 5 microns, 30 mm x 250 mm, 230 nm UV, 100% MeOH). The resolved products were twice diluted with TBME and concentrated, then dried in a vacuum oven (50 °C) to give:
S-(+)-tert-butyl 4-(1-(3-methyl-4-oxo-4,5,6,7-tetrahydrothieno[3,2-c]pyridin-2-yl)propyl)piperidine-1-carboxylate (670 mg): 96.6% ee, [α]_{D} = +40° (c = 0.25, MeOH, 24 °C). MS(ES) [M+Na]⁺ 415.2.
R-(-)-tert-buty14-(1-(3-methyl-4-oxo-4,5,6,7-tetrahydrothieno[3,2-c]pyridin-2-yl)propyl)piperidine-1-carboxylate (690 mg): 99.74% ee; [α]_{D} = -39° (c = 0.25, MeOH, 21 °C). MS(ES) [M+Na]⁺ 415.2.
(Note: the absolute stereochemistry of the ethyl group was assigned based on a known preference for R-isomer with regards to EZH2 inhibition).

### g) (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)propyl)piperidine-1-carboxylate

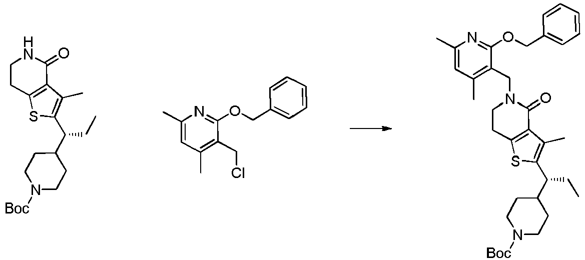

To a cooled (0 °C) solution of (R)-tert-butyl 4-(1-(3-methyl-4-oxo-4,5,6,7-tetrahydrothieno[3,2-c]pyridin-2-yl)propyl)piperidine-1-carboxylate (690 mg, 1.758 mmol) in DMF (6 mL) was added KOtBu (2.1 mL, 2.100 mmol). The reaction was stirred for 15 min, at which time a solution of 2-(benzyloxy)-3-(chloromethyl)-4,6-dimethylpyridine (556 mg, 2.124 mmol) in THF (2.4 mL) was added. The reaction was allowed to warm to r.t. for 40 min. Following consumption of starting material, the reaction was quenched with saturated NH₄Cl (4 mL) and evaporated to dryness. The residue was partitioned between CHCl₃ (10 mL) and saturated aqueous Na₂CO₃ and the organic layer was dried over Na₂SO₄, adsorbed onto silica, and purified by column chromatography (Isco® Companion; 0 - 20% EtOAc/hexanes; 40 g column) to give (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)propyl)piperidine-1-carboxylate (934 mg, 1.512 mmol, 86% yield) as a colorless oil. MS(ES) [M+H]⁺ 618.

### h) (R)-5-((4,6-dimethyl-2-oxo-1,2-dihydropyridin-3-yl)methyl)-3-methyl-2-(1-(piperidin-4-yl)propyl)-6,7-dihydrothieno[3,2-c]pyridin-4(5H)-one

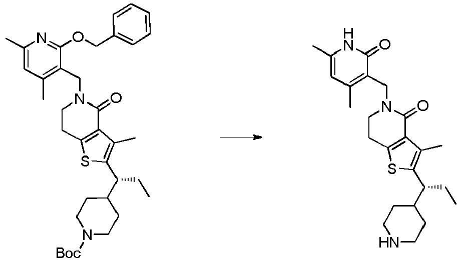

A mixture of (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)propyl)piperidine-1-carboxylate (934 mg, 1.512 mmol) in TFA (15 mL, 195 mmol) was heated at 45 °C for 1 h. The reaction mixture was concentrated to dryness, taken up in MeOH (2 mL), adsorbed onto silica and purified by column chromatography (Isco® CombiFlash Rf; 0 - 40% 80:20:2 [CHCl₃/MeOH/NH₄OH]/CHCl₃ w/ 0.5% isopropyl amine; 40 g column, pre-flushed with 5 column volumes 80:20:2 CHCl₃/MeOH/NH₄OH to neutralize acidic sites, followed by 3 column volumes CHCl₃ w/ 0.5% isopropyl amine). The fractions were concentrated, dissolved in CHCl₃, and washed with H₂O (2 x 100 mL) to remove excess isopropyl amine. The organic layer was concentrated to give (R)-5-((4,6-dimethyl-2-oxo-1,2-dihydropyridin-3-yl)methyl)-3-methyl-2-(1-(piperidin-4-yl)propyl)-6,7-dihydrothieno[3,2-c]pyridin-4(5H)-one (691 mg, 1.616 mmol, >100% yield) as a light yellow solid. MS(ES) [M+H]⁺ 428.

### i) (R)-5-((4,6-dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-c]pyridin-4(5H)-one

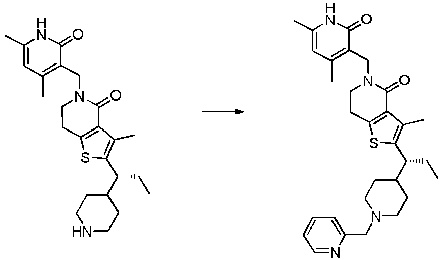

To a solution of (R)-5-((4,6-dimethyl-2-oxo-1,2-dihydropyridin-3-yl)methyl)-3-methyl-2-(1-(piperidin-4-yl)propyl)-6,7-dihydrothieno[3,2-c]pyridin-4(5H)-one (100.1 mg, 0.234 mmol) and picolinaldehyde (64.7 mg, 0.604 mmol) in DCM (2.5 mL) was added AcOH (60 µL, 1.048 mmol), followed by NaBH(OAc)₃ (109 mg, 0.514 mmol). The reaction was stirred at r.t. for 30 min, at which time NaHCO₃ was added slowly until basic. The mixture was extracted with CHCl₃, dried over Na₂SO₄, and concentrated. The residue was dissolved in MeOH (2 mL), adsorbed onto silica and purified by column chromatography (Isco® CombiFlash Rf; 0 - 30% 80:20:2 [CHCl₃/MeOH/NH₄OH]; 12 g column). The colorless oil was taken up in hexanes (15 mL) and sonicated and a white powder formed. The solvent was decanted and the solid dried to furnish (R)-5-((4,6-dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-c]pyridin-4(5H)-one (66 mg, 0.121 mmol, 51.6% yield) as a white solid. ¹H NMR (400 MHz, DMSO-*d*₆) δ ppm 11.53 (br. s., 1 H) 8.39 - 8.50 (m, 1 H) 7.73 (td, *J*=7.58, 1.77 Hz, 1 H) 7.38 (d, *J*=7.83 Hz, 1 H) 7.18 - 7.27 (m, 1 H) 5.87 (s, 1 H) 4.48 (s, 2 H) 3.45 - 3.57 (m, 4 H) 2.84 (t, *J*=6.69 Hz, 3 H) 2.66 - 2.79 (m, 2 H) 2.30 (s, 3 H) 2.16 (s, 3 H) 2.12 (s, 3 H) 1.91 - 2.00 (m, 1 H) 1.77 - 1.91 (m, 3 H) 1.29 - 1.43 (m, 3 H) 1.11 - 1.26 (m, 2 H) 0.71 (t, *J*=7.33 Hz, 3 H). MS(ES) [M+H]⁺ 519.

### Example 2

### (R)-2-(1-(1-Benzylpiperidin-4-yl)propyl)-5-((4,6-dimethyl-2-oxo-1,2-dihydropyridin-3-yl)methyl)-3-methyl-6,7-dihydrothieno[3,2-c]pyridin-4(5H)-one

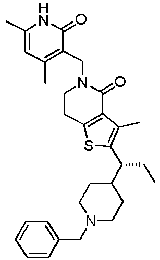

Following the procedure of Example 1(i), (R)-2-(1-(1-benzylpiperidin-4-yl)propyl)-5-((4,6-dimethyl-2-oxo-1,2-dihydropyridin-3-yl)methyl)-3-methyl-6,7-dihydrothieno[3,2-c]pyridin-4(5H)-one (1.1 g, 2 mmol, 63% yield) was prepared as a white solid. ¹H NMR (400 MHz, DMSO-*d*₆) δ ppm 11.54 (s, 1 H) 7.10 -7.40 (m, 5 H) 5.87 (s, 1 H) 4.48 (s, 2 H) 3.50 (t, *J*=6.82 Hz, 2 H) 3.38 (d, *J*=2.53 Hz, 2 H) 2.65 - 2.89 (m, 5 H) 2.30 (s, 3 H) 2.12 (s, 3 H) 2.15 (s, 3 H) 1.67 - 1.94 (m, 4 H) 1.25 - 1.42 (m, 3 H) 1.04 - 1.25 (m, 2 H) 0.70 (t, J=7.20 Hz, 3 H). MS(ES) [M+H]⁺ 518.

### Example 3

### (R)-5-((4,6-Dimethyl-2-oxo-1,2-dihydropyridin-3-yl)methyl)-3-methyl-2-(1-(1-(pyridin-4-ylmethyl)piperidin-4-yl)propyl)-6,7-dihydrothieno[3,2-c]pyridin-4(5H)-one

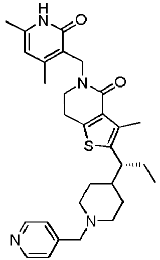

Following the procedure of Example 1(i), (R)-5-((4,6-dimethyl-2-oxo-1,2-dihydropyridin-3-yl)methyl)-3-methyl-2-(1-(1-(pyridin-4-ylmethyl)piperidin-4-yl)propyl)-6,7-dihydrothieno[3,2-c]pyridin-4(5H)-one (69 mg, 0.13 mmol, 51% yield) was prepared as a white solid. ¹H NMR (400 MHz, DMSO-*d*₆) δ ppm 11.53 (br. s., 1 H) 8.40 - 8.54 (m, 2 H) 7.27 (d, *J*=6.06 Hz, 2 H) 5.87 (s, 1 H) 4.48 (s, 2 H) 3.51 (t, *J*=6.69 Hz, 2 H) 3.43 (s, 2 H) 2.63 - 2.91 (m, 5 H) 2.30 (s, 3 H) 2.16 (s, 3 H) 2.09 - 2.14 (s, 3 H) 1.74 - 1.97 (m, 4 H) 1.28 - 1.42 (m, 3 H) 1.10 - 1.28 (m, 2 H) 0.71 (t, *J*=7.20 Hz, 3 H). MS(ES) [M+H]⁺ 519.

### Example 4

### (R)-2-(1-(1-((5-Chloropyridin-2-yl)methyl)piperidin-4-yl)propyl)-5-((4,6-dimethyl-2-oxo-1,2-dihydropyridin-3-yl)methyl)-3-methyl-6,7-dihydrothieno[3,2-c]pyridin-4(5H)-one

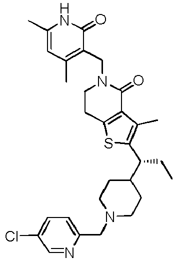

Following the procedure of Example 1(i), (R)-2-(1-(1-((5-chloropyridin-2-yl)methyl)piperidin-4-yl)propyl)-5-((4,6-dimethyl-2-oxo-1,2-dihydropyridin-3-yl)methyl)-3-methyl-6,7-dihydrothieno[3,2-c]pyridin-4(5H)-one (64 mg, 0.11 mmol, 46% yield) was prepared as a white solid. ¹H NMR (400 MHz, DMSO-*d*₆) δ ppm 11.53 (br. s., 1 H) 8.51 (d, *J*=2.02 Hz, 1 H) 7.86 (dd, *J*=8.46, 2.65 Hz, 1 H) 7.43 (d, *J*=8.34 Hz, 1 H) 5.87 (s, 1 H) 4.48 (s, 2 H) 3.44 - 3.57 (m, 4 H) 2.64 - 2.93 (m, 5 H) 2.30 (s, 3 H) 2.16 (s, 3 H) 2.12 (s, 3 H) 1.75 - 2.02 (m, 4 H) 1.25 - 1.47 (m, 3 H) 1.12 - 1.25 (m, 2 H) 0.71 (t, *J*=7.20 Hz, 3 H). MS(ES) [M+H]⁺ 553.

### Example 5

### N-((4,6-Dimethyl-2-oxo-1,2-dihydropyridin-3-yl)methyl)-5-(1-(1-((2-methoxypyridin-4-yl)methyl)piperidin-4-ylidene)propyl)-4-methylthiophene-3-carboxamide

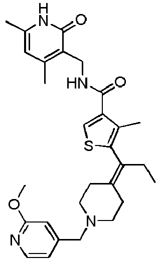

### a) Methyl 4-methyl-5-(1-(piperidin-4-ylidene)propyl)thiophene-3-carboxylate, hydrochloride

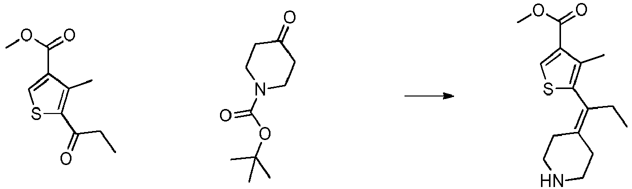

To a cooled (0 °C) suspension of zinc (16.76 g, 256 mmol) in THF (150 mL) under an inert atmosphere was added slowly TiCl₄ (13.72 mL, 124 mmol) *via* syringe by running a steady drip down the wall of the flask. Plumbs of yellow powder erupted from the stirred reaction (care must be taken to add the TiCl₄ slowly). The wall of the flask was washed down with THF (60 mL). The reaction was stirred at 0 °C for 10 min. The ice-bath was removed and the flask was equipped with a condenser and the reaction was heated at 70 °C for 1 h. The condenser was replaced with an addition funnel and a solution of methyl 4-methyl-5-propionylthiophene-3-carboxylate (4 g, 18.84 mmol), tert-butyl 4-oxopiperidine-1-carboxylate (12.01 g, 60.3 mmol), and pyridine (16.00 mL, 198 mmol) in THF (51.0 mL, 622 mmol) was added at a steady drip over 30 min. The reaction was stirred at 70 °C for 4 days. The reaction was allowed to cool and was poured into a mixture of diatomaceous earth, sat. NH₄Cl (200 mL), and EtOAc (400 mL). The mixture was stirred vigorously, then decanted and filtered through a bed of diatomaceous earth. The remaining sludge was extracted with EtOAc (3 x 400 mL). The cake was filtered and washed with EtOAc (200 mL). The layers were separated and the organics washed with brine, dried over Na₂SO₄, filtered and concentrated. The residue was taken up in DCM (30 mL) and the undisolved salts were filtered off. The DCM was concentrated *in vacuo.* The residue was taken up in DCM (5 mL) and loaded onto silica and purified by column chromatography using: ISCO® Redisep 80 gram column, 0-20% EtOAc/heptanes over 12 min, 60 mL/min, then 20-60% EtOAc/heptanes over 12 min. The product did not elute. The gradient was increased to 60-90% over 5 min, then held at 90% for 8 min. The desired fractions were combined, concentrated and dried under vacuum to give methyl 4-methyl-5-(1-(piperidin-4-ylidene)propyl)thiophene-3-carboxylate, hydrochloride (4.08 g, 12.79 mmol, 67.9% yield) as a tan solid. ¹H NMR (400 MHz, CDCl₃) δ ppm 8.84-9.00 (m, 2H), 8.06 (s, 1H), 3.87 (s, 3H), 3.46-3.68 (m, 2H), 2.84 (br. s., 2H), 2.40 (t, *J*=6.06 Hz, 4H), 2.24 (s, 3H), 1.60-1.86 (m, 2H), 0.95 (t, *J*=7.45 Hz, 3H).

### b) tert-Butyl4-(1-(4-(((4,6-dimethyl-2-oxo-1,2-dihydropyridin-3-yl)methyl)carbamoyl)-3-methylthiophen-2-yl)propylidene)piperidine-1-carboxylate

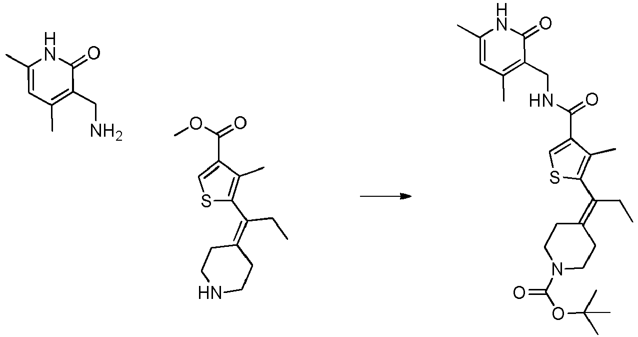

To a solution of methyl 4-methyl-5-(1-(piperidin-4-ylidene)propyl)thiophene-3-carboxylate, hydrochloride (4 g, 12.66 mmol) in DCM (20.37 mL, 317 mmol) was added Et₃N (2.295 mL, 16.46 mmol) and Boc₂O (5.00 mL, 21.53 mmol). The reaction was maintained at r.t. After 15 min, LCMS showed the reaction was complete. The DCM was removed *in vacuo* and the remaining residue was partitioned between EtOAc (80 mL) and sat. NaHCO₃ (35 mL). White solid salts cashed out of the aqueous layer. The solids were filtered off and the layers separated. The organics were washed with water (20 mL), 1N HCl (20 mL), and brine (10 mL). The EtOAc layer was dried over Na₂SO₄, filtered and concentrated to give the Boc-protected intermediate (4.63 g).

To a mixture of the above residue in MeOH (25 mL) and THF (75 mL) was added 8 M NaOH (4.12 mL, 32.9 mmol). The suspension was heated at 50 °C overnight. The reaction was monitored by LCMS. An additional portion of 8 M NaOH (4.12 mL, 32.9 mmol) was added and the reaction was heated at 50 °C for 4 h. Additional 8M NaOH (0.997 mL, 7.98 mmol) was added and the reaction was was heated at 50 °C for 0.5 h. The organic solvents were removed *in vacuo* and the remaining aqueous residue was diluted with water (30 mL). The aqueous solution was cooled (ice bath) and the pH adjusted to ∼5-6 with 6 N HCl (8.44 mL, 50.7 mmol). The water was removed *in vacuo* and the remaining residue dried under high vacuum for 3 days to give the carboxylic acid intermediate.

To a suspension of the above residue in DMF (34.3 mL, 443 mmol) and DCM (40.7 mL, 633 mmol) was added NMM (5.57 mL, 50.7 mmol), followed by HATU (4.82 g, 12.66 mmol) and 3-(aminomethyl)-4,6-dimethylpyridin-2(1H)-one, hydrochloride (2.509 g, 13.30 mmol). The reaction was stirred at r.t. for 16 h, at which time the solids were filtered and partioned between EtOAc (20 mL) and water (20 mL). The organic layer was searated and set aside. The mother liquor from above was concentrated until a thick oil remained (∼15-20 mL). The oily residue was added dropwise to a stirred solution of 0.2 N HCl (50 mL). The solids were collected and partitioned between EtOAc (100 mL) and water (30 mL). The organic layer was separated and combined with the EtOAc layer previously set aside. The combined solution was washed with brine (12 mL), dried over Na₂SO₄, filtered and concentrated to give tert-butyl 4-(1-(4-(((4,6-dimethyl-2-oxo-1,2-dihydropyridin-3-yl)methyl)carbamoyl)-3-methylthiophen-2-yl)propylidene)piperidine-1-carboxylate (4.63 g, 9.27 mmol, 73.2% yield) as a tan solid. ¹H NMR (400 MHz, DMSO-*d₆*) δ ppm 11.43-11.59 (m, 1H), 8.04 (t, *J*=5.18 Hz, 1H), 7.81 (s, 1H), 5.88 (s, 1H), 4.24 (d, *J*=4.80 Hz, 2H), 3.40 (br. s., 2H), 3.23 (br. s., 2H), 2.36 (t, *J*=5.56 Hz, 2H), 2.28 (d, *J*=7.33 Hz, 2H), 2.16-2.21 (m, 3H), 2.12 (s, 3H), 2.07 (s, 3H), 1.90 (t, *J*=5.43 Hz, 2H), 1.39 (s, 9H), 0.87 (t, *J*=7.45 Hz, 3H). MS(ES) [M+H]⁺ 500.2.

### c) 5-(1-(1-(2,2-Difluoropropyl)piperidin-4-ylidene)propyl)-N-((4,6-dimethyl-2-oxo-1,2-dihydropyridin-3-yl)methyl)-4-methylthiophene-3-carboxamide

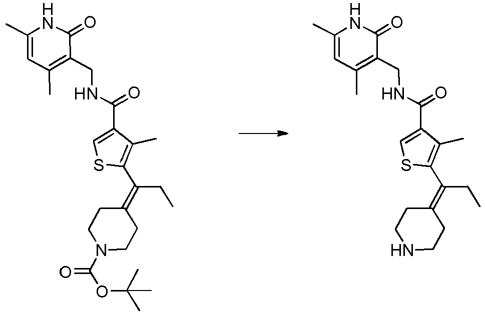

To a solution of tert-butyl 4-(1-(4-(((4,6-dimethyl-2-oxo-1,2-dihydropyridin-3-yl)methyl)carbamoyl)-3-methylthiophen-2-yl)propylidene)piperidine-1-carboxylate (4 g, 8.01 mmol) in DCM (45 mL) was added 4 N HCl in 1,4-dioxane (10.01 mL, 40.0 mmol). The reaction was stirred at r.t. overnight, at which time the solvent was decanted off. The remaining residue was dissolved in MeOH (25 mL) and mixture was concentrated. Three additional put and takes from MeOH yielded the intermediate product, N-((4,6-dimethyl-2-oxo-1,2-dihydropyridin-3-yl)methyl)-4-methyl-5-(1-(piperidin-4-ylidene)propyl)thiophene-3-carboxamide, hydrochloride (3.45 g, 5.78 mmol, 72.2% yield) as a foam. The foam was ground into a powder and was carried on without further purification. ¹H NMR (400 MHz, DMSO-*d₆*) δ ppm 11.56 (br. s., 1H), 8.91 (br. s., 1H), 8.03-8.12 (m, 1H), 7.86 (s, 1H), 5.89 (s, 1H), 4.24 (d, *J*=4.29 Hz, 2H), 3.08-3.21 (m, 3H), 2.96 (br. s., 2H), 2.60 (t, *J*=5.94 Hz, 2H), 2.24-2.37 (m, 2H), 2.19 (s, 3H), 2.10-2.15 (m, 5H), 2.09 (s, 3H), 0.88 (t, *J*=7.45 Hz, 3H). MS(ES) [M+H]⁺ 400.1.

### d) N-((4,6-Dmethyl-2-oxo-1,2-dihydropyridin-3-yl)methyl)-5-(1-(1-((2-methoxypyridin-4-yl)methyl)piperidin-4-ylidene)propyl)-4-methylthiophene-3-carboxamide

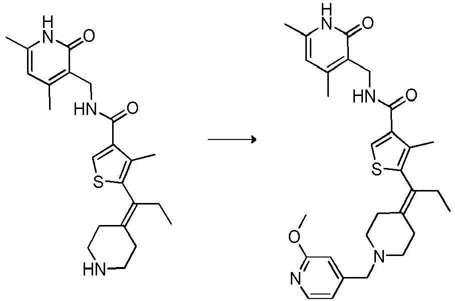

To a solution of N-((4,6-dimethyl-2-oxo-1,2-dihydropyridin-3-yl)methyl)-4-methyl-5-(1-(piperidin-4-ylidene)propyl)thiophene-3-carboxamide, Hydrochloride (3.6 g, 6.03 mmol), in MeOH (80 mL) was added DIPEA (3.16 mL, 18.08 mmol) and MgSO₄ (1.814 g, 15.07 mmol). The reaction was stirred at r.t. for 5 min, at which time 2-methoxyisonicotinaldehyde (1.653 g, 12.05 mmol) was added. The reaction was stirred for 15 min, at which time AcOH (0.690 mL, 12.05 mmol) was added. The reaction was stirred for 30 min, at which time NaBH₃CN (2.273 g, 36.2 mmol) was added. The reaction was stirred overnight, at which time it was filtered and the solids washed with MeOH (40 mL), followed by DCM (40 mL). The filtrate was concentrated and the residue was partitioned between DCM (100 mL) and sat. NaHCO₃ (15 mL) and water (15 mL). The phases were split and the organics were washed with brine (6 mL), dried over Na₂SO₄ and concentrated. The residue was charged onto silica and purified by normal phase flash chromatography. The solid was partitioned between DCM (75 mL) and sat. NH₄Cl (25 mL). The organic layer was washed with sat NH₄Cl (4 x 10 mL) and brine (6 mL), dried over Na₂SO₄, and concentrated to give N-((4,6-dimethyl-2-oxo-1,2-dihydropyridin-3-yl)methyl)-5-(1-(1-((2-methoxypyridin-4-yl)methyl)piperidin-4-ylidene)propyl)-4-methylthiophene-3-carboxamide (1.2 g, 2.282 mmol, 37.9% yield) as a white solid. ¹H NMR (400 MHz, DMSO-*d₆*) δ ppm 11.47 (s, 1H), 8.08 (d, *J*=5.31 Hz, 1H), 8.00 (t, *J*=5.05 Hz, 1H), 7.78 (s, 1H), 6.94 (d, *J*=5.31 Hz, 1H), 6.73 (s, 1H), 5.86 (s, 1H), 4.23 (d, *J*=5.05 Hz, 2H), 3.83 (s, 3H), 3.45 (s, 2H), 2.39-2.45 (m, 4H), 2.23-2.34 (m, *J*=5.05 Hz, 4H), 2.18 (s, 3H), 2.11 (s, 3H), 2.07 (s, 3H), 1.91-1.99 (m, *J*=4.29 Hz, 2H), 0.86 (t, *J*=7.33 Hz, 3H). MS(ES) [M+H]⁺ 521.3.

### Example 6

### (R)-5-((4,6-Dimethyl-2-oxo-1,2-dihydropyridin-3-yl)methyl)-3-methyl-2-(1-(1-(pyridin-2-ylmethyl)piperidin-4-yl)ethyl)-5,6,7,8-tetrahydro-4H-thieno[3,2-c]azepin-4-one

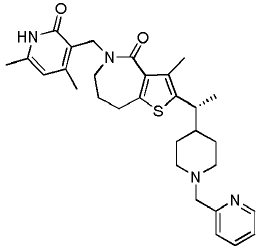

### a) Methyl 4-methyl-5-(4,4,5,5-tetramethyl-1,3,2-dioxaborolan-2-yl)thiophene-3-carboxylate

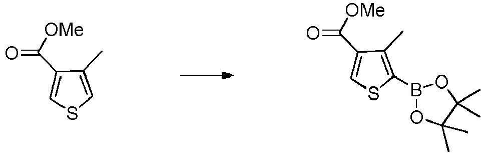

To a 100-mL round-bottom flask charged with (1,5-cycooctadiene)(methoxy)iridinum(1)dimer (325 mg, 0.490 mmol) was added 4,4,5,5-tetramethyl-1,3,2-dioxaborolane (7.10 g, 55.5 mmol) with stirring, followed by a solution of 4,4'-di-tert-butyl-2,2'-dipyridine (260 mg, 0.969 mmol) in n-hexane (35 mL). The mixture was stirred at r.t. for 2 min and methyl 4-methylthiophene-3-carboxylate (5 g, 32 mmol) was added dropwise. The mixture was stirred at r.t. for 18 h. The reaction mixture was then concentrated and the residue was purified using column chromatography (silica gel, 0 to 100% DCM/hexanes) to give 5.8 g of product as a colorless oil. ¹H NMR (400 MHz, CDCl₃) δ 1.36 (s, 12 H), 2.70 (s, 3 H), 3.87 (s, 3 H), 8.32 (s, 1 H). MS(ES) [M+H]⁺ 283.1.

### b) tert-Butyl 4-(1-(4-(methoxycarbonyl)-3-methylthiophen-2-yl)vinyl)piperidine-1-carboxylate

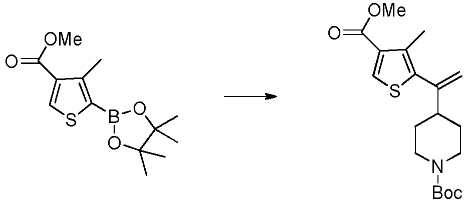

To a solution of methyl 4-methyl-5-(4,4,5,5-tetramethyl-1,3,2-dioxaborolan-2-yl)thiophene-3-carboxylate (2.65 g, 9.39 mmol) in 1,4-dioxane (72 mL) were added *tert-*butyl 4-(1-(((trifluoromethyl)sulfonyl)oxy)vinyl)piperidine-1-carboxylate (3.38 g, 9.39 mmol), Na₂CO₃ (2.489 g, 23.48 mmol), and water (24 mL). The mixture was degassed for 10 min by bubbling N₂. Pd(PPh₃)₄ (0.543 g, 0.470 mmol) was added and the mixture was heated at 70 °C for 1 h. The reaction mixture was allowed to cool to r.t. and extracted with EtOAc (3x). The combined extracts were dried over Na₂SO₄ and concentrated. The residue was purified using column chromatography (silica gel, 0 to 40% EtOAc/hexanes) to give 2.8 g of product as a colorless oil. ¹H NMR (400 MHz, CDCl₃) δ 1.27 - 1.55 (m, 11 H), 1.78 (m, 2 H), 2.26 - 2.46 (m, 4 H), 2.71 (t, *J*=11.49 Hz, 2 H), 3.87 (s, 3 H), 4.18 (br. s., 2 H), 5.13 (s, 1 H), 5.29 - 5.39 (m, 1 H), 8.02 (s, 1 H). MS(ES) [M+Na]⁺ 388.1.

### c) (R)-tert-Butyl 4-(1-(4-(methoxycarbonyl)-3-methylthiophen-2-yl)ethyl)piperidine-1-carboxylate

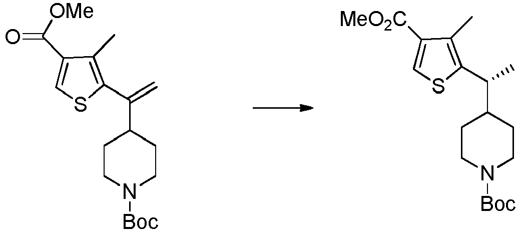

A solution of *tert*-butyl 4-(1-(4-(methoxycarbonyl)-3-methylthiophen-2-yl)vinyl)piperidine-1-carboxylate (1.2 g, 3.28 mmol) and ((4*R*,5*R*)-(+)-O-[1-Benzyl-1-(5-methyl-2-phenyl-4,5-dihydrooxazol-4-yl)-2-phenylethyl] (dicyclohexylphosphinite)(1,5-COD)iridium(I)tetrakis(3,5-bis(trifluoromethyl)phenylborate (63 mg, 0.036 mmol) in DCM (50 mL) was hydrogenated at 344.7 kPa (50 psi) hydrogen pressure for 30 h on a Parr shaker. The mixture was concentrated and the residue was purified using column chromatography (silica gel, 0 to 40% EtOAc/hexanes) to give 1.1 g of product as a colorless oil. The optical purity of the product was determined to be 98% ee by chiral HPLC (Chiralpak AY-H, 5 microns, 4.6 mm x 150 mm; 245, 250 nm UV; 90:10:0.1 n-heptane:EtOH: isopropylamine, isocratic, 1.0 mL/min). ¹H NMR (400 MHz, CDCl₃) δ 1.03 - 1.33 (m, 5 H), 1.38 - 1.58 (m, 11 H), 1.88 (d, *J*=12.38 Hz, 1 H), 2.37 (s, 3 H), 2.48-2.77 (m, 2 H), 2.94 (quin, *J*=7.26 Hz, 1 H), 3.85 (s, 3 H), 4.05-4.15 (m, 1 H), 7.97 (s, 1 H). MS(ES) [M+H]⁺ 390.2.

### d) (R)-tert-Butyl 4-(1-(5-bromo-4-(methoxycarbonyl)-3-methylthiophen-2-yl)ethyl)piperidine-1-carboxylate

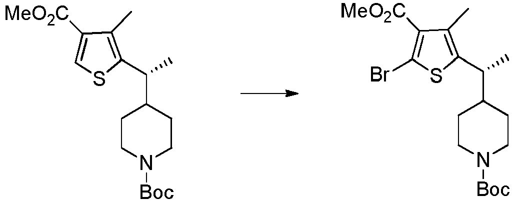

To a solution of (*R*)-*tert*-butyl 4-(1-(4-(methoxycarbonyl)-3-methylthiophen-2-yl)ethyl)piperidine-1-carboxylate (2.1 g, 5.71 mmol) in DMF (30 mL) was added NBS (1.322 g, 7.43 mmol), and the mixture was stirred at r.t. for 5 h. The mixture was quenched with water and extracted with EtOAc (3x), The extract was dried over Na₂SO₄ and concentrated. The residue was purified using column chromatography (silica gel, 0 to 50% EtOAc/hexanes) to give 1.98 g of product as a pale brown oil. ¹H NMR (400 MHz, CDCl₃) δ 1.03 -1.65 (m, 16 H), 1.86 (d, *J*=12.88 Hz, 1 H), 2.21 - 2.30 (m, 3 H), 2.54 - 2.72 (m, 2 H), 2.85 - 2.98 (m, 1 H), 3.88 - 3.95 (m, 3 H), 4.08 (d, *J*=13.64 Hz, 1 H), 4.17 (d, *J*=13.64 Hz, 1 H). MS(ES) [M+Na]⁺ 468.1, 470.1.

### e) (R)-tert-Butyl 4-(1-(4-(methoxycarbonyl)-3-methyl-5-(3-oxopropyl)thiophen-2-yl)ethyl)piperidine-1-carboxylate

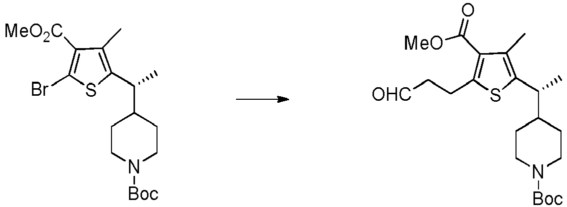

To a solution of (*R*)-*tert*-butyl 4-(1-(5-bromo-4-(methoxycarbonyl)-3-methylthiophen-2-yl)ethyl)piperidine-1-carboxylate (950 mg, 2.128 mmol) in DMF (14 mL) were added prop-2-en-1-ol (0.579 mL, 8.51 mmol), NaHCO₃ (468 mg, 5.58 mmol), Bu₄NCl (591 mg, 2.128 mmol) and Pd(OAc)₂ (23.89 mg, 0.106 mmol). The mixture was degassed for 10 min by bubbling Ar. The reaction mixture was then heated at 65 °C for 3 h. The reaction was allowed to cool to r.t., quenched with water (20 mL) and filtered. The filtrate was extracted with EtOAc (3x). The combined extracts were dried over Na₂SO₄ and concentrated. The residue was purified using column chromatography (silica gel, 0 to 40% EtOAc/hexanes) to give 680 mg of product as a pale brown oil. ¹H NMR (400 MHz, CDCl₃) δ 1.04 -1.69 (m, 16 H), 1.86 (d, *J*=12.88 Hz, 1 H), 2.25 (s, 3 H), 2.55 - 2.77 (m, 2 H), 2.81 - 2.94 (m, 3 H), 3.26 - 3.46 (m, 2 H), 3.82 - 3.92 (m, 3 H), 4.05 (br. s., 1 H), 4.15 (br. s., 1 H), 9.84 (t, *J*=1.26 Hz, 1 H). MS(ES) [M+H]⁺ 446.3.

### f) (R)-tert-Butyl 4-(1-(5-(3-(((2-methoxy-4,6-dimethylpyridin-3-yl)methyl)amino)propyl)-4-(methoxycarbonyl)-3-methylthiophen-2-yl)ethyl)piperidine-1-carboxylate

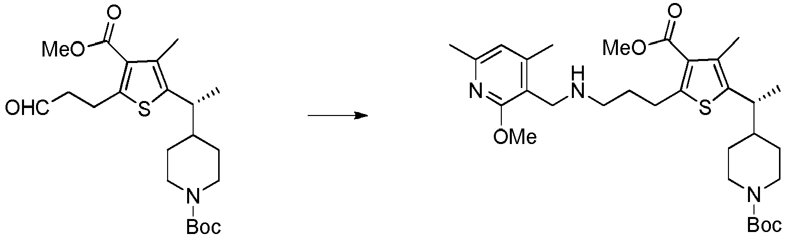

To a solution of (*R*)-*tert*-butyl 4-(1-(4-(methoxycarbonyl)-3-methyl-5-(3-oxopropyl)thiophen-2-yl)ethyl)piperidine-1-carboxylate (675 mg, 1.594 mmol) in MeOH (14 mL) was added (2-methoxy-4,6-dimethylpyridin-3-yl)methanamine (371 mg, 2.231 mmol). The mixture was stirred at r.t. for 18 h. The reaction was cooled (0 °C ice bath) and NaBH₄ (109 mg, 2.87 mmol) was added. The reaction mixture was stirred at r.t. for 20 min. The reaction was quenched with sat. aqueous NaHCO₃ and extracted with EtOAc (3x). The combined extracts were dried over Na₂SO₄ and concentrated. The residue was purified using column chromatography (silica gel, 0 to 100% EtOAc/hexanes, then 0 to 15% MeOH/DCM) to give 550 mg of product as a pale brown oil. ¹H NMR (400 MHz, CDCl₃) δ 1.17-1.76 (m, 16 H), 1.86 (quin, *J*=7.33 Hz, 3 H), 2.19- 2.95 (m, 14 H), 3.06 (dq, J=15.51, 7.46 Hz, 2 H), 3.75 (s, 2 H), 3.84 (s, 3 H), 3.94 (s, 3 H), 4.05 - 4.29 (m, 2 H). MS(ES) [M+H]⁺ 574.1.

### g) (R)-tert-Butyl 4-(1-(5-((2-methoxy-4,6-dimethylpyridin-3-yl)methyl)-3-methyl-4-oxo-5,6,7,8-tetrahydro-4H-thieno[3,2-c]azepin-2-yl)ethyl)piperidine-1-carboxylate

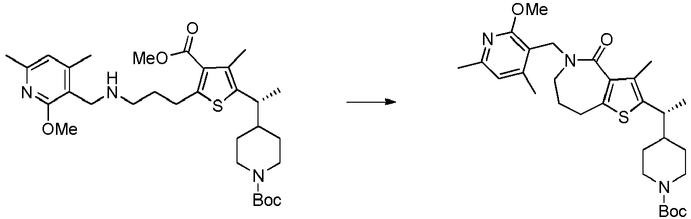

To a solution of (R)-*tert*-butyl 4-(1-(5-(3-(((2-methoxy-4,6-dimethylpyridin-3-yl)methyl)amino)propyl)-4-(methoxycarbonyl)-3-methylthiophen-2-yl)ethyl)piperidine-1-carboxylate (530 mg, 0.924 mmol) in MeOH (6 mL) was added 8 N NaOH (1.4 mL, 11.2 mmol). The mixture was heated at 50 °C for 20 h. The mixture was allowed to cool to r.t., quenched with aqueous 6 N HCl (1.878 mL, 11.27 mmol), and concentrated. The residue was dried under vacuum and treated with DMSO (6 mL). To this mixture were added EDC (354 mg, 1.847 mmol), HOAt (251 mg, 1.847 mmol), and NMM (0.609 mL, 5.54 mmol). The mixture was stirred at r.t. for 5 h. The reaction was then quenched with water (20 mL). The resulting precipitate was collected by filtration and purified using column chromatography (silica gel, 0 to 80% EtOAc/hexanes) to give 450 mg of product as a white solid. ¹H NMR (400 MHz, CDCl₃) δ 1.00 - 1.68 (m, 18 H), 1.86 (d, *J*=12.38 Hz, 1 H), 2.27 (s, 3 H), 2.35 (s, 3 H), 2.43 (s, 3 H), 2.53 - 2.79 (m, 4 H), 2.79 - 2.96 (m, 1 H), 3.29 (t, *J*=6.32 Hz, 2 H), 3.97 (s, 3 H), 4.14 (m, 1 H), 4.80 (d, *J*=14.15 Hz, 1 H), 4.89 (d, *J*=14.15 Hz, 1 H). MS(ES) [M+H]⁺ 542.1.

### h) (R)-5-((4,6-Dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one

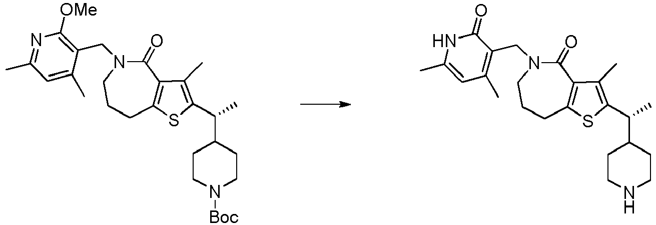

To a solution of (R)-*tert*-butyl 4-(1-(5-((2-methoxy-4,6-dimethylpyridin-3-yl)methyl)-3-methyl-4-oxo-5,6,7,8-tetrahydro-4H-thieno[3,2-c]azepin-2-yl)ethyl)piperidine-1-carboxylate (350 mg, 0.646 mmol) in 1,4-dioxane (3 mL) was added 6 N HCl (0.5 mL, 3 mmol). The mixture was heated at 70 °C for 20 h. The mixture was concentrated and the residue was dried under vacuum to give 340 mg of product as an off-white solid (*bis-*HCl salt). ¹H NMR (400 MHz, DMSO-*d*₆) δ 1.10 - 2.02 (m, 10 H), 2.15 (s, 3H), 2.16 (s, 3H), 2.14 (s, 3 H), 2.61 - 2.87 (m, 4 H), 2.93 (t, *J*=7.33 Hz, 1 H), 3.13-3.34 (m, 4 H), 4.50 (d, *J*=13.64 Hz, 1 H), 4.62 (d, *J*=13.64 Hz, 1 H), 5.92 (s, 1 H). MS(ES) [M+H]⁺ 428.5.

### i) (R)-5-((4,6-Dimethyl-2-oxo-1,2-dihydropyridin-3-yl)methyl)-3-methyl-2-(1-(1-(pyridin-2-ylmethyl)piperidin-4-yl)ethyl)-5,6,7,8-tetrahydro-4H-thieno[3,2-c]azepin-4-one

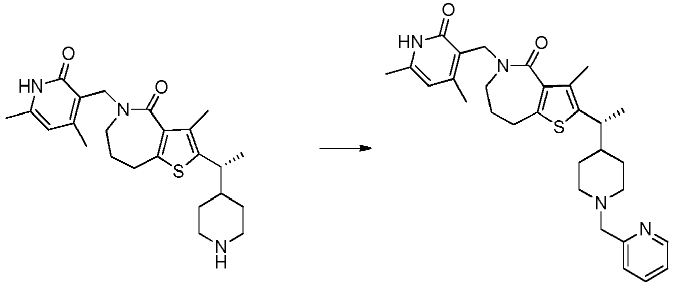

To a solution of (*R*)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one, *bis-*HCl (100 mg, 0.200 mmol) in MeOH (2 mL) were added DIPEA (0.080 mL, 0.460 mmol), picolinaldehyde (0.038 mL, 0.400 mmol), and AcOH (0.038 mL, 0.659 mmol). The mixture was stirred at r.t. for 30 min. NaBH₃CN (50.2 mg, 0.799 mmol) was added and the mixture was stirred at r.t. for 18 h. The mixture was purified using reverse-phase HPLC. The fractions containing product were treated with 1 N HCl and concentrated to give 52 mg of product as a white solid. ¹H NMR (400 MHz, MeOH-*d*₄) δ 1.33 (d, *J*=7.07 Hz, 3 H), 1.56 - 1.75 (m, 2 H), 1.80 (d, *J*=11.87 Hz, 2 H), 2.16 - 2.37 (m, 6 H), 2.52 (s, 3 H), 2.65 (s, 3 H), 2.93 - 3.00 (m, 2 H), 3.03 - 3.24 (m, 3 H), 3.35 - 3.45 (m, 2 H), 3.55 (d, *J*=11.62 Hz, 1 H), 3.64 (d, *J*=12.38 Hz, 1 H), 4.59 (s, 2 H), 4.86 (s, 2 H), 6.98 (s, 1 H), 7.73 (dd, *J*=7.07, 5.56 Hz, 1 H), 7.85 (d, *J*=7.83 Hz, 1 H), 8.20 (td, *J*=7.83, 1.52 Hz, 1 H), 8.73-8.86 (m, 1 H). MS(ES) [M+H]⁺ 519.4.

### Example 7

### (R)-5-((4,6-Dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4H-thieno[3,2-c]azepin-4-one

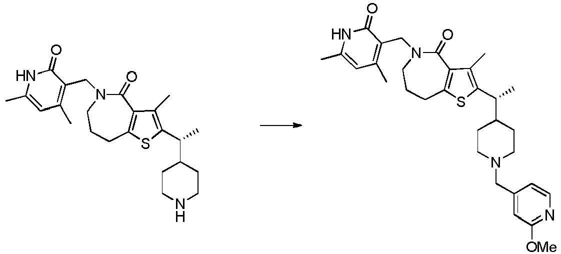

To a solution of (*R*)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one, *bis-*HCl (100 mg, 0.200 mmol) in MeOH (1.5 mL) were added DIPEA (0.080 mL, 0.460 mmol), 2-methoxyisonicotinaldehyde (54.8 mg, 0.400 mmol), and AcOH (0.038 mL, 0.659 mmol). The mixture was stirred at r.t. for 30 min. NaBH₃CN (50.2 mg, 0.799 mmol) was added and the mixture was stirred at r.t. for 18 h. The mixture was purified using reverse-phase HPLC. The fractions containing product were treated with 1 N HCl and concentrated to give 54 mg of product as a white solid (HCl salt). ¹H NMR (400 MHz, MeOH-*d*₄) δ 1.32 (d, *J*=7.10 Hz, 3 H), 1.60 - 1.87 (m, 4 H), 2.13 - 2.31 (m, 6 H), 2.51 (s, 3 H), 2.63 (s, 3 H), 2.90 - 3.18 (m, 5 H), 3.35 - 3.43 (m, 2 H), 3.45 - 3.54 (m, 1 H), 3.58 (d, *J*=11.66 Hz, 1 H), 4.17 (s, 3 H), 4.46 (s, 2 H), 4.85 (s, 2 H), 6.93 (s, 1 H), 7.48 (dd, *J*=5.83, 1.27 Hz, 1 H), 7.64 (s, 1 H), 8.39 (d, *J*=5.83 Hz, 1 H). MS(ES) [M+H]⁺ 549.7.

Alternatively, the compound of Example 7 may be prepared according to the following procedures:

### a) Methyl 4-methyl-5-(4,4,5,5-tetramethyl-1,3,2-dioxaborolan-2-yl)thiophene-3-carboxylate

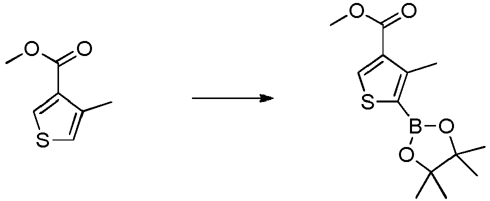

To a 500 mL round-bottom flask under Ar was added (1,5-cyclooctadiene)(methoxy)iridium(I) dimer (1.3 g, 1.961 mmol). With stirring, 4,4,5,5-tetramethyl-1,3,2-dioxaborolane (32 mL, 222 mmol) was added via syringe followed by a solution of 4,4'-di-tert-butyl-2,2'-dipyridyl (1.04 g, 3.87 mmol) in n-hexane (75 mL) (the reaction was kept cool 5-10 °C in a ice bath). After stirring for 1 minute, methyl 4-methylthiophene-3-carboxylate (20.0 g, 128 mmol) was added (gas evolution seen). The reaction was stirred overnight at r.t. The reaction was evaporated to dryness under vacuum and purified by silica gel chromatography (Isco® RediSep Rf Gold 330 g, 0 to 10% EtOAc in hexanes). The pure fractions were combined and evaporated to dryness to give methyl 4-methyl-5-(4,4,5,5-tetramethyl-1,3,2-dioxaborolan-2-yl)thiophene-3-carboxylate (33.1 g, 117 mmol, 92% yield) as a colorless oil, which solidified to a waxy white solid under vacuum. MS(ES) [M+H]⁺ 200.9 (boronic acid), 283.1 (boronate).

### b) tert-Butyl 4-(1-(((trifluoromethyl)sulfonyl)oxy)vinyl)piperidine-1 -carboxylate

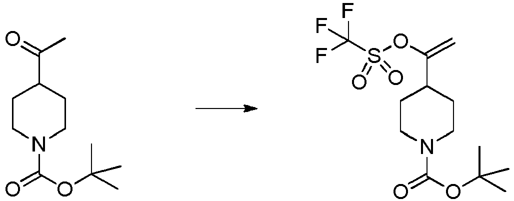

To a stirred solution of tert-butyl 4-acetylpiperidine-1-carboxylate (25 g, 110 mmol) in THF (250 mL) at -78 °C under nitrogen was added dropwise 1 N NaHMDS in THF (130 mL, 130 mmol). The reaction was stirred at -78 °C for 1 h. A solution of 1,1,1-trifluoro-N-(pyridin-2-yl)-N-((trifluoromethyl)sulfonyl)methanesulfonamide (45 g, 126 mmol) in THF (100 mL) was next added dropwise over 15 min. The reaction was stirred for 1 h at -78 °C, then at 0 °C for 30 min. The reaction was quenched with cold water (500 mL), extracted with EtOAc (2 x 250 mL), washed with brine, dried (Na₂SO₄), filtered and concentrated under vacuum. The crude product was purified by silica gel chromatography (Isco® RediSep Rf Gold 330 g, 0 to 20% EtOAc in hexanes). (UV negative, visualized by charring with H₂SO₄ in EtOH.) The fractions containing product were combined and evaporated to dryness to give tert-butyl 4-(1-(((trifluoromethyl)sulfonyl)oxy)vinyl)piperidine-1-carboxylate (29.1 g, 81 mmol, 73.6% yield) as a colorless oil. (LCMS and ¹H NMR showed ∼16% of N-Boc-4-ethynylpiperidine) MS(ES) [M+H]⁺ 304.0 (-isobutylene), 283.1 (-Boc).

### c) tert-Butyl 4-(1-(4-(methoxycarbonyl)-3-methylthiophen-2-yl)vinyl)piperidine-1-carboxylate

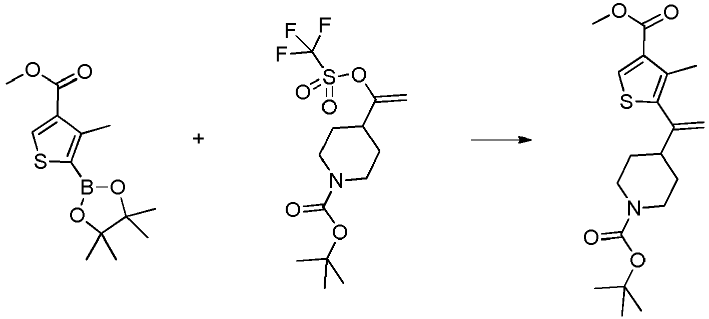

A stirred solution of methyl 4-methyl-5-(4,4,5,5-tetramethyl-1,3,2-dioxaborolan-2-yl)thiophene-3-carboxylate (50 g, 177 mmol), tert-butyl 4-(1-(((trifluoromethyl)sulfonyl)oxy)vinyl)piperidine-1-carboxylate (76 g, 211 mmol) and Na₂CO₃ (45 g, 536 mmol) in 1,4-dioxane (450 mL) and water (150 mL) was purged with N₂ by bubbling for 5 min. The reaction was charged with Pd(PPh₃)₄ (8 g, 6.92 mmol) and heated at 70 °C under N₂ for 1 h (vigourous gas evolution). The reaction was diluted with EtOAc (500 mL), washed with water (500 mL) and brine, dried (Na₂SO₄), and concentrated under vacuum. The residue was purified by silica gel chromatography (Isco® RediSep Rf Gold 330 g, 0 to 20% EtOAc/hexanes) to give tert-butyl 4-(1-(4-(methoxycarbonyl)-3-methylthiophen-2-yl)vinyl)piperidine-1-carboxylate (58.8 g, 161 mmol, 91% yield) as a light yellow oil. MS(ES) [M+H]⁺ 266.1 (-Boc), [M+H]⁺ 278.0 (-isobutylene, -MeOH), [M+H]⁺ 310.1 (-isobutylene), [M+Na]⁺ 388.1.

### d) (R)-tert-Butyl 4-(1-(4-(methoxycarbonyl)-3-methylthiophen-2-yl)ethyl)piperidine-1-carboxylate

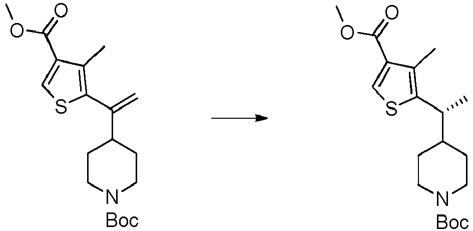

A solution of tert-butyl 4-(1-(4-(methoxycarbonyl)-3-methylthiophen-2-yl)vinyl)piperidine-1-carboxylate (31.0 g, 85 mmol) in DCM (500 mL) was purged with a N₂ stream for 10 min. To the purged solution was added (*R*,*R*)-[COD]Ir[cy₂PThrePHOX] (2.64 g, 1.527 mmol). The mixture was charged with H₂ (344.7 kPa (50 psi)) and shaken (Parr reactor) for 22 h, at which time it was filtered through Celite®, washed with DCM (50 mL), and concentrated. Purification of the residue (330 gram Isco® silica column; gradient B: 3-30%; A = heptane; B = EtOAc) gave (R)-tert-butyl 4-(1-(4-(methoxycarbonyl)-3-methylthiophen-2-yl)ethyl)piperidine-1-carboxylate (30.9 g, 80 mmol, 94% yield) as a thick oil. MS(ES) [M+H]⁺ 390.2. The optical purity of the product was determined to be 97.6% ee by chiral HPLC (Chiralpak AY-H, 5 microns, 4.6 mm x 150 mm; 245, 250 nm UV; 90:10:0.1 n-heptane:EtOH:isopropylamine, isocratic, 1.0 mL/min).

### e) (R)-tert-Butyl 4-(1-(5-bromo-4-(methoxycarbonyl)-3-methylthiophen-2-yl)ethyl)piperidine-1-carboxylate

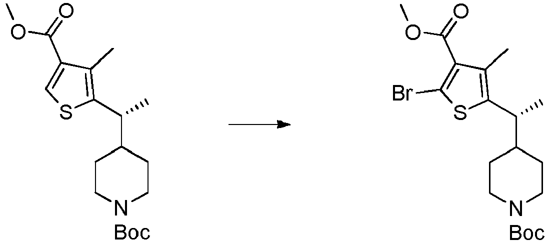

To a solution of (R)-tert-butyl 4-(1-(4-(methoxycarbonyl)-3-methylthiophen-2-yl)ethyl)piperidine-1-carboxylate (33.2 g, 90 mmol) in DMF (500 mL) was added NBS (20.9 g, 117 mmol). The reaction was maintained for approximately 4 h, at which time it was diluted with water and extracted with Et₂O (1.5 L). The organics were washed with water, brine, dried over MgSO₄, filtered and evaporated. Purification of the residue by column chromatography (5 to 20% EtOAc/hexanes) gave (R)-tert-butyl 4-(1-(5-bromo-4-(methoxycarbonyl)-3-methylthiophen-2-yl)ethyl)piperidine-1-carboxylate (34 g, 72.4 mmol, 80% yield). MS(ES) [M+H]⁺ 468.2, 470.2 (M+Na).

### f) (R)-tert-Butyl 4-(1-(4-(methoxycarbonyl)-3-methyl-5-(3-oxopropyl)thiophen-2-yl)ethyl)piperidine-1-carboxylate

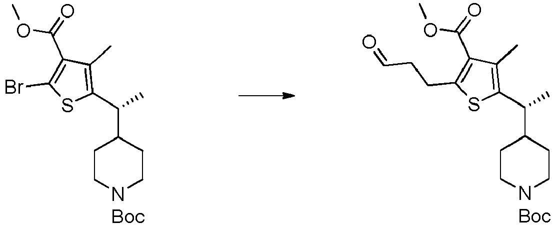

To a solution of (R)-tert-butyl 4-(1-(5-bromo-4-(methoxycarbonyl)-3-methylthiophen-2-yl)ethyl)piperidine-1-carboxylate (50 g, 112 mmol) in DMF (1 L) were added prop-2-en-1-ol (0.023 L, 560 mmol), Bu₄NCl (37.4 g, 134 mmol) and NaHCO₃ (37.6 g, 448 mmol). The reaction mixture was degassed with N₂ and Pd(OAc)₂ (3.77 g, 16.8 mmol) was added. The reaction mixture was heated at 65 °C for 2 h, at which time it allowed to cool to rt. The mixture was diluted with water (1.3 L) and extracted with Et₂O (2x). The combined extracts were dried (MgSO₄) and concentrated. The residue was purified by column chromatography (silica gel, 10 to 40% EtOAc/hexanes) to give (R)-tert-butyl 4-(1-(4-(methoxycarbonyl)-3-methyl-5-(3-oxopropyl)thiophen-2-yl)ethyl)piperidine-1-carboxylate (42 g, 94 mmol, 84% yield) as a pale yellow oil. MS(ES) [M+H]⁺ 446.2 (M+Na) 464.3 (M+MeCN).

### g) (R)-tert-Butyl 4-(1-(5-(3-(((2-methoxy-4,6-dimethylpyridin-3-yl)methyl)amino)propyl)-4-(methoxycarbonyl)-3-methylthiophen-2-yl)ethyl)piperidine-1-carboxylate

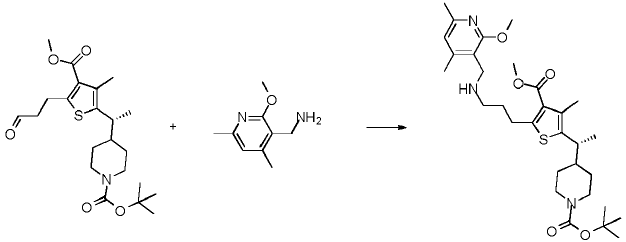

To a solution of (R)-tert-butyl 4-(1-(4-(methoxycarbonyl)-3-methyl-5-(3-oxopropyl)thiophen-2-yl)ethyl)piperidine-1-carboxylate (36.3 g, 86 mmol) in MeOH (600 mL) was added (2-methoxy-4,6-dimethylpyridin-3-yl)methanamine (16.38 g, 99 mmol) as a frozen solid. The reaction was maintained at r.t. for 1.5 h. The reaction was then cooled in an ice bath for 10 min, at which time NaBH₄ (8.11 g, 214 mmol) was added as a solid (some foaming/gas evolution). The mixture was stirred for 15 min. The ice bath was removed and the reaction was stirred at r.t. for 2 h. The reaction was recooled in an ice bath and quenched with sat. aqueous NH₄Cl (200 mL). The ice bath was removed and the reaction was concentrated *in vacuo* to ∼1/4 volume. The mixture was diluted with sat. aqueous NH₄Cl and extracted with EtOAc (2x). The combined organics were washed with sat. aqueous NH₄Cl, dried over MgSO₄ (stirred for 15 min), filtered through Celite®, and concentrated to give (R)-tert-butyl 4-(1-(5-(3-(((2-methoxy-4,6-dimethylpyridin-3-yl)methyl)amino)propyl)-4-(methoxycarbonyl)-3-methylthiophen-2-yl)ethyl)piperidine-1-carboxylate (55.5 g, 87 mmol based on 90% purity by HPLC, 100% yield) as an oil. The material was dried *in vacuo* for 30 min. MS(ES) [M+H]⁺ 574.5.

### h) (R)-5-(1-(1-(tert-Butoxycarbonyl)piperidin-4-yl)ethyl)-2-(3-(((2-methoxy-4,6-dimethylpyridin-3-yl)methyl)amino)propyl)-4-methylthiophene-3-carboxylic acid

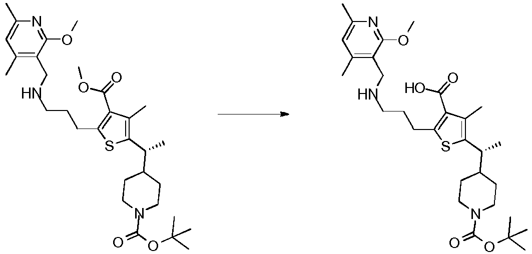

To a solution of (R)-tert-butyl 4-(1-(5-(3-(((2-methoxy-4,6-dimethylpyridin-3-yl)methyl)amino)propyl)-4-(methoxycarbonyl)-3-methylthiophen-2-yl)ethyl)piperidine-1-carboxylate (55 g, 96 mmol) in MeOH (600 mL) and THF (130 mL) was added 5 N NaOH (192 mL, 959 mmol). The reaction was heated at 63 °C for 22 h, at which time it was concentrated *in vacuo.* The residue was diluted with water (400 mL) and DCM (400 mL) and cooled in an ice bath. To the mixture was added 6 N HCl (158 mL, 949 mmol) to adjust the pH to 5-6 (paper). The mixture was stirred well and the layers were separated. The aqueous layer was extracted with DCM (200 mL) and the combined organics were dried over MgSO₄ (stirred for 30 min), filtered through Celite® and concentrated to give (R)-5-(1-(1-(tert-butoxycarbonyl)piperidin-4-yl)ethyl)-2-(3-(((2-methoxy-4,6-dimethylpyridin-3-yl)methyl)amino)propyl)-4-methylthiophene-3-carboxylic acid (52.5 g, 87 mmol, 91% yield). The residue was dried *in vacuo* for 2 h. MS(ES) [M+H]⁺ 560.4.

### i) (R)-tert-Butyl 4-(1-(5-((2-methoxy-4,6-dimethylpyridin-3-yl)methyl)-3-methyl-4-oxo-5,6,7,8-tetrahydro-4H-thieno[3,2-c]azepin-2-yl)ethyl)piperidine-1-carboxylate

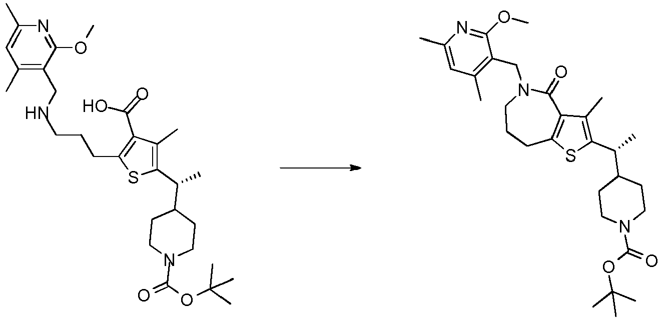

To a solution of (R)-5-(1-(1-(tert-butoxycarbonyl)piperidin-4-yl)ethyl)-2-(3-(((2-methoxy-4,6-dimethylpyridin-3-yl)methyl)amino)propyl)-4-methylthiophene-3-carboxylic acid (52.5 g, 94 mmol), EDC (21.58 g, 113 mmol) and HOAt (15.32 g, 113 mmol) in DMSO (400 mL) was added NMM (25.8 mL, 234 mmol). The reaction was maintained for 18 h, at which time it was poured slowly into ice water (1500 mL). The mixture was vigorously stirred (overhead stirrer) for 40 min. The mixture was filtered and the solids were washed with water and air-dried for ∼1 h. The still wet solid was dissolved in DCM and washed with sat. aqueous NH₄Cl, dried (Mg₂SO₄), filtered through Celite®, and concentrated. Purification of the residue by column chromatography (330 g Isco® silica column; gradient B: 4-40%; A = heptane. B = 3:1 EtOAc/EtOH) gave (R)-tert-butyl 4-(1-(5-((2-methoxy-4,6-dimethylpyridin-3-yl)methyl)-3-methyl-4-oxo-5,6,7,8-tetrahydro-4H-thieno[3,2-c]azepin-2-yl)ethyl)piperidine-1-carboxylate (34.3 g, 60 mmol, 64% yield) as a glassy yellow solid. MS(ES) [M+H]⁺ 542.4.

### j) (R)-5-((4,6-Dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one

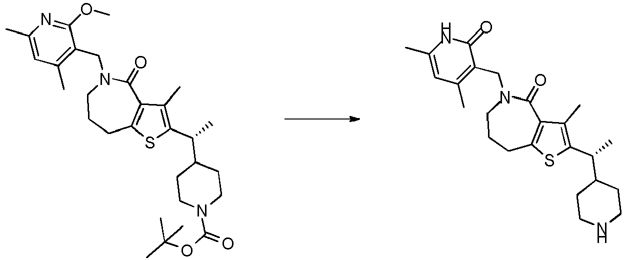

To a solution of (R)-tert-butyl 4-(1-(5-((2-methoxy-4,6-dimethylpyridin-3-yl)methyl)-3-methyl-4-oxo-5,6,7,8-tetrahydro-4H-thieno[3,2-c]azepin-2-yl)ethyl)piperidine-1-carboxylate (34.3 g, 63.3 mmol) in MeOH (450 mL) was added 4 N HCl in 1,4-dioxane (222 mL, 886 mmol). The reaction was maintained for 15 min at r.t., then heated at 70 °C for 24 h. The reaction was allowed to cool to r.t. and concentrated. The residue was diluted with DCM (500 mL) and water (300 mL) and the pH was adjusted to approximately 11 with concentrated NH₄OH. The mixture was stirred for 15 min, at which time the layers were separated. The aqueous layer was extracted with DCM and the combined organics were dried (Mg₂SO₄), filtered, and concentrated. The residue was dried *in vacuo* for 18 h to give (R)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one (29.3 g, 65.1 mmol, 100% yield). MS(ES) [M+H]⁺ 428.3.

### k) (R)-5-((4,6-Dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4H-thieno[3,2-c ]azepin-4-one

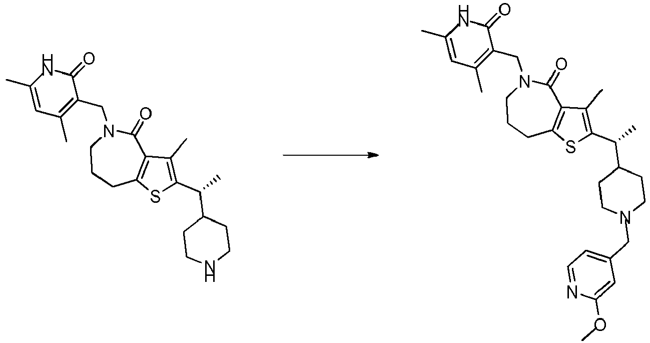

To a solution of (R)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one (20 g, 46.8 mmol) in DCE (400 mL) was added 2-methoxyisonicotinaldehyde (7.38 g, 53.8 mmol). The reaction was stirred for 5 min, at which time AcOH (5.36 mL, 94 mmol) was added. After 15 min, NaBH(OAc)₃ (29.7 g, 140 mmol) was added as a solid and the reaction was stirred at r.t. for 18 h. The reaction was diluted with DCM (100 mL) and water. The pH was adjusted to 10 with a combination of sat. NaHCO₃ and sat. Na₂CO₃. The mixture was stirred for 30 min and the layers were separated. The aqueous layer was extracted with DCM and the combined organics were dried over Mg₂SO₄, filtered and concentrated. Purification of the residue (330 g Isco® GOLD silica column; gradient B: 10-90%; A = heptane; B = 3:1 EtOAc/EtOH + 1% NH₄OH) gave a white solid. The solid was treated with 10% EtOH/heptane and concentrated. The residue was then treated with 100% heptane and concentrated. The solid was dried in a vacuum oven at 50 °C for 35 h to give (R)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4H-thieno[3,2-c]azepin-4-one (19.9 g, 35.6 mmol, 76% yield). ¹H NMR (DMSO-d6): δ 11.56 (s, 1H), 8.06 (d, *J*=5.2 Hz, 1H), 6.89 (d, *J*=5.2 Hz, 1H), 6.69 (s, 1H), 5.90 (s, 1H), 4.58-4.63 (m, 1H), 4.49-4.54 (m, 1H), 3.82 (s, 3H), 3.39 (s, 2H), 3.21-3.26 (m, 2H), 2.85-2.91 (m, 1H), 2.73-2.79 (m, 1H), 2.67 (t, *J*=7.3 Hz, 2H), 2.16 (s, 3H), 2.14 (s, 3H), 2.12 2.15 (m, 3H), 1.85-1.92 (m, 1H), 1.75-1.81 (m, 1H), 1.63-1.69 (m, 2H), 1.40 (br d, *J*=12.3 Hz, 1H), 1.23-1.31 (m, 1H), 1.17 (d, *J*=6.9 Hz, 3H), 1.14-1.27 (m, 2H), 0.82-0.89 (m, 1H). MS(ES) [M+H]⁺ 549.4.

### Example 8

### (R)-2-(1-(1-((5-Chloropyridin-2-yl)methyl)piperidin-4-yl)ethyl)-5-((4,6-dimethyl-2-oxo-1,2-dihydropyridin-3-yl)methyl)-3-methyl-5,6,7,8-tetrahydro-4H-thieno[3,2-c]azepin-4-one

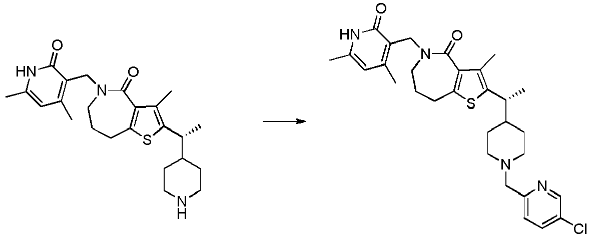

To a solution of (*R*)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one, *bis-*HCl (80 mg, 0.160 mmol) in MeOH (1.5 mL) were added DIPEA (0.064 mL, 0.368 mmol), 5-chloropicolinaldehyde (45.3 mg, 0.320 mmol), and AcOH (0.030 mL, 0.527 mmol), and the mixture was stirred at r.t. for 30 min. NaBH₃CN (40.2 mg, 0.639 mmol) was added and the mixture was stirred at r.t. for 18 h. The mixture was purified using reverse-phase HPLC. The fractions containing product were treated with 1 N HCl and concentrated to give 45 mg of product as a white solid (HCl salt). ¹H NMR (400 MHz, MeOH-*d*₄) δ 1.33 (d, *J*=6.84 Hz, 3 H), 1.50 - 1.72 (m, 2 H), 1.72 - 1.89 (m, 2 H), 2.16-2.38 (m, 6 H), 2.53 (s, 3 H), 2.66 (s, 3 H), 2.91 - 3.19 (m, 5 H), 3.35 - 3.45 (m, 2 H) 3.48-3.60 (m, 1 H), 3.64 (d, *J*=12.42 Hz, 1 H), 4.46 (s, 2 H), 4.86 (s, 2 H), 7.01 (s, 1 H), 7.54 (d, *J*=8.11 Hz, 1 H), 7.97 (dd, *J*=8.24, 2.41 Hz, 1 H), 8.70 (d, *J*=1.77 Hz, 1 H). MS(ES) [M+H]⁺ 553.6.

### Example 9

### (R)-5-((4,6-Dimethyl-2-oxo-1,2-dihydropyridin-3-yl)methyl)-3-methyl-2-(1-(1-((6-methylpyridin-2-yl)methyl)piperidin-4-yl)ethyl)-5,6,7,8-tetrahydro-4H-thieno[3,2-c]azepin-4-one

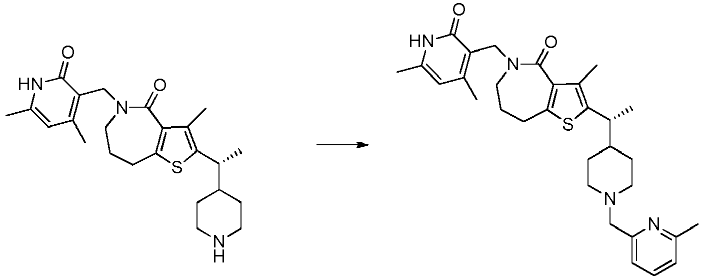

To a solution of (*R*)-5-((4,6-dimethyl-2-oxo-1,2-dihydropyridin-3-yl)methyl)-3-methyl-2-(1-(piperidin-4-yl)ethyl)-6,7-dihydrothieno[3,2-c]pyridin-4(5H)-one, *bis*-HCl (80 mg, 0.164 mmol) in MeOH (1.5 mL) were added DIPEA (0.066 mL, 0.378 mmol), 6-methylpicolinaldehyde (39.8 mg, 0.329 mmol), and AcOH (0.031 mL, 0.543 mmol). The mixture was stirred at r.t. for 30 min. NaBH₃CN (36.2 mg, 0.576 mmol) was added and the mixture was stirred at r.t. for 18 h. The mixture was purified using reverse-phase HPLC. The fractions containing product were treated with 1 N HCl and concentrated to give 43 mg of product as a white solid (HCl salt). ¹H NMR (400 MHz, MeOH-*d*₄) δ 1.33 (d, *J*=7.10 Hz, 3 H), 1.58 - 1.77 (m, 2 H), 1.81 (m, 2 H), 2.15 - 2.34 (m, 6 H), 2.51 (s, 3 H), 2.63 (s, 3 H), 2.83 (s, 3 H), 2.89 - 3.01 (m, 2 H), 3.01 - 3.29 (m, 3 H), 3.35 - 3.45 (m, 2 H), 3.57 (d, *J*=11.66 Hz, 1 H), 3.66 (d, *J*=12.17 Hz, 1 H), 4.67 (s, 2 H), 4.85 (s, 2 H), 6.94 (s, 1 H), 7.86 (d, *J*=7.86 Hz, 1 H), 7.95 (d, *J*=7.60 Hz, 1 H), 8.37 (t, *J*=7.86 Hz, 1 H). MS(ES) [M+H]⁺ 533.6.

### Example 10

### (R)-2-(1-(1-Benzylpiperidin-4-yl)ethyl)-5-((4,6-dimethyl-2-oxo-1,2-dihydropyridin-3-yl)methyl)-3-methyl-5,6,7,8-tetrahydro-4H-thieno[3,2-c]azepin-4-one

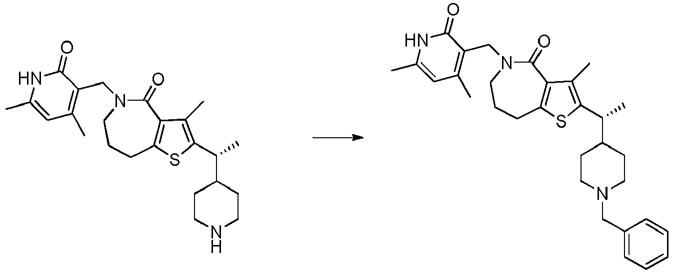

To a solution of (*R*)-5-((4,6-dimethyl-2-oxo-1,2-dihydropyridin-3-yl)methyl)-3-methyl-2-(1-(piperidin-4-yl)ethyl)-6,7-dihydrothieno[3,2-c]pyridin-4(5H)-one, *bis*-HCl (80 mg, 0.164 mmol) in MeOH (1.5 mL) were added DIPEA (0.066 mL, 0.378 mmol), 6-methylpicolinaldehyde (39.8 mg, 0.329 mmol), and AcOH (0.031 mL, 0.543 mmol). The mixture was stirred at r.t. for 30 min. NaBH₃CN (36.2 mg, 0.576 mmol) was added and the mixture was stirred at r.t. for 18 h. The mixture was purified using reverse-phase HPLC. The fractions containing product were treated with 1 N HCl and concentrated to give 46 mg of product as a white solid (HCl salt). ¹H NMR (400 MHz, MeOH-*d*₄) δ 1.24-1.39 (m, 3 H), 1.43 - 1.63 (m, 2 H), 1.67 - 1.86 (m, 2 H), 2.12 - 2.30 (m, 6 H), 2.50 (s, 3 H), 2.62 (s, 3 H), 2.88 - 3.10 (m, 5 H), 3.36 - 3.49 (m, 3 H), 3.53 (d, *J*=12.67 Hz, 1 H), 4.30 (s, 2 H), 4.81 - 4.88 (m, 2 H), 6.91 (s, 1 H), 7.44 - 7.60 (m, 5 H). MS(ES) [M+H]⁺ 533.6.

### Example 11

### (R)-5-((4,6-Dimethyl-2-oxo-1,2-dihydropyridin-3-yl)methyl)-2-(1-(1-((2-methoxypyridin-4-yl)methyl)piperidin-4-yl)ethyl)-3-methyl-6,7-dihydrothieno[3,2-c]pyridin-4(5H)-one

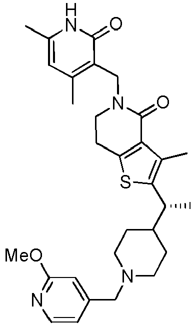

### a) (R)-tert-Butyl 4-(1-(5-bromo-4-(methoxycarbonyl)-3-methylthiophen-2-yl)ethyl)piperidine-1-carboxylate

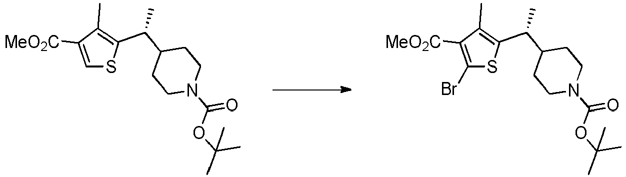

To a solution of (R)-tert-butyl 4-(1-(4-(methoxycarbonyl)-3-methylthiophen-2-yl)ethyl)piperidine-1-carboxylate (12 g, 32.7 mmol) in DMF (70 mL) was added NBS (8.14 g, 45.7 mmol). The mixture was stirred at RT for 18 h. The mixture was poured into water and extracted with CHCl₃ (3 x 100 mL). The combined organic layers were concentrated. The residue was purified using column chromatography (silica gel, 0 to 50% EtOAc/hexanes) to give (R)-tert-butyl 4-(1-(5-bromo-4-(methoxycarbonyl)-3-methylthiophen-2-yl)ethyl)piperidine-1-carboxylate (13.62 g, 30.5 mmol, 93% yield) as a colorless oil.

### b) (R)-tert-Butyl 4-(1-(5-(isoxazol-4-yl)-4-(methoxycarbonyl)-3-methylthiophen-2-yl)ethyl)piperidine-1-carboxylate

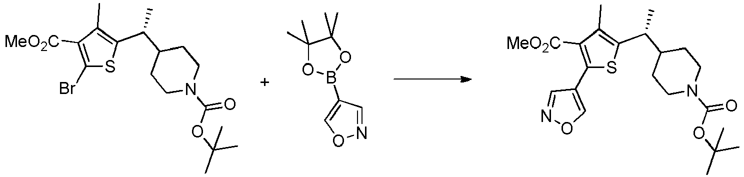

To a solution of (R)-tert-butyl 4-(1-(5-bromo-4-(methoxycarbonyl)-3-methylthiophen-2-yl)ethyl)piperidine-1-carboxylate (12.24 g, 27.4 mmol) in 1,4-dioxane (72 mL) were added 4-(4,4,5,5-tetramethyl-1,3,2-dioxaborolan-2-yl)isoxazole (6.42 g, 32.9 mmol), Na₂CO₃ (7.27 g, 68.5 mmol), and water (24 mL). The mixture was degassed for 10 min by bubbling nitrogen through the solution. Pd(PPh₃)₄ (1.584 g, 1.371 mmol) was added and the mixture was heated at 80 °C for 5 h. The mixture was then quenched with water (100 mL) and extracted with EtOAc (3x). The extract was dried over Na₂SO₄ and concentrated. The residue was purified using column chromatography (silica gel, 0 to 50% EtOAc/hexanes) to provide (R)-tert-butyl 4-(1-(5-(isoxazol-4-yl)-4-(methoxycarbonyl)-3-methylthiophen-2-yl)ethyl)piperidine-1-carboxylate (7.1 g, 16.34 mmol, 59.6% yield) as a pale brown oil. ¹H NMR (400MHz, MeOD-d₄) δ 8.94 (s, 1H), 8.61 (s, 1H), 4.91 (s, 1H), 4.18 -4.11 (m, 1H), 4.04 (dd, *J*=1.8, 13.1 Hz, 1H), 3.82 (s, 2H), 3.33 (td, *J*=1.5, 3.2 Hz, 1H), 3.02 (dd, *J*=6.9, 8.5 Hz, 1H), 2.28 (s, 2H), 1.93 (br. s., 1H), 1.47 - 1.44 (m, 9H), 1.35 - 1.28 (m, 5H), 1.21 - 1.08 (m, 2H), 0.95 - 0.87 (m, 2H).

### c) (R)-tert-Butyl 4-(1-(5-(cyanomethyl)-4-(methoxycarbonyl)-3-methylthiophen-2-yl)ethyl)piperidine-1-carboxylate

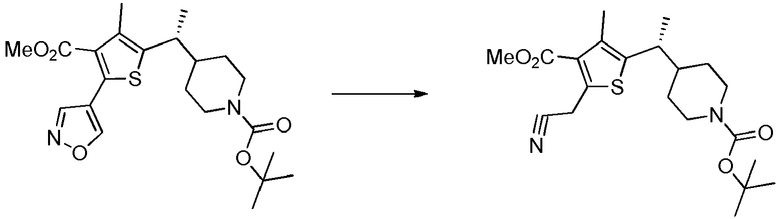

To a solution of (R)-tert-butyl 4-(1-(5-(isoxazol-4-yl)-4-(methoxycarbonyl)-3-methylthiophen-2-yl)ethyl)piperidine-1-carboxylate (7.5 g, 17.26 mmol) in MeOH (20 mL) was added a solution of KF (3.01 g, 51.8 mmol) in water (20 mL). The mixture was heated with stirring at 100 °C for 60 h. The mixture was allowed to cool to RT and was concentrated. The residue was treated with water (50 mL) and extracted with EtOAc (3x). These combined extracts were dried over Na₂SO₄ and concentrated. The residue was then further dried under vacuum to provide (R)-tert-butyl 4-(1-(5-(cyanomethyl)-4-(methoxycarbonyl)-3-methylthiophen-2-yl)ethyl)piperidine-1-carboxylate (6.06 g, 14.91 mmol, 86% yield) as pale brown oil. MS(ES) [M+Na]⁺ 429.6.

### d) (R)-tert-Butyl 4-(1-(3-methyl-4-oxo-4,5,6,7-tetrahydrothieno[3,2-c]pyridin-2-yl)ethyl)piperidine-1-carboxylate

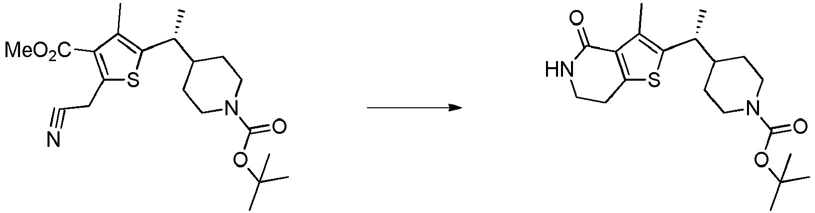

To a cooled (0 °C) solution of (R)-tert-butyl 4-(1-(5-(cyanomethyl)-4-(methoxycarbonyl)-3-methylthiophen-2-yl)ethyl)piperidine-1-carboxylate (6.00 g, 14.76 mmol) in EtOH (200 mL) was added cobalt(II) chloride hexahydrate (2.63 g, 11.07 mmol). The reaction was stirred for 15 min, at which time NaBH₄ (2.62 g, 69.4 mmol) was added portion wise over 15 min (gas evolution observed). The reaction was stirred for 1 h, at which time the ice bath was removed and the reaction was stirred at RT for 18 h. The dark colored reaction was monitored by LCMS. After 18 h, there was mostly product, some impurities, and a small amount of uncyclized amine intermediate. The reaction was stirred for an additional 2 h. The solvent was removed *in vacuo.* The residue was treated with saturated NH₄Cl and the pH was adjusted to pH ∼7 with 1 M HCl. The mixture was extracted with DCM (3 x 100 mL) and the combined organics were dried over Mg₂SO₄, filtered, and concentrated. The dark brown residue was dried in vacuo for 1 h, dissolved in DCM, and purified by flash column (200 g Isco® GOLD silica column; Gradient B: 5-75%, A: heptane, B: 3 to 1 EtOAc to EtOH to give (R)-tert-butyl 4-(1-(3-methyl-4-oxo-4,5,6,7-tetrahydrothieno[3,2-c]pyridin-2-yl)ethyl)piperidine-1-carboxylate (3.32 g, 8.33 mmol, 56.5% yield). MS(ES) [M+Na]⁺ 401.7.

### e) (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)ethyl)piperidine-1-carboxylate

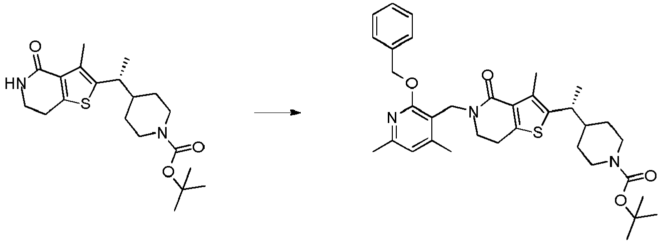

To a cooled (ice bath) solution of (R)-tert-butyl 4-(1-(3-methyl-4-oxo-4,5,6,7-tetrahydrothieno[3,2-c]pyridin-2-yl)ethyl)piperidine-1-carboxylate (2.40 g, 6.34 mmol) in DMF (50 mL) was added KOtBu (7.61 mL, 7.61 mmol) via syringe over 1 min. The reaction mixture was stirred for 10 min, at which time 2-(benzyloxy)-3-(chloromethyl)-4,6-dimethylpyridine (1.826 g, 6.97 mmol) was added as a solid. The mixture was stirred for 30 min and monitored by LCMS. The reaction was poured into a mixture of ice and saturated NH₄Cl (200 mL) and was stirred for 10 min. Et₂O (200 mL) was added the mixture was stirred for 15 min. The layers were separated and the aqueous was extracted with Et₂O. The combined organics were washed with brine, dried over Mg₂SO₄, filtered and concentrated. The residue was further dried in vacuo for 1 h and purified by flash chromatography (120 g Isco® GOLD silica column. Gradient B: 5-40%. A:heptane. B: EtOAc) to give (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)ethyl)piperidine-1-carboxylate (3.30 g, 5.19 mmol, 82% yield) as a glassy solid. MS(ES) [M+Na]⁺ 604.9.

### f) 5-((4,6-Dimethyl-2-oxo-2,3-dihydropyridin-3-yl)methyl)-3-methyl-2-((R)-1-(piperidin-4-yl)ethyl)-6,7-dihydrothieno[3,2-c]pyridin-4(5H)-one

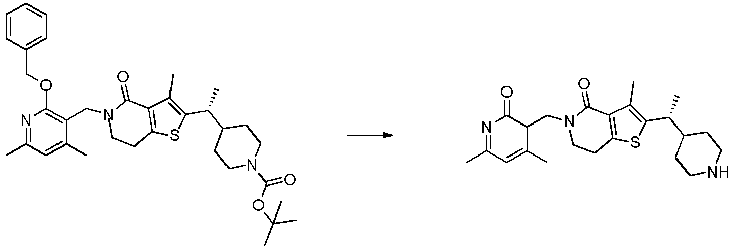

To a solution of (R)-tert-butyl 4-(1-(5-((2-(benzyloxy)-4,6-dimethylpyridin-3-yl)methyl)-3-methyl-4-oxo-4,5,6,7-tetrahydrothieno[3,2-c]pyridin-2-yl)ethyl)piperidine-1-carboxylate (3.3 g, 5.47 mmol) in CHCl₃ (40 mL) was added TFA (21.05 mL, 273 mmol) *via* syringe over 1 min. The reaction was maintained for 10 min, then heated at 35 °C for 2 h. The reaction was allowed to cooled to r.t. and the volatiles were removed in vacuo. The reaction was diluted with toluene and concentrated (2x). The residue was diluted with water and slowly basified to pH ∼11 with NH₄OH. The white suspension was treated with DCM (100 mL) and stirred for 15 min. The layers were separated and the aqueous was extracted with DCM. The combined organics were dried over MgSO₄, filtered, and concentrated. The residue was treated with TBME and concentrated in vacuo to give 5-((4,6-dimethyl-2-oxo-2,3-dihydropyridin-3-yl)methyl)-3-methyl-2-((R)-1-(piperidin-4-yl)ethyl)-6,7-dihydrothieno[3,2-c]pyridin-4(5H)-one (2.60 g, 5.66 mmol, 104 % yield) as a white solid. MS(ES) [M+Na]⁺ 414.3.

### g)(R)-5-((4,6-Dimethyl-2-oxo-1,2-dihydropyridin-3-yl)methyl)-2-(1-(1-((2-methoxypyridin-4-yl)methyl)piperidin-4-yl)ethyl)-3-methyl-6,7-dihydrothieno[3,2-c]pyridin-4(5H)-one

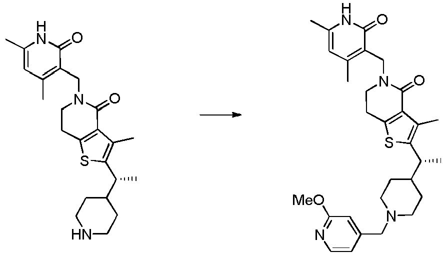

To a solution of (R)-5-((4,6-dimethyl-2-oxo-1,2-dihydropyridin-3-yl)methyl)-3-methyl-2-(1-(piperidin-4-yl)ethyl)-6,7-dihydrothieno[3,2-c]pyridin-4(5H)-one (1.3 g, 3.14 mmol) in DCE (40 mL) was added 2-methoxyisonicotinaldehyde (0.474 g, 3.46 mmol). After 5 min, AcOH (0.540 mL, 9.43 mmol) was added and the reaction was stirred for 15 min. To the mixture was added NaBH(OAc)₃ (2.66 g, 12.57 mmol) as a solid. The reaction was stirred at r.t. for 2 h. The reaction was diluted with DCM (125 mL) and water. The reaction was basified to pH ∼10 with a mixture of saturated NaHCO₃ and saturated Na₂CO₃. The mixture was stirred for 15 min, the layers were separated, and the aqueous was extracted with DCM. The combined organic were dried over MgSO₄, filtered, and concentrated. Purification of the residue by flash column (80 g Isco® GOLD silica column. Gradient B: 8-75%. A: DCM. B: 90:10:1 DCM/MeOH/NH₄OH). The residue was treated with TBME, concentrated, and dried in a vacuum oven at 40 °C for 18 h to give (R)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-6,7-dihydrothieno[3,2-c]pyridin-4(5H)-one (1.19 g, 2.159 mmol, 68.7% yield) as a white solid. ¹H NMR (400 MHz, DMSO-d6) δ 1.12 - 1.32 (m, 6 H) 1.38 (d, J=12.17 Hz, 1 H) 1.74 - 1.93 (m, 3 H) 2.13 (d, J=8.62 Hz, 6 H) 2.26 - 2.36 (m, 3 H) 2.71 (d, J=11.15 Hz, 1 H) 2.76 - 2.96 (m, 4 H) 3.39 (s, 2 H) 3.43 - 3.55 (m, 2 H) 3.82 (s, 3 H) 4.48 (s, 2 H) 5.87 (s, 1 H) 6.65 - 6.71 (m, 1 H), 6.89 (dd, J=5.32, 1.27 Hz, 1 H) 8.06 (d, J=5.58 Hz, 1 H) 11.55 (s, 1 H). MS(ES) [M+Na]⁺ 535.4.

### Example 12

### (R)-5-((4,6-Dimethyl-2-oxo-1,2-dihydropyridin-3-yl)methyl)-2-(1-(1-((6-methoxypyridin-2-yl)methyl)piperidin-4-yl)ethyl)-3-methyl-5,6,7,8-tetrahydro-4H-thieno[3,2-c]azepin-4-one

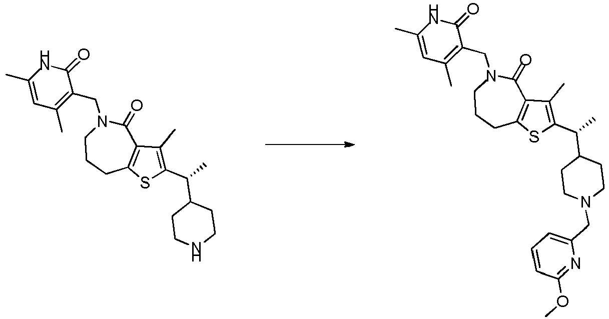

To a solution of (R)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one (8.6 g, 20.1 mmol) in DCE (200 mL) was added 6-methoxypicolinaldehyde (3.31 g, 24.13 mmol). The reaction was stirred for 5 min, at which time AcOH (2.3 mL, 40 mmol) was added. After 15 min, NaBH(OAc)₃ (12.8 g, 60 mmol) was added as a solid and the reaction was stirred at r.t. for 18 h. The reaction was diluted with DCM (200 mL) and water. The pH was adjusted to 10 with a combination of sat. NaHCO₃ and sat. Na₂CO₃. The mixture was stirred for 30 min and the layers were separated. The aqueous layer was extracted with DCM and the combined organics were dried over Mg₂SO₄, filtered and concentrated. Purification of the residue (200 g Isco® GOLD silica column; gradient B: 10-90%; A = heptane; B = 3:1 EtOAc/EtOH +1% NH₄OH) gave a white solid.

To a solution of the solid in EtOH (220 mL) was added Silicycle Thiol resin (6 g), The mixture was heated at 50 °C for 48 h, at which time it was filtered through a pad of Celite® and washed with EtOH (2 x 15 mL). The filtrate was concentrated and the residue was treated with 10% EtOH/heptane (100 mL) and concentrated, treated with 5% EtOH/heptane (100 mL) and concentrated, and treated with 100% heptane (100 mL) and concentrated. The product was dried in a vacuum oven at 45 °C for 60 h to give (R)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4H-thieno[3,2-c]azepin-4-one (9.3 g, 16.5 mmol, 82% yield) as a white solid. ¹H NMR (DMSO-d₆) δ 11.59 (s, 1H), 8.06 (d, J=5.1 Hz, 1H), 6.89 (dd, J=5.3, 1.3 Hz, 1H), 6.69 (s, 1H), 5.90 (s, 1H), 4.60 (d, J=13.7 Hz, 1H), 4.50 (d, J=13.4 Hz, 1H), 3.82 (s, 3H), 3.39 (s, 2H), 3.15-3.32 (m, 2H), 2.62-2.92 (m, 5H), 2.05-2.26 (m, 9H), 1.74-1.93 (m, 3H), 1.65 (quin, J=6.6 Hz, 2H), 1.40 (d, J=11.9 Hz, 1H), 1.09-1.31 (m, 6H). MS(ES) [M+H]⁺ 549.4.

### Example 13

### (R)-2-(1-(1-(cyclohexylmethyl)piperidin-4-yl)ethyl)-5-((4,6-dimethyl-2-oxo-1,2-dihydropyridin-3-yl)methyl)-3-methyl-5,6,7,8-tetrahydro-4H-thieno[3,2-c]azepin-4-one

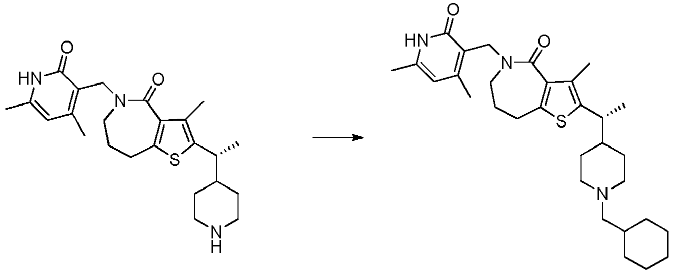

To a solution of (R)-5-((4,6-dimethyl-2-oxo-1,2-dihydropyridin-3-yl)methyl)-3-methyl-2-(1-(piperidin-4-yl)ethyl)-5,6,7,8-tetrahydro-4H-thieno[3,2-c]azepin-4-one (100 mg, 0.234 mmol) in MeOH (1.5 mL) was added cyclohexanecarbaldehyde (0.057 mL, 0.468 mmol) and AcOH (0.020 mL, 0.351 mmol). The reaction was stirred at r.t. for 2.5 h, at which time NaBH₃CN (58.8 mg, 0.935 mmol) was added and the mixture was stirred at r.t. for 18 h. The mixture was purified using reverse-phase HPLC (Phenomenex Gemini C18(2) 100A, AXIA, 5 microns, 30 mm x 100 mm; 254 nm UV; 38% CH₃CN/H₂O, 0.1% formic acid to 80% CH₃CN/H₂O, 0.1% formic acid, 30.0 mL/min) to give (R)-2-(1-(1-(cyclohexylmethyl)piperidin-4-yl)ethyl)-5-((4,6-dimethyl-2-oxo-1,2-dihydropyridin-3-yl)methyl)-3-methyl-5,6,7,8-tetrahydro-4H-thieno[3,2-c]azepin-4-one, formic acid salt (47 mg, 0.079 mmol, 34% yield) as a white solid. ¹H NMR (400 MHz, methanol-*d*₄) δ 0.98-1.14 (m, 2 H), 1.20 - 1.44 (m, 6 H), 1.46 - 1.67 (m, 2 H), 1.67 - 1.90 (m, 11 H), 2.12 - 2.25 (m, 4 H), 2.28 (s, 3 H), 2.32 (s, 3 H), 2.71 - 2.90 (m, 3 H), 2.90 - 3.09 (m, 4 H), 3.35 - 3.46 (m, 3 H), 3.46 - 3.67 (m, 2 H), 4.73 (d, *J*=13.94 Hz, 1 H), 4.82 (d, *J*=13.94 Hz, 1 H), 6.15 (s, 1 H). MS(ES) [M+H]⁺ 524.4.

### Example 14

### (R)-5-((4,6-Dimethyl-2-oxo-1,2-dihydropyridin-3-yl)methyl)-3-methyl-2-(1-(1-((1-methylcyclohexyl)methyl)piperidin-4-yl)ethyl)-5,6,7,8-tetrahydro-4H-thieno[3,2-c]azepin-4-one

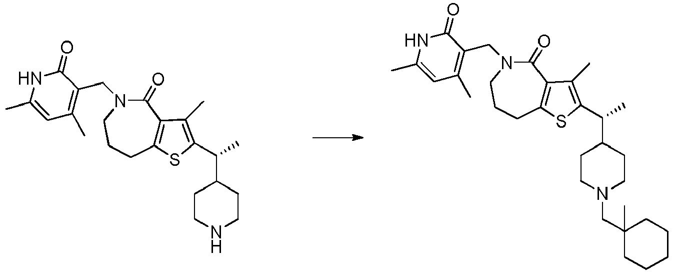

Following the general procedure of Example 13, (R)-5-((4,6-dimethyl-2-oxo-1,2-dihydropyridin-3-yl)methyl)-3-methyl-2-(1-(1-((1-methylcyclohexyl)methyl)piperidin-4-yl)ethyl)-5,6,7,8-tetrahydro-4H-thieno[3,2-c]azepin-4-one was prepared. ¹H NMR (DMSO-d₆) δ 11.58 (br. s., 1 H) 5.91 (s, 1 H) 4.46 - 4.67 (m, 2 H) 3.20 - 3.27 (m, 2 H) 2.62 - 2.90 (m, 6 H) 2.12 - 2.19 (m, 9 H) 1.98 - 2.08 (m, 3 H) 1.61 - 1.77 (m, 3 H) 1.30 - 1.48 (m, 6 H) 1.11 - 1.27 (m, 11 H) 0.81 (s, 3 H). MS(ES) [M+H]⁺ 538.4.

### Assay Protocol

Compounds contained herein were evaluated for their ability to inhibit the methyltransferase activity of EZH2 within the PRC2 complex. Human PRC2 complex was prepared by co-expressing each of the 5 member proteins (FLAG-EZH2, EED, SUZ12, RbAp48, AEBP2) in Sf9 cells followed by co-purification. Enzyme activity was measured in a scintillation proximity assay (SPA) where a tritiated methyl group is transferred from 3H-SAM to a lysine residue on a biotinylated, unmethylated peptide substrate derived from histone H3. The peptides were captured on streptavidin-coated SPA beads and the resulting signal was read on a ViewLux plate reader.

### Part A. Compound Preparation

1. Prepare 10 mM stock of compounds from solid in 100% DMSO.
2. Set up an 11-point serial dilution (1:4 dilution, top concentration 10 mM) in 100% DMSO for each test compound in a 384 well plate leaving columns 6 and 18 for DMSO controls.
3. Dispense 10 nL of compound from the dilution plate into reaction plates (Corning, 384-well polystyrene NBS, Cat# 3673).

### Part B. Reagent Preparation

Prepare the following solutions:
1. 1x Base Buffer, 50 mM Tris-HCl, pH 8, 2 mM MgCl₂: Per 1 L of base buffer, combine 1 M Tris-HCl, pH 8 (50 mL), 1 M MgCl₂ (2 mL), and distilled water (948 mL).
2. 1x Assay Buffer: Per 10 mL of 1x Assay Buffer, combine 1x Base Buffer (9.96 mL), 1 M DTT (40 uL), and 10% Tween-20 (1 uL) to provide a final concentration of 50 mM Tris-HCl, pH 8, 2 mM MgCl₂, 4 mM DTT, 0.001% Tween-20.
3. 2x Enzyme Solution: Per 10 mL of 2x Enzyme Solution, combine 1x Assay Buffer (9.99 mL) and 3.24 uM EZH2 5 member complex (6.17 uL) to provide a final enzyme concentration of 1 nM.
4. SPA Bead Solution: Per 1 mL of SPA Bead Solution, combine Streptavidin coated SPA beads (PerkinElmer, Cat# RPNQ0261, 40 mg) and 1x Assay Buffer (1 mL) to provide a working concentration of 40 mg/mL.
5. 2x Substrate Solution: Per 10 mL of 2x Substrate Solution, combine 40 mg/mL SPA Bead Solution (375 uL), 1 mM biotinylated histone H3K27 peptide (200 uL), 12.5 uM 3H-SAM (240 uL; 1 mCi/mL), 1 mM cold SAM (57 uL), and 1x Assay Buffer (9.13 mL) to provide a final concentration of 0.75 mg/mL SPA Bead Solution, 10 uM biotinylated histone H3K27 peptide, 0.15 uM 3H-SAM (∼12 uCi/mL 3H-SAM), and 2.85 uM cold SAM.
6. 2.67x Quench Solution: Per 10 mL of 2.67x Quench Solution, combine 1x Assay Buffer (9.73 mL) and 10 mM cold SAM (267 uL) to provide a final concentration of 100 uM cold SAM.

### Part C. Assay Reaction in 384-well Grenier Bio-One Plates

### Compound Addition

1. Stamp 10 nL/well of 1000x Compound to test wells (as noted above).
2. Stamp 10 nL/well of 100% DMSO to columns 6 & 18 (high and low controls, respectively).

### Assay

1. Dispense 5 uL/well of 1x Assay Buffer to column 18 (low control reactions).
2. Dispense 5 uL/well of 2x Substrate Solution to columns 1 - 24 (note: substrate solution should be mixed to ensure homogeneous bead suspension before dispensing into matrix reservoir).
3. Dispense 5 uL/well of 2x Enzyme Solution to columns 1 - 17, 19 - 24.
4. Incubate the reaction for 60 min at room temperature.

### Quench

1. Dispense 6 uL/well of the 2.67x Quench Solution to columns 1 - 24.
2. Seal assay plates and spin for ∼1 min at 500 rpm.
3. Dark adapt plates in the ViewLux instrument for 15 - 60 min.

### Read plates

1. Read the assay plates on the Viewlux Plate Reader utilizing the 613 nm emission filter or clear filter (300 s exposure).
Reagent addition can be done manually or with automated liquid handler.

### Results

Percent inhibition was calculated relative to the DMSO control for each compound concentration and the resulting values were fit using standard IC₅₀ fitting parameters within the ABASE data fitting software package.

The exemplified compounds were generally tested according to the above or an analogous assay and were found to be inhibitors of EZH2. Specific biological activities tested according to such assays are listed in the following table. The IC₅₀ values of ≤ 10 nM indicate that the activity of compound was approaching the limit of detection in the assay. Repeating the assay run(s) may result in somewhat different IC₅₀ values.

| Example | EZH2 IC₅₀ (nM) | Example | EZH2 IC₅₀ (nM) | Example | EZH2 IC₅₀ (nM) |
|---|---|---|---|---|---|
| 1 | ≤ 10 | 6 | ≤ 10 | 11 | ≤ 10 |
| 2 | ≤ 10 | 7 | ≤ 10 | 12 | ≤ 10 |
| 3 | ≤ 10 | 8 | ≤ 10 | 13 | ≤ 10 |
| 4 | ≤ 10 | 9 | ≤ 10 | 14 | ≤ 10 |
| 5 | ≤ 10 | 10 | ≤ 10 | | |

## Claims

1. A compound according to Formula (I) or a pharmaceutically acceptable salt thereof: 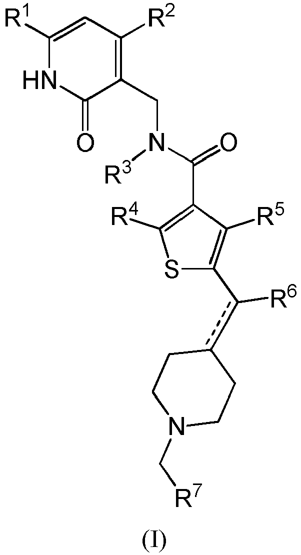 wherein:
----- represents a single or double bond;
R¹ is -NH₂, (C₁-C₄)alkyl, or hydroxy(C₁-C₄)alkyl;
R² is (C₁-C₄)alkyl;
R³ and R⁴ are each hydrogen;
or R³ and R⁴ taken together represent -CH₂CH₂- or -CH₂CH₂CH₂-;
R⁵ is hydrogen, halogen, or (C₁-C₃)alkyl;
R⁶ is hydrogen or (C₁-C₃)alkyl; and
R⁷ is a 6-membered saturated or unsaturated ring optionally containing one, two, or three heteroatoms independently selected from oxygen, nitrogen, and sulfur, wherein said ring is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, -CO₂H, -CO₂(C₁-C₄)alkyl,-CONH₂, -CONH(C₁-C₄)alkyl, -CON(C₁-C₄)alkyl(C₁-C₄)alkyl, -NH₂,-NH(C₁-C₄)alkyl, -N(C₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-C₄)alkoxy-.

2. The compound or pharmaceutically acceptable salt thereof according to claim 1, represented by Formula (II): 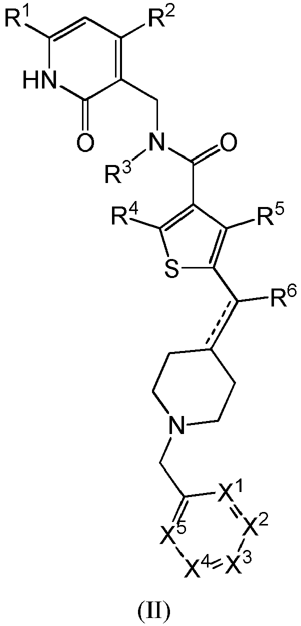 wherein:
=-== represents a single or double bond;
X¹, X², X³, X⁴, and X⁵ are each independently N, CH, or CR⁸, provided that at least two of X¹, X², X³, X⁴, and X⁵ are CH;
each R⁸ is independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano, - CO₂H, -CO₂(C₁-C₄)alkyl, -
CONH₂, -CONH(C₁-C₄)alkyl, -CON(C₁-C₄)alkyl(C₁-C₄)alkyl, -NH₂, -NH(C₁-C₄)alkyl, -N(C ₁-C₄)alkyl(C₁-Ca)alkyl, hydroxyl, (C₁-C₄)alkoxy, hydroxy(C₂-C₄)alkoxy-, and (C₁-C₄)alkoxy(C₂-C₄)alkoxy-.

3. The compound or pharmaceutically acceptable salt thereof according to claim 1, represented by Formula (III): 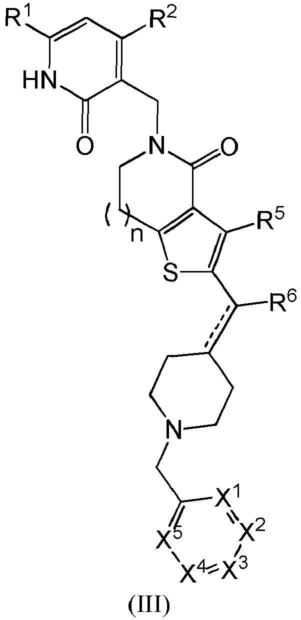 wherein:
X¹, X², X³, X⁴, and X⁵ are each independently N, CH, or CR⁸, provided that at least two of X¹, X², X³, X⁴, and X⁵ are CH;
n is 1 or 2,
and R⁸ is as defined in claim 2.

4. The compound or pharmaceutically acceptable salt thereof according to claim 1, represented by Formula (IV): 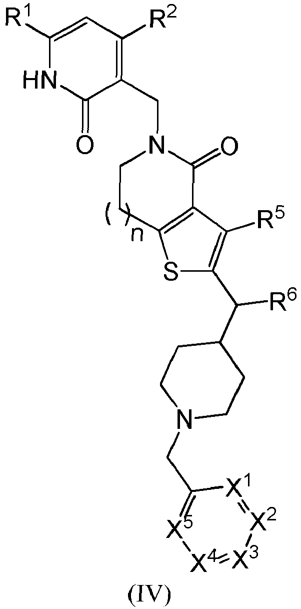 wherein:
X¹, X², X³, X⁴, and X⁵ are each independently N, CH, or CR⁸, provided that at least two of X¹, X², X³, X⁴, and X⁵ are CH;
R⁸ is as defined in claim 2; and
n is 1 or 2.

5. The compound or pharmaceutically acceptable salt thereof according to claim 1, represented by Formula (V): 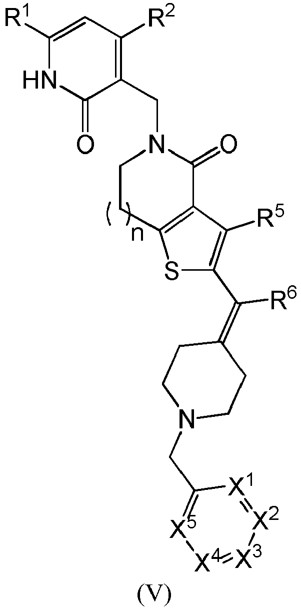 wherein:
X¹, X², X³, X⁴, and X⁵ are each independently N, CH, or CR⁸, provided that at least two of X¹, X², X³, X⁴, and X⁵ are CH;
R⁸ is as defined in claim 2; and
n is 1 or 2.

6. The compound or pharmaceutically acceptable salt thereof according to claim 1, wherein R⁷ is selected from the group consisting of phenyl, pyridinyl, pyridazinyl, pyrimidinyl, pyrazinyl, and triazinyl, each of which is optionally substituted by one, two, or three groups independently selected from halogen, (C₁-C₄)alkyl, halo(C₁-C₄)alkyl, hydroxy(C₁-C₄)alkyl, (C₁-C₄)alkoxy(C₁-C₄)alkyl, (C₃-C₆)cycloalkyl, cyano,-CO₂H, -CO₂(C₁-C₄)alkyl,-CONH₂, -CONH(C₁-C₄)alkyl, -CON(C₁-C₄)alkyl(C₁-C₄)alkyl, -NH₂, -NH(C₁-C₄)alkyl, -N(C ₁-C₄)alkyl(C₁-C₄)alkyl, oxo, hydroxyl, (C₁-C₄)alkoxy, hydroxy(C₂-C₄)alkoxy-, and (C₁-C₄)alkoxy(C₂-Ca)alkoxy-.

7. The compound or pharmaceutically acceptable salt thereof according to claim 1, wherein R⁷ is phenyl or pyridinyl optionally substituted by one or two groups independently selected from halogen, (C₁-C₄)alkyl, halo(C₁-C₄)alkyl, hydroxyl, and (C₁-C₄)alkoxy.

8. The compound or pharmaceutically acceptable salt thereof according to any one of claims 2-5, wherein X¹, X², X³, X⁴, and X⁵ are each independently N, CH, or CR⁸, provided that not more than one of X¹, X², X³, X⁴, and X⁵ is N and at least three of X¹, X², X³, X⁴, and X⁵ are CH.

9. The compound or pharmaceutically acceptable salt thereof according to any one of claims 2-5, wherein each R⁸ is independently selected from halogen, (C₁-C₄)alkyl, halo(C₁-C₄)alkyl, hydroxyl, and (C₁-C₄)alkoxy.

10. The compound or pharmaceutically acceptable salt thereof according to any one of claims 1-8, wherein R⁶ is hydrogen, methyl, or ethyl.

11. The compound according to claim 1 which is:
(R)-5-((4,6-dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-*c*]pyridin-4(5*H*)-one;
(*R*)-2-(1-(1-benzylpiperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)-one;
(*R*)-5-((4,6-dimethyl-2-oxo-1,2-dihydropyridin-3-yl)methyl)-3-methyl-2-(1-(1-(pyridin-4-ylmethyl)piperidin-4-yl)propyl)-6,7-dihydrothieno[3,2-*c*]pyridin-4(5*H*)-one;
(*R*)-2-(1-(1-((5-chloropyridin-2-yl)methyl)piperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)one;
*N*-((4,6-dimethyl-2-oxo-1,2-dihydropyridin-3-yl)methyl)-5-(1-(1-((2-methoxypyridin-4-yl)methyl)piperidin-4-ylidene)propyl)-4-methylthiophene-3-carboxamide;
(*R*)-5-((4,6-dimethyl-2-oxo-1,2-dihydropyridin-3-yl)methyl)-3-methyl-2-(1-(1-(pyridin-2-ylmethyl)piperidin-4-yl)ethyl)-5,6,7,8-tetrahydro-4*H*-thieno[3,2-*c*]azepin-4-one;
(*R*)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-5,6,7,8-tetrahydro-4*H-*thieno[3,2-*c*]azepin-4-one;
(*R*)-2-(1-(1-((5-chloropyridin-2-yl)methyl)piperidin-4-yl)ethyl)-5-((4,6-dimethyl-2-oxo-1,2-dihydropyridin-3-yl)methyl)-3-methyl-5,6,7,8-tetrahydro-4*H-t*hieno[3,2-c]azepin-4-one;
(*R*)-5-((4,6-dimethyl-2-oxo-1,2-dihydropyridin-3-yl)methyl)-3-methyl-2-(1-(1-((6-methylpyridin-2-yl)methyl)piperidin-4-yl)ethyl)-5,6,7,8-tetrahydro-4*H*-thieno[3,2-*c*]azepin-4-one;
(*R*)-2-(1-(1-benzylpiperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-one;
(*R*)-5-((4,6-dimethyl-2-oxo-1,2-dihydropyridin-3-yl)methyl)-2-(1-(1-((2-methoxypyridin-4-yl)methyl)piperidin-4-yl)ethyl)-3-methyl-6,7-dihydrothieno[3,2-*c*]pyridin-4(5*H*)-one;
(*R*)-5-((4,6-dimethyl-2-oxo-1,2-dihydropyridin-3-yl)methyl)-2-(1-(1-((6-methoxypyridin-2-yl)methyl)piperidin-4-yl)ethyl)-3-methyl-5,6,7,8-tetrahydro-4*H-*thieno[3,2-*c*]azepin-4-one;
(*R*)-2-(1-(1-(cyclohexylmethyl)piperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-one; or
(*R*)-5-((4,6-dimethyl-2-oxo-1,2-dihydropyridin-3-yl)methyl)-3-methyl-2-(1-(1-((1-methylcyclohexyl)methyl)piperidin-4-yl)ethyl)-5,6,7,8-tetrahydro-4*H*-thieno[3,2-*c*]azepin-4-one;
or a pharmaceutically acceptable salt thereof.

12. A pharmaceutical composition comprising the compound or pharmaceutically acceptable salt thereof according to any one of claims 1-11 and a pharmaceutically acceptable excipient.

13. A compound or pharmaceutically acceptable salt thereof according to any one of claims 1-11 for use in therapy.

14. A compound or pharmaceutically acceptable salt thereof according to any one of claims 1-11 for use in the treatment of cancer.

15. A compound or pharmaceutically acceptable salt thereof for use according to claim 14 wherein said cancer is selected from the group consisting of: brain (gliomas), glioblastomas, leukemias, lymphomas, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, gastric, bladder, head and neck, kidney, lung, liver, melanoma, renal, ovarian, pancreatic, prostate, sarcoma, osteosarcoma, giant cell tumor of bone, and thyroid.

## Patentansprüche

1. Verbindung der Formel (I) oder ein pharmazeutisch annehmbares Salz davon: 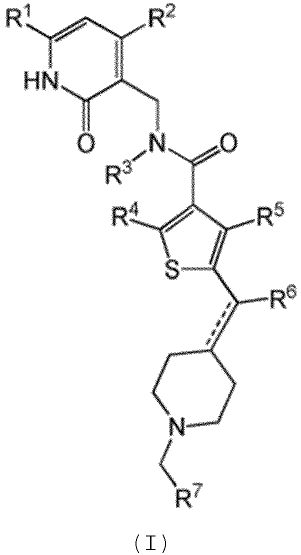 worin:
----- eine Einzel- oder Doppelbindung darstellt;
R¹ -NH₂, (C₁-C₄)Alkyl oder Hydroxy(C₁-C₄)alkyl ist;
R² (C₁-C₄)Alkyl ist;
R³ und R⁴ jeweils Wasserstoff sind;
oder R³ und R⁴ zusammen genommen -CH₂CH₂- oder -CH₂CH₂CH₂-darstellen;
R⁵ Wasserstoff, Halogen oder (C₁-C₃)Alkyl ist;
R⁶ Wasserstoff oder (C₁-C₃)Alkyl ist; und
R⁷ ein 6-gliedriger gesättigter oder ungesättigter Ring ist, der gegebenenfalls ein, zwei oder drei Heteroatome enthält, unabhängig ausgewählt aus Sauerstoff, Stickstoff und Schwefel, worin der Ring gegebenenfalls mit einer, zwei oder drei Gruppen substituiert ist, unabhängig ausgewählt aus Halogen, (C₁-C₄)Alkyl, Halo (C₁-C₄) alkyl, Hydroxy (C₁-C₄) alkyl, (C₁-C₄)Alkoxy(C₁-C₄)alkyl, (C₃-C₆)Cycloalkyl, Cyano, -CO₂H,-CO₂(C₁-C₄)Alkyl, -CONH₂, -CONH(C₁-C₄)Alkyl, -CON(C₁-C₄)Alkyl(C₁-C₄)alkyl, -NH₂, -NH(C₁-C₄)Alkyl, -N(C₁-C₄)Alkyl(C₁-C₄) alkyl, Oxo, Hydroxyl, (C₁-C₄)Alkoxy, Hydroxy(C₂-C₄) alkoxy- und (C₁-C₄)Alkoxy(C₂-C₄)alkoxy-.

2. Verbindung oder pharmazeutisch annehmbares Salz davon gemäß Anspruch 1, dargestellt durch Formel (II): 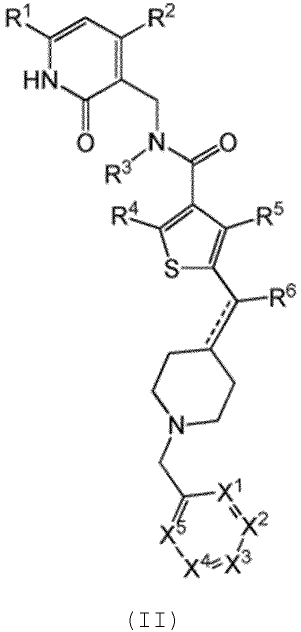 worin:
----- eine Einzel- oder Doppelbindung darstellt;
X¹, X², X³, X⁴ und X⁵ jeweils unabhängig N, CH oder CR⁸ sind, vorausgesetzt, dass mindestens zwei aus X¹, X², X³, X⁴ und X⁵ CH sind;
jedes R⁸ unabhängig ausgewählt ist aus Halogen, (C₁-C₄)Alkyl, Halo (C₁-C₄) alkyl, Hydroxy (C₁-C₄) alkyl, (C₁-C₄)Alkoxy(C₁-C₄)alkyl, (C₃-C₆)Cycloalkyl, Cyano, -CO₂H,-CO₂(C₁-C₄)Alkyl, -CONH₂, -CONH(C₁-C₄)Alkyl, -CON(C₁-C₄)Alkyl(C₁-C₄)alkyl, -NH₂, -NH(C₁-C₄)Alkyl, -N(C₁-C₄)Alkyl(C₁-C₄)alkyl, Hydroxyl, (C₁-C₄)Alkoxy, Hydroxy(C₂-C₄)alkoxy- und (C₁-C₄)Alkoxy(C₂-C₄)alkoxy-.

3. Verbindung oder pharmazeutisch annehmbares Salz davon gemäß Anspruch 1, dargestellt durch Formel (III) : 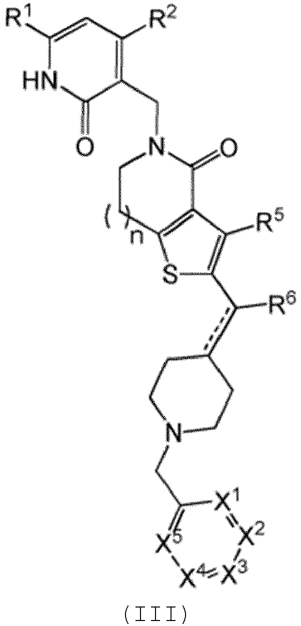 worin:
X¹, X², X³, X⁴ und X⁵ jeweils unabhängig N, CH oder CR⁸ sind, vorausgesetzt, dass mindestens zwei aus X¹, X², X³, X⁴ und X⁵ CH sind;
n 1 oder 2 ist
und R⁸ wie in Anspruch 2 definiert ist.

4. Verbindung oder pharmazeutisch annehmbares Salz davon gemäß Anspruch 1, dargestellt durch Formel (IV): 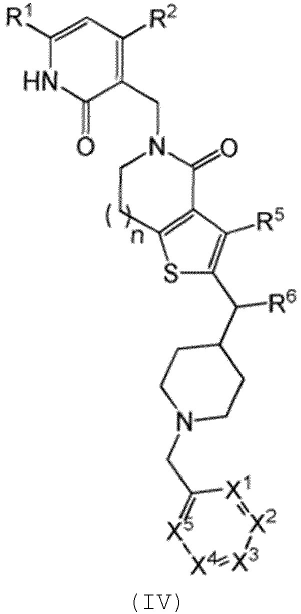 worin:
X¹, X², X³, X⁴ und X⁵ jeweils unabhängig N, CH oder CR⁸ sind, vorausgesetzt, dass mindestens zwei aus X¹, X², X³, X⁴ und X⁵ CH sind;
R⁸ wie in Anspruch 2 definiert ist; und
n 1 oder 2 ist.

5. Verbindung oder pharmazeutisch annehmbares Salz davon gemäß Anspruch 1, dargestellt durch Formel (V): 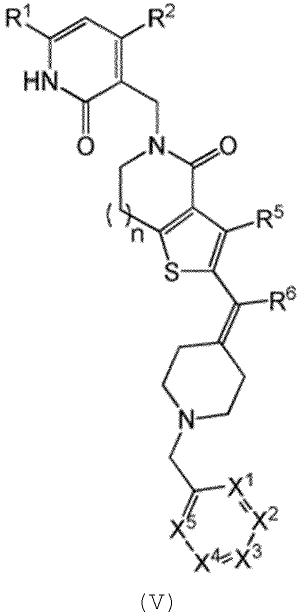 worin:
X¹, X², X³, X⁴ und X⁵ jeweils unabhängig N, CH oder CR⁸ sind, vorausgesetzt, dass mindestens zwei aus X¹, X², X³, X⁴ und X⁵ CH sind;
R⁸ wie in Anspruch 2 definiert ist; und
n 1 oder 2 ist.

6. Verbindung oder pharmazeutisch annehmbares Salz davon gemäß Anspruch 1, worin R⁷ ausgewählt ist aus der Gruppe, bestehend aus Phenyl, Pyridinyl, Pyridazinyl, Pyrimidinyl, Pyrazinyl und Triazinyl, wobei jedes gegebenenfalls mit einer, zwei oder drei Gruppen substituiert ist, unabhängig ausgewählt aus Halogen, (C₁-C₄)Alkyl, Halo (C₁-C₄) alkyl, Hydroxy(C₁-C₄)alkyl, (C₁-C₄)Alkoxy(C₁-C₄)alkyl, (C₃-C₆)Cycloalkyl, Cyano, -CO₂H, CO₂(C₁-C₄)Alkyl, -CONH₂,-CONH(C₁-C₄)Alkyl, -CON(C₁-C₄)Alkyl(C₁-C₄)alkyl, -NH₂, -NH(C₁-C₄)Alkyl, -N(C₁-C₄)Alkyl(C₁-C₄)alkyl, Oxo, Hydroxyl, (C₁-C₄)Alkoxy, Hydroxy(C₂-C₄)alkoxy- und (C₁-C₄)Alkoxy(C₂-C₄)alkoxy-.

7. Verbindung oder pharmazeutisch annehmbares Salz davon gemäß Anspruch 1, worin R⁷ Phenyl oder Pyridinyl ist, das gegebenenfalls mit einer oder zwei Gruppen substituiert ist, unabhängig ausgewählt aus Halogen, (C₁-C₄)Alkyl, Halo(C₁-C₄)alkyl, Hydroxyl und (C₁-C₄)Alkoxy.

8. Verbindung oder pharmazeutisch annehmbares Salz davon gemäß mindestens einem der Ansprüche 2-5, worin X¹, X², X³, X⁴ und X⁵ jeweils unabhängig N, CH oder CR⁸ sind, vorausgesetzt, dass nicht mehr als eines aus X¹, X², X³, X⁴ und X⁵ N ist und mindestens drei aus X¹, X², X³, X⁴ und X⁵ CH sind.

9. Verbindung oder pharmazeutisch annehmbares Salz davon gemäß mindestens einem der Ansprüche 2-5, worin jedes R⁸ unabhängig ausgewählt ist aus Halogen, (C₁-C₄)Alkyl, Halo(C₁-C₄)alkyl, Hydroxyl und (C₁-C₄)Alkoxy.

10. Verbindung oder pharmazeutisch annehmbares Salz davon gemäß mindestens einem der Ansprüche 1-8, worin R⁶ Wasserstoff, Methyl oder Ethyl ist.

11. Verbindung gemäß Anspruch 1, die:
(*R*)-5-((4,6-Dimethyl-2-oxo-1,2-dihydropyridin-3-yl)methyl)-3-methyl-2-(1-(1-(pyridin-2-ylmethyl)piperidin-4-yl)propyl)-6,7-dihydrothieno[3,2-c]pyridin-4(5H)-on;
(*R*)-2-(1-(1-Benzylpiperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)-on;
(*R*)-5-((4,6-Dimethyl-2-oxo-1,2-dihydropyridin-3-yl)methyl)-3-methyl-2-(1-(1-(pyridin-4-ylmethyl)piperidin-4-yl)propyl)-6,7-dihydrothieno[3,2-*c*]pyridin-4(5*H*)-on;
(*R*)-2-(1-(1-((5-Chlorpyridin-2-yl)methyl)piperidin-4-yl)propyl)-5-((4,6-dimethyl-2-oxo-1,2-dihydropyridin-3-yl)methyl)-3-methyl-6,7-dihydrothieno[3,2-*c*]pyridin-4(5*H*)-on;
*N*-((4,6-Dimethyl-2-oxo-1,2-dihydropyridin-3-yl)methyl)-5-(1-(1-((2-methoxypyridin-4-yl)methyl)piperidin-4-yliden)propyl)-4-methylthiophen-3-carboxamid;
(*R*)-5-((4,6-Dimethyl-2-oxo-1,2-dihydropyridin-3-yl)methyl)-3-methyl-2-(1-(1-(pyridin-2-ylmethyl)piperidin-4-yl)ethyl)-5,6,7,8-tetrahydro-*4H*-thieno[3,2-*c*]azepin-4-on;
(*R*)-5-((4,6-Dimethyl-2-oxo-1,2-dihydropyridin-3-yl)methyl)-2-(1-(1-((2-methoxypyridin-4-yl)methyl) piperidin-4-yl)ethyl)-3-methyl-5,6,7,8-tetrahydro-*4H*-thieno[3,2-*c*]azepin-4-on;
(*R*)-2-(1-(1-((5-Chlorpyridin-2-yl)methyl)piperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-on;
(*R*)-5-((4,6-Dimethyl-2-oxo-1,2-dihydropyridin-3-yl)methyl)-3-methyl-2-(1-(1-((6-methylpyridin-2-yl)methyl)piperidin-4-yl)ethyl)-5,6,7,8-tetrahydro-*4H-*thieno[3,2-c]azepin-4-on;
(*R*)-2-(1-(1-Benzylpiperidin-4-yl)ethyl)-5-((4,6-dimethyl-2-oxo-1,2-dihydropyridin-3-yl)methyl)-3-methyl-5,6,7,8-tetrahydro-*4H*-thieno[3,2*-*c]azepin-4-on;
(*R*)-5-((4,6-Dimethyl-2-oxo-1,2-dihydropyridin-3-yl)methyl)-2-(1-(1-((2-methoxypyridin-4-yl)methyl) piperidin-4-yl)ethyl)-3-methyl-6,7-dihydrothieno[3,2-*c*]pyridin-4(5*H*)-on;
(*R*)-5-((4,6-Dimethyl-2-oxo-1,2-dihydropyridin-3-yl)methyl)-2-(1-(1-((6-methoxypyridin-2-yl)methyl)piperidin-4-yl)ethyl)-3-methyl-5,6,7,8-tetrahydro-4*H*-thieno[3,2-*c*]azepin-4-on;
(*R*)-2-(1-(1-(Cyclohexylmethyl)piperidin-4-yl)ethyl)-5-((4,6-dimethyl-2-oxo-1,2-dihydropyridin-3-yl)methyl)-3-methyl-5,6,7,8-tetrahydro-4*H*-thieno[3,2-*c*]azepin-4-on; oder
(*R*)-5-((4,6-Dimethyl-2-oxo-1,2-dihydropyridin-3-yl)methyl)-3-methyl-2-(1-(1-((1-methylcyclohexyl)methyl)piperidin-4-yl)ethyl)-5,6,7,8-tetrahydro-*4H*-thieno[3,2-*c*]azepin-4-on;
oder ein pharmazeutisch annehmbares Salz davon ist.

12. Pharmazeutische Zusammensetzung, umfassend die Verbindung oder ein pharmazeutisch annehmbares Salz davon gemäß mindestens einem der Ansprüche 1-11 und einen pharmazeutisch annehmbaren Hilfsstoff.

13. Verbindung oder pharmazeutisch annehmbares Salz davon gemäß mindestens einem der Ansprüche 1-11 zur Verwendung bei einer Heilbehandlung.

14. Verbindung oder pharmazeutisch annehmbares Salz davon gemäß mindestens einem der Ansprüche 1-11 zur Verwendung bei der Behandlung von Krebs.

15. Verbindung oder pharmazeutisch annehmbares Salz davon zur Verwendung gemäß Anspruch 14, worin der Krebs ausgewählt ist aus der Gruppe, bestehend aus: Gehirn (Gliome), Glioblastomen, Leukämien, Lymphomen, Bannayan-Zonana-Syndrom, Cowden-Krankheit, Lhermitte-Duclos-Krankheit, Brust, entzündlichem Brustkrebs, Wilm-Tumor, Ewing-Sarkom, Rhabdomyosarkom, Ependymom, Medulloblastom, Dickdarm, Magen, Blase, Kopf und Hals, Niere, Lunge, Leber, Melanom, renal, Eierstock, Bauchspeicheldrüse, Prostata, Sarkom, Osteosarkom, Riesenzelltumor des Knochens und Schilddrüse.

## Revendications

1. Composé selon la Formule (I) ou un sel de celui-ci acceptable sur le plan pharmaceutique : 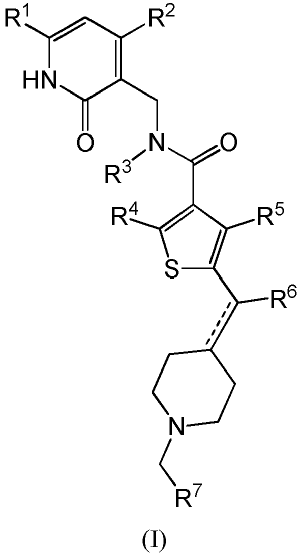 dans lequel :
----- représente une liaison simple ou double ;
R¹ est -NH₂, alkyle en (C₁ à C₄), ou hydroxy-alkyle en (C₁ à C₄) ;
R² est alkyle en (C₁ à C₄) ;
R³ et R⁴ sont chacun hydrogène ;
ou R³ et R⁴ pris ensemble représentent -CH₂CH₂- ou -CH₂CH₂CH₂- ; R⁵ est hydrogène, halogène, ou alkyle en (C₁ à C₃) ;
R⁶ est hydrogène ou alkyle en (C₁ à C₃) ; et
R⁷ est un cycle saturé ou insaturé à 6 chaînons contenant éventuellement un, deux, ou trois hétéroatomes indépendamment sélectionnés à partir d'oxygène, azote et soufre, dans lequel ledit cycle est éventuellement substitué par un, deux, ou trois groupes indépendamment sélectionnés à partir d'halogène, alkyle en (C₁ à C₄), halo-alkyle en (C₁ à C₄), hydroxy-alkyle en (C₁ à C₄), alcoxy en (C₁ à C₄)-alkyle en (C₁ à C₄), cycloalkyle en (C₃ à C₆), cyano, -CO₂H, -CO₂-alkyle en (C₁ à C₄), -CONH₂, -CONH-alkyle en (C₁ à C₄), -CON-alkyl en (C₁ à C₄)-alkyle en (C₁ à C₄), -NH₂, -NH-alkyle en (C₁ à C₄), -N-alkyl en (C₁ à C₄)-alkyle en (C₁ à C₄), oxo, hydroxyle, alcoxy en (C₁ à C₄), hydroxy-alcoxy en (C₂ à C₄)-, et alcoxy en (C₁ à C₄)-alcoxy en (C₂ à C₄)-.

2. Composé ou sel de celui-ci acceptable sur le plan pharmaceutique selon la revendication 1, représenté par la Formule (II) : 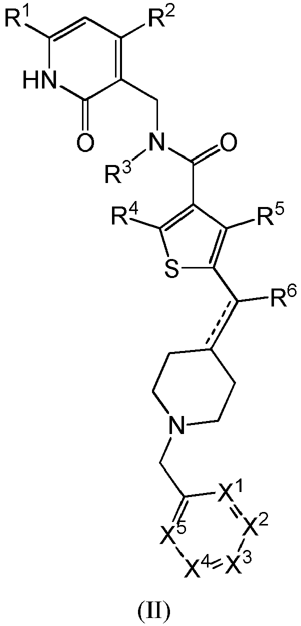 dans lequel :
----- représente une liaison simple ou double ;
X¹, X², X³, X⁴ et X⁵ sont chacun indépendamment N, CH, ou CR⁸, à condition qu'au moins deux de X¹, X², X³, X⁴ et X⁵ soient CH ;
chaque R⁸ est indépendamment choisi parmi halogène, alkyle en (C₁ à C₄), halo-alkyle en (C₁ à C₄), hydroxy-alkyle en (C₁ à C₄), alcoxy en (C₁ à C₄)-alkyle en (C₁ à C₄), cycloalkyle en (C₃ à C₆), cyano, -CO₂H, -CO₂-alkyle en (C₁ à C₄), -CONH₂, -CONH-alkyle en (C₁ à C₄), -CON-alkyl en (C₁ à C₄)-alkyle en (C₁ à C₄), -NH₂, -NH-alkyle en (C₁ à C₄), -N-alkyl en (C₁ à C₄)-alkyle en (C₁ à C₄), hydroxyle, alcoxy en (C₁ à C₄), hydroxy-alcoxy en (C₂ à C₄)- et alcoxy en (C₁ à C₄)-alcoxy en (C₂ à C₄)-.

3. Composé ou sel de celui-ci acceptable sur le plan pharmaceutique selon la revendication 1, représenté par la Formule (III) : 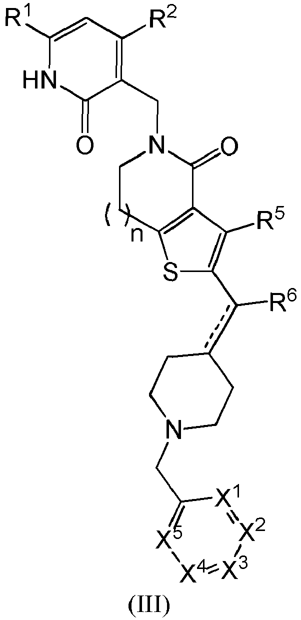 dans lequel :
X¹, X², X³, X⁴ et X⁵ sont chacun indépendamment N, CH, ou CR⁸, à condition qu'au moins deux de X¹, X², X³, X⁴ et X⁵ soient CH ;
n vaut 1 ou 2
et R⁸ est tel que défini dans la revendication 2.

4. Composé ou sel de celui-ci acceptable sur le plan pharmaceutique selon la revendication 1, représenté par la Formule (IV) : 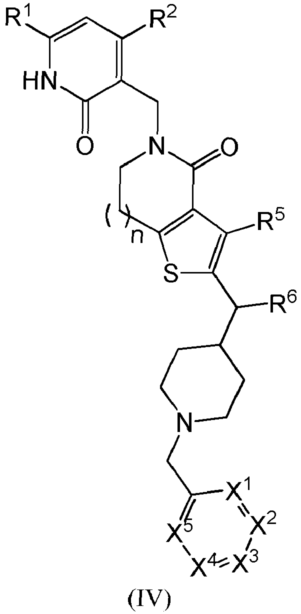 dans lequel :
X¹, X², X³, X⁴ et X⁵ sont chacun indépendamment N, CH, ou CR⁸, à condition qu'au moins deux de X¹, X², X³, X⁴ et X⁵ soient CH ;
R⁸ est tel que défini dans la revendication 2 ; et
n vaut 1 ou 2.

5. Composé ou sel de celui-ci acceptable sur le plan pharmaceutique selon la revendication 1, représenté par la Formule (V) : 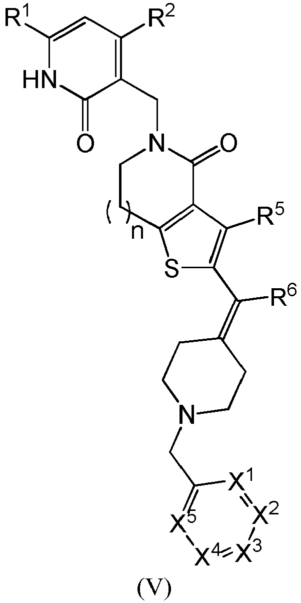 dans lequel :
X¹, X², X³, X⁴ et X⁵ sont chacun indépendamment N, CH, ou CR⁸, à condition qu'au moins deux de X¹, X², X³, X⁴ et X⁵ soient CH ;
R⁸ est tel que défini dans la revendication 2 ; et
n vaut 1 ou 2.

6. composé ou sel de celui-ci acceptable sur le plan pharmaceutique selon la revendication 1, dans lequel R⁷ est sélectionné à partir du groupe constitué par phényle, pyridinyle, pyridazinyle, pyrimidinyle, pyrazinyle, et triazinyle, dont chacun est éventuellement substitué par un, deux, ou trois groupes indépendamment sélectionnés à partir d'halogène, alkyle en (C₁ à C₄), halo-alkyle en (C₁ à C₄), hydroxy-alkyle en (C₁ à C₄), alcoxy en (C₁ à C₄)-alkyle en (C₁ à C₄), cycloalkyle en (C₃ à C₆), cyano, -CO₂H, -CO₂-alkyle en (C₁ à C₄), -CONH₂, -CONH-alkyle en (C₁ à C₄), -CON-alkyl en (C₁ à C₄)-alkyle en (C₁ à C₄), -NH₂, -NH-alkyle en (C₁ à C₄), -N-alkyl en (C₁ à C₄)-alkyle en (C₁ à C₄), oxo, hydroxyle, alcoxy en (C₁ à C₄), hydroxy-alcoxy en (C₂ à C₄)-, et alcoxy en (C₁ à C₄)-alcoxy en (C₂ à C₄)-.

7. Composé ou sel de celui-ci acceptable sur le plan pharmaceutique selon la revendication 1, dans lequel R⁷ est phényle ou pyridinyle éventuellement substitué par un ou deux groupes indépendamment sélectionnés à partir d'halogène, alkyle en (C₁ à C₄), halo-alkyle en (C₁ à C₄), hydroxyle, et alcoxy en (C₁ à C₄).

8. Composé ou sel de celui-ci acceptable sur le plan pharmaceutique selon l'une quelconque des revendications 2 à 5, dans lequel X¹, X², X³, X⁴ et X⁵ sont chacun indépendamment N, CH, ou CR⁸, à condition que pas plus d'un parmi X¹, X², X³, X⁴ et X⁵ ne soit N et qu'au moins trois parmi X¹, X², X³, X⁴ et X⁵ soient CH.

9. Composé ou sel de celui-ci acceptable sur le plan pharmaceutique selon l'une quelconque des revendications 2 à 5, dans lequel chaque R⁸ est indépendamment sélectionné à partir d'halogène, alkyle en (C₁ à C₄), halo-alkyle en (C₁ à C₄), hydroxyle, et alcoxy en (C₁ à C₄).

10. Composé ou sel de celui-ci acceptable sur le plan pharmaceutique selon l'une quelconque des revendications 1 à 8, dans lequel R⁶ est hydrogène, méthyle ou éthyle.

11. Composé selon la revendication 1 qui est :
(*R*)-5-((4,6-diméthyl-2-oxo-1,2-dihydropyridin-3-yl)méthyl)-3-méthyl-2-(1-(1-(pyridin-2-ylméthyl)pipéridin-4-yl)propyl)-6,7-dihydrothiéno[3,2-*c*]pyridin-4(5*H*)-one ;
*(R)-*2*-(*1*-*(1-benzylpipéridin-4-yl)propyl)-5-((4,6-diméthyl-2-oxo-1,2-dihydropyridin-3-yl)méthyl)-3-méthyl-6,7-dihydrothiéno[3,2-*c*]pyridin-4(5*H*)-one ;
(*R*)-5-((4,6-diméthyl-2-oxo-1,2-dihydropyridin-3-yl)méthyl)-3-méthyl-2-(1-(1-(pyridin-4-ylméthyl)pipéridin-4-yl)propyl)-6,7-dihydrothiéno[3,2-*c*]pyridin-4(5*H*)-one ;
(*R*)-2-(1-(1-((5-chloropyridin-2-yl)méthyl)pipéridin-4-yl)propyl)-5-((4,6-diméthyl-2-oxo-1,2-dihydropyridin-3-yl)méthyl)-3-méthyl-6,7-dihydrothiéno[3,2-*c*]pyridin-4(5*H*)-one ;
*N*-((4,6-diméthyl-2-oxo-1,2-dihydropyridin-3-yl)méthyl)-5-(1-(1-((2-méthoxypyridin-4-yl)méthyl)pipéridin-4-ylidène)propyl)-4-méthylthiophène-3-carboxamide ;
(*R*)-5-((4,6-diméthyl-2-oxo-1,2-dihydropyridin-3-yl)méthyl)-3-méthyle-2-(1-(1-(pyridin-2-ylméthyl)pipéridin-4-yl)éthyl)-5,6,7,8-tétrahydro-4*H-*thiéno[3,2-*c*]azépin-4-one ;
(*R*)-5-((4,6-diméthyl-2-oxo-1,2-dihydropyridin-3-yl)méthyl)-2-(1-(1-((2-methoxypyridin-4-yl)méthyl)pipéridin-4-yl)éthyl)-3-méthyl-5,6,7,8-tétrahydro-4*H*-thiéno[3,2-*c*]azépin-4-one ;
(*R*)-2-(1-(1-((5-chloropyridin-2-yl)méthyl)pipéridin-4-yl)éthyl)-5-((4,6-diméthyl-2-oxo-1,2-dihydropyridin-3-yl)méthyl)-3-méthyl-5,6,7,8-tétrahydro-4*H-*thiéno[3,2-*c*]azépin-4-one ;
(*R*)-5-((4,6-diméthyl-2-oxo-1,2-dihydropyridin-3-yl)méthyl)-3-méthyl-2-(1-(1-((6-méthylpyridin-2-yl)méthyl)pipéridin-4-yl)éthyl)-5,6,7,8-tétrahydro-4*H-*thiéno[3,2-*c*]azépin-4-one ; (*R*)*-*2*-(*1-(1-benzylpipéridin-4-yl)éthyl)-5-((4,6-diméthyl-2-oxo-1,2-dihydropyridin-3-yl)méthyl)-3-méthyl-5,6,7,8-tétrahydro-4*H*-thiéno[3,2-*c*]azépin-4-one ;
(*R*)-5-((4,6-diméthyl-2-oxo-1,2-dihydropyridin-3-yl)méthyl)-2-(1-(1-((2-methoxypyridin-4-yl)méthyl)pipéridin-4-yl)éthyl)-3-méthyl-6,7-dihydrothiéno[3,2-*c*]pyridin-4(5*H*)-one ;
(*R*)-5-((4,6-diméthyl-2-oxo-1,2-dihydropyridin-3-yl)méthyl)-2-(1-(1-((6-methoxypyridin-2-yl)méthyl)pipéridin-4-yl)éthyl)-3-méthyl-5,6,7,8-tétrahydro-4*H*-thiéno[3,2-*c*]azépin-4-one ;
(*R*)-2-(1-(1-(cyclohexylméthyl)pipéridin-4-yl)éthyl)-5-((4,6-diméthyl-2-oxo-1,2-dihydropyridin-3-yl)méthyl)-3-méthyl-5,6,7,8-tétrahydro-4*H*-thiéno[3,2-*c*]azépin-4-one ; ou
(*R*)-5-((4,6-diméthyl-2-oxo-1,2-dihydropyridin-3-yl)méthyl)-3-méthyl-2-(1-(1-((1-méthylcyclohexyl)méthyl)pipéridin-4-yl)éthyl)-5,6,7,8-tétrahydro-4*H-*thiéno[3,2-*c*]azépin-4-one ;
ou un sel de ceux-ci acceptable sur le plan pharmaceutique.

12. Composition pharmaceutique comprenant le composé ou sel de celui-ci acceptable sur le plan pharmaceutique selon l'une quelconque des revendications 1 à 11 et un excipient acceptable sur le plan pharmaceutique.

13. Composé ou sel de celui-ci acceptable sur le plan pharmaceutique selon l'une quelconque des revendications 1 à 11 pour une utilisation en thérapie.

14. Composé ou sel de celui-ci acceptable sur le plan pharmaceutique selon l'une quelconque des revendications 1 à 11 pour utilisation dans le traitement d'un cancer.

15. Composé ou sel de celui-ci acceptable sur le plan pharmaceutique pour utilisation selon la revendication 14 dans lequel ledit cancer est sélectionné à partir du groupe constitué par : cerveau (gliomes), glioblastomes, leucémie, lymphomes, syndrome de Bannayan-Zonana, maladie de Cowden, maladie de Lhermitte-Duclos, sein, cancer du sein inflammatoire, tumeur de Wilm, sarcome d'Ewing, rhabdomyosarcome, épendymome, médulloblastome, côlon, gastrique, vessie, tête et cou, rein, poumon, foie, mélanome, rénal, ovarien, pancréatique, prostate, sarcome, ostéosarcome, tumeur à cellules géantes de l'os et thyroïde.
